# EUROPEAN PATENT APPLICATION

(11) **EP 4 534 528 A1**
(43) Date of publication of application: **09.04.2025**
(21) Application number: 23816418.0
(22) Date of filing: 05.06.2023
(51) Int. Cl.: C07D 213/24, A61K 31/44, A61K 31/505, A61K 31/444, A61K 31/5377, A61P 1/16, A61P 13/12, A61P 11/00, A61P 25/28, A61P 17/06

(54) **NOVEL SULFANONE DERIVATIVE OR PHARMACEUTICALLY ACCEPTABLE SALT THEREOF, AND USE THEREOF**

(30) Priority: 03.06.2022 KR 20220068235
(71) Applicant: Korea Institute of Science and Technology, Seoul 02792 (KR)
(72) Inventor: PARK, Ki Duk, Seoul 02792 (KR); PARK, Jong-Hyun, Seoul 02792 (KR); CHOI, Ji Won, Seoul 02792 (KR); CHO, Sung Jin, Seoul 02792 (KR); KIM, Hyeon Jeong, Seoul 02792 (KR); PARK, Sun Jun, Seoul 02792 (KR); KIM, Yoowon, Seoul 02792 (KR); KIM, Byungeun, Seoul 02792 (KR); KIM, Jushin, Seoul 02792 (KR); LEE, Elijah Hwejin, Seoul 02792 (KR); KIM, Rium, Seoul 02792 (KR); KIM, Jaehwan, Seoul 02792 (KR)
(74) Representative: advotec.
(86) International application number: PCT/KR2023/007673
(87) International publication number: WO 2023/234759

(57) **Abstract**

The present invention relates to a sulfanone derivative or a pharmaceutically acceptable salt thereof, and a composition containing the derivative as an active ingredient for the prevention or treatment of cancer, and the sulfanone derivative of the present invention or a pharmaceutically acceptable salt thereof exhibits an excellent effect on Nrf2 activation and a high solubility and pharmaco-metabolic stability, has anti-inflammatory and antioxidant effects, and improves motor performance and cognitive ability, and thus can be used for the prevention or treatment of diseases induced by a decrease in the Nrf2 activity, preferably liver diseases, kidney diseases, lung diseases, neurodegenerative diseases, mitochondrial myopathy, Friedreich's ataxia, corneal endothelial cell loss, psoriasis, and the like.

## Description

### TECHNICAL FIELD

The present disclosure relates to a novel sulfanone derivative or a pharmaceutically acceptable salt thereof, a method for preparing the same, an Nrf2 activator including the same as an active ingredient, and a pharmaceutical composition for preventing or treating a disease induced by decreased Nrf2 activity.

### BACKGROUND ART

A nuclear factor erythroid-derived 2-related factor 2 (NRF2) is a Cap'n'Collar family basic region-leuzine zipper transcription factor that binds to the Antioxidant Response Element (ARE) sequence, a region involved in the expression of various genes that protect cells, and induces the transcription of these genes. Although ARE inducers do not induce the expression of all antioxidant enzymes, it was revealed through studies using Nrf2-null mice, from which this transcription factor is artificially knocked out, that the expression of antioxidant enzymes is induced by the ARE-mediated activation of Nrf2.

Nrf2 is negatively regulated by Kelch-like ECH associated protein 1 (Keap1). In the absence of oxidative stress, Nrf2 is ubiquitinated through binding to Keap1, and thus is degraded by the proteasome. However, in the presence of oxidative stress, the binding to NRF2 is separated through the modification of the cysteine residue of Keap1, and Nrf2 is moved into the nucleus to bind to ARE, thereby increasing the transcription of various antioxidant genes in the promoter region.

Compounds of various structures that induce the transcription of protective genes through the Nrf2-Keap1 pathway have been discovered in natural products, foods, metabolites, organic synthetic compounds, and the like. All compounds known so far are electrophilic compounds capable of reacting with cysteine of Keap1, or are those that are converted into electrophilic compounds through intracellular metabolic processes. Such electrophilic compounds or reactive oxygen species react with cysteine residue of Keap1 to oxidize or covalently bond the thiol group, thereby causing a change in the structure of the KEAP1. Nrf2, which is separated due to the change in cysteine residue of Keap1, moves to the nucleus and binds to ARE, thereby inducing the expression of antioxidant enzymes. It has been reported that Nrf2 activators can prevent the development of degenerative brain diseases through a chemical defense mechanism that defends against oxidative stress by pre-activating the Nrf2-Keap1 pathway using electrophilic compounds.

Sulforaphane (1-isothiocyanato-4-methylsulfinylbutane), a drug that can react with the cysteine residue of Keap1, is mainly present in cruciferous plants such as broccoli and cabbage, and activates Nrf2 to protect dopaminergic neurons from oxidative stress. However, sulforaphane can induce cytotoxicity by non-selectively denaturing various intracellular proteins having thiol groups, and has the disadvantage of very low blood-brain barrier permeability. In addition, since it disappears within 1 hour when administered to the body, the effect of inducing Nrf2 activation is not maintained. When administered at a high concentration such that constant activity is exhibited, sulforaphane causes cytotoxicity.

Therefore, there is a need to develop a novel Nrf2 activator capable of overcoming the problems of the existing Nrf2 activators. In this context, the present inventors confirmed that a novel sulfanone derivative or a pharmaceutically acceptable salt thereof provides an excellent Nrf2 activation effect, has high solubility and drug metabolic stability, has anti-inflammatory and antioxidant effects, and improves exercise ability and cognitive ability to create the present disclosure.

### DISCLOSURE OF THE INVENTION

### TECHNICAL GOALS

The technical goal to be achieved by the present disclosure is to provide a sulfanone derivative or a pharmaceutically acceptable salt thereof.

An additional technical goal to be achieved by the present disclosure is to provide a composition for preventing, improving, or treating a disease induced by decreased Nrf2 activity, including the sulfanone derivative or a pharmaceutically acceptable salt thereof as an active ingredient.

An additional technical goal to be achieved by the present disclosure is to provide a method of preparing the sulfanone derivative or a pharmaceutically acceptable salt thereof.

However, the technical goals to be achieved by the present disclosure are not limited to those mentioned above, and other goals not mentioned will be clearly understood by one of ordinary skill in the art from the following description.

### TECHNICAL SOLUTIONS

In order to achieve the above mentioned goals, the present disclosure provides a sulfanone derivative represented by the following [Chemical Formula 1] or a racemate, isomer, solvate or pharmaceutically acceptable salt thereof:

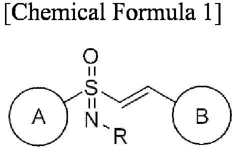

In the above [Chemical Formula 1],
A and B are each independently phenyl or pyridine;
R is hydrogen, a cyano group, a trimethylsilyl group (Trimethylsilane, TMS), or a methyl group;
wherein each of A and B is unsubstituted or substituted with any one or more substituents selected from the group consisting of a hydroxy group, a halogen group, a cyano group, a nitro group, a C₁-C₇ alkyl group, and a C₁-C₇ alkoxy group;
wherein one or more hydrogen of the alkyl group or alkoxy group may be unsubstituted or substituted with any one or more substituent selected from the group consisting of a hydroxy group, a cyano group, a nitro group, and a morpholine group.

In an embodiment of the present disclosure, the sulfanone derivative represented by [chemical formula 1] may be any one selected from the group consisting of the following compounds:
(1) (E)-(2-fluorophenyl)(imino)(2-(pyridin-2-yl)vinyl)-λ⁶-sulfanone;
(2) (E)-imino(4-methoxyphenyl)(2-(pyridin-2-yl)vinyl)-λ⁶-sulfanone;
(3) (E)-(2-chlorophenyl)(2-(3-fluoropyridin-2-yl)vinyl)(imino)-λ⁶-sulfanone;
(4) (E)-(2-chlorophenyl)(2-(3-chloropyridin-2-yl)vinyl)(imino)-λ⁶-sulfanone;
(5) (E)-(2-fluorophenyl)(2-(3-fluoropyridin-2-yl)vinyl)(imino)-λ⁶-sulfanone;
(6) (E)-(2-(3-chloropyridin-2-yl)vinyl)(2-fluorophenyl)(imino)-λ⁶-sulfanone;
(7) (E)-(2-(3-fluoropyridin-2-yl)vinyl)(imino)(3-methoxyphenyl)-λ⁶-sulfanone;
(8) (E)-(2-(3-chloropyridin-2-yl)vinyl)(imino)(2-methoxyphenyl)-λ⁶-sulfanone;
(9) (E)-(2-(3-chloropyridin-2-yl)vinyl)(imino)(3-methoxyphenyl)-λ⁶-sulfanone;
(10) (E)-(2-(3-chloropyridin-2-yl)vinyl)(imino)(4-methoxyphenyl)-λ⁶-sulfanone;
(11) (E)-imino(2-methoxyphenyl)(2-(3-(trifluoromethyl)pyridin-2-yl)vinyl)-λ⁶-sulfanone;
(12) (E)-(2-chlorophenyl)(imino)(2-(pyridin-3-yl)vinyl)-λ⁶-sulfanone;
(13) (E)-(2-chlorophenyl)(2-(2-chloropyridin-3-yl)vinyl)(imino)-λ⁶-sulfanone;
(14) (E)-(2-chlorophenyl)(imino)(2-(2,4,6-trichloropyrimidin-5-yl)vinyl)-λ⁶-sulfanone;
(15) (E)-(2-chlorostyryl)(imino)(pyridin-2-yl)-λ⁶-sulfanone;
(16) (E)-(3-chlorostyryl)(imino)(pyridin-2-yl)-λ⁶-sulfanone;
(17) (E)-imino(pyridin-2-yl)(2-(trifluoromethyl)styryl)-λ⁶-sulfanone;
(18) (E)-imino(pyridin-2-yl)(2-(trifluoromethoxy)styryl)-λ⁶-sulfanone;
(19) (E)-(2,6-dichlorostyryl)(imino)(pyridin-2-yl)-λ⁶-sulfanone;
(20) (E)-(4-chlorostyryl)(imino)(pyridin-2-yl)-λ⁶-sulfanone;
(21) (E)-(5-chloropyridin-2-yl)(2-chlorostyryl)(imino)-λ⁶-sulfanone;
(22) (E)-(2,6-difluorostyryl)(imino)(pyridin-2-yl)-λ⁶-sulfanone;
(23) (E)-imino(pyridin-2-yl)(2,4,6-trifluorostyryl)-λ⁶-sulfanone;
(24) (E)-(2-fluorostyryl)(imino)(pyridin-2-yl)-λ⁶-sulfanone;
(25) (E)-imino(3-methoxypyridin-2-yl)(2-(trifluoromethyl)styryl)-λ⁶-sulfanone;
(26) (E)-imino(4-methoxypyridin-2-yl)(2-(trifluoromethyl)styryl)-λ⁶-sulfanone;
(27) (E)-imino(4-methoxypyridin-2-yl)(2-(3-(trifluoromethyl)pyridin-2-yl)vinyl)-λ⁶-sulfanone;
(28) (E)-(2-(3-chloropyridin-2-yl)vinyl)(imino)(4-methoxypyridin-2-yl)-λ⁶-sulfanone;
(29) (E)-(2-(3-fluoropyridin-2-yl)vinyl)(imino)(4-methoxypyridin-2-yl)-λ⁶-sulfanone;
(30) (E)-(2-chlorostyryl)(imino)(4-methoxypyridin-2-yl)-λ⁶-sulfanone;
(31) (E)-(2-fluorostyryl)(imino)(4-methoxypyridin-2-yl)-λ⁶-sulfanone;
(32) (E)-imino(4-methoxypyridin-2-yl)(2-(3-methoxypyridin-2-yl)vinyl)-λ⁶-sulfanone;
(33) (E)-(2-(3-chloropyridin-2-yl)vinyl)(imino)(3-methoxypyridin-2-yl)-λ⁶-sulfanone;
(34) (E)-imino(3-methoxypyridin-2-yl)(2-(3-(trifluoromethyl)pyridin-2-yl)vinyl)-λ⁶-sulfanone;
(35) (E)-(2-(3-chloropyridin-2-yl)vinyl)(imino)(5-methoxypyridin-2-yl)-λ⁶-sulfanone;
(36) (E)-(2-(3-fluoropyridin-2-yl)vinyl)(imino)(5-methoxypyridin-2-yl)-λ⁶-sulfanone;
(37) (E)-imino(5-methoxypyridin-2-yl)(2-(3-(trifluoromethyl)pyridin-2-yl)vinyl)-λ⁶-sulfanone;
(38) (E)-(3-chloropyridin-2-yl)(2-chlorostyryl)(imino)-λ⁶-sulfanone;
(39) (E)-(2-chlorostyryl)(imino)(5-methoxypyridin-2-yl)-λ⁶-sulfanone;
(40) (E)-(2-chlorostyryl)(3-fluoropyridin-2-yl)(imino)-λ⁶-sulfanone;
(41) (E)-(3-chloropyridin-4-yl)(2-chlorostyryl)(imino)-λ⁶-sulfanone;
(42) (E)-(5-chloropyridin-2-yl)(2-chlorostyryl)(imino)-λ⁶-sulfanone;
(43) (E)-(2-chlorostyryl)(imino)(3-methoxypyridin-2-yl)-λ⁶-sulfanone;
(44) (E)-(2-fluorostyryl)(imino)(3-methoxypyridin-2-yl)-λ⁶-sulfanone;
(45) (E)-(4-chloropyridin-2-yl)(2-chlorostyryl)(imino)-λ⁶-sulfanone;
(46) (E)-(2-chlorostyryl)(3-fluoropyridin-4-yl)((trimethylsilyl)imino)-λ⁶-sulfanone;
(47) (E)-(2-chlorostyryl)(3-fluoropyridin-4-yl)(imino)-λ⁶-sulfanone;
(48) (E)-(4-chloropyridin-3-yl)(2-chlorostyryl)(imino)-λ⁶-sulfanone;
(49) (E)-(2-(3-chloropyridin-2-yl)vinyl)(imino)(pyridin-2-yl)-λ⁶-sulfanone;
(50) (E)-imino(2-(3-methoxypyridin-2-yl)vinyl)(pyridin-2-yl)-λ⁶-sulfanone;
(51) (E)-(2-(3-fluoropyridin-2-yl)vinyl)(imino)(pyridin-2-yl)-λ⁶-sulfanone;
(52) (E)-imino(pyridin-2-yl)(2-(3-(trifluoromethyl)pyridin-2-yl)vinyl)-λ⁶-sulfanone;
(53) (E)-(2-(3-fluoropyridin-2-yl)vinyl)(imino)(5-(3-morpholinopropoxy)pyridin-2-yl)-λ⁶-sulfanone;
(54) (E)-imino(5-methoxypyridin-2-yl)(2-(trifluoromethyl)styryl)-λ⁶-sulfanone;
(55) (E)-(2-fluorostyryl)(imino)(5-methoxypyridin-2-yl)-λ⁶-sulfanone;
(56) (E)-(2-fluorostyryl)(5-methoxypyridin-2-yl)(methylimino)-λ⁶-sulfanone;
(57) (E)-N-((2-chlorostyryl)(2-methoxyphenyl)(oxo)-λ⁶-sulfanylidene)cyanamide; and
(58) (E)-N-((2-(3-chloropyridin-2-yl)vinyl)(2-methoxyphenyl)(oxo)-λ⁶-sulfaneylidene)cyanamide.

In another embodiment of the present disclosure, the sulfanone derivative may activate nuclear factor erythroid-derived 2-related factor 2 (NRF2).

In another embodiment of the present disclosure, the pharmaceutically acceptable salts of the sulfanone derivatives may be any one or more selected from the group consisting of hydrochloride, bromate, sulfate, phosphate, nitrate, citrate, acetate, lactate, tartrate, maleate, gluconate, succinate, formate, trifluoroacetate, oxalate, fumarate, glutarate, adipate, methanesulfonate, benzenesulfonate, paratoluenesulfonate, camphorsulfonate, sodium salt, potassium salt, lithium salt, calcium salt, and magnesium salt.

In addition, the present disclosure provides a pharmaceutical composition for preventing or treating a disease induced by decreased Nrf2 activity, including the sulfanone derivative or a pharmaceutically acceptable salt thereof as an active ingredient.

In an embodiment of the present disclosure, the disease induced by decreased Nrf2 activity may be any one or more selected from the group consisting of liver disease, kidney disease, pulmonary disease, neurodegenerative disease, mitochondrial myopathy, Friedreich's ataxia, corneal endothelial cell loss, and psoriasis, but is not limited thereto.

The liver disease may include alcoholic liver disease, non-alcoholic liver disease, non-alcoholic fatty liver disease (NAFLD), chronic liver injury, viral hepatitis, hepatocellular carcinoma and the like, but is not limited thereto.

The kidney disease may include diabetic nephropathy, focal segmental glomerulosclerosis, renal fibrosis, lupus-like autoimmune nephritis, chronic kidney disease (CKD), hypertensive kidney disease, etc., but is not limited thereto.

The pulmonary disease may include, but is not limited to, chronic obstructive pulmonary disease (COPD), pulmonary emphysema, ventilation-associated lung injury, acute lung injury (ALI), acute respiratory distress syndrome (ARDS), pulmonary artery hypertension (PAH), right heart failure induced by the pulmonary artery hypertension, etc.

The neurodegenerative disease may include, but is not limited to, Parkinson's disease (PD), Alzheimer's disease (AD), Huntington's disease, Lou Gehrig's disease, epilepsy, depression, insomnia, anxiety, multiple sclerosis (MS), etc.

In another embodiment of the present disclosure, the pharmaceutical composition may further include any one or more additional components selected from the group consisting of a pharmaceutically acceptable carrier, excipient, diluent, stabilizer and preservative; in addition to the sulfanone derivative or a pharmaceutically acceptable salt thereof.

In another embodiment of the present disclosure, the pharmaceutical composition may have any one or more dosage form selected from the group consisting of powders, granules, tablets, capsules, and injections.

In addition, the present disclosure provides a method for preventing or treating a disease induced by decreased Nrf2 activity, including a step of administering the sulfanone derivative or a pharmaceutically acceptable salt thereof to an individual.

In addition, the present disclosure provides the use of the sulfanone derivative or a pharmaceutically acceptable salt thereof for the manufacture of a medicament for preventing or treating a disease induced by decreased Nrf2 activity.

In addition, the present disclosure provides a cosmetic composition for preventing or improving a disease induced by decreased Nrf2 activity, including the sulfanone derivative or a cosmetically acceptable salt thereof as an active ingredient.

In addition, the present disclosure provides a food composition for preventing or improving a disease induced by decreased Nrf2 activity, including the sulfanone derivative or a food-related acceptable salt thereof as an active ingredient.

In addition, the present disclosure provides a feed composition for preventing or improving a disease induced by decreased Nrf2 activity, including the sulfanone derivative or a feed-related acceptable salt thereof as an active ingredient.

In addition, the present disclosure provides a method for preparing the sulfanone derivative or a pharmaceutically acceptable salt thereof, including the following steps:
(1) preparing a compound represented by [Chemical Formula 3] from a compound represented by [Chemical Formula 2];
(2) preparing a compound represented by [Chemical Formula 4] from the compound represented by [Chemical Formula 3];
(3) preparing a compound represented by [Chemical Formula 5] from the compound represented by [Chemical Formula 4]; and
(4) preparing a compound represented by [Chemical Formula 1] by adding a compound represented by [Chemical Formula 6] to the compound represented by [Chemical Formula 5]. 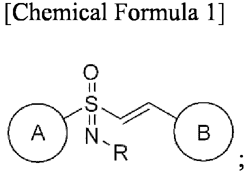 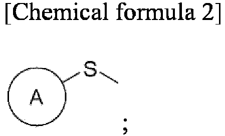 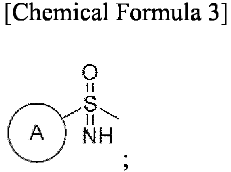 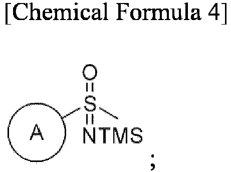 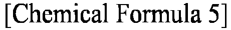 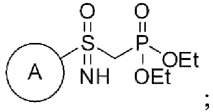 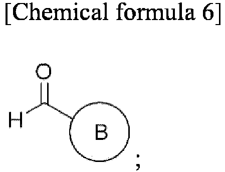 (in Chemical Formulae 1 to 6,
   A and B are each independently phenyl or pyridine;
   R is hydrogen, a trimethylsilyl group (Trimethylsilane, TMS), or a methyl group;
   wherein each of A and B is unsubstituted or substituted with any one or more substituents selected from the group consisting of a hydroxy group, a halogen group, a cyano group, a nitro group, a C₁-C₇ alkyl group, and a C₁-C₇ alkoxy group;
   wherein one or more hydrogen of the alkyl group or alkoxy group may be unsubstituted or substituted with any one or more substituent selected from the group consisting of a hydroxy group, a cyano group, a nitro group, and a morpholine group.

In an another embodiment of the present disclosure, in step (1), the compound represented by [Chemical Formula 2] may be dissolved in methanol (MeOH), and then ammonium carbonate ((NH₄)₂CO₃) and diacetoxyiodobenzene (PhI(OAc)₂) may be sequentially added.

In another embodiment of the present disclosure, in step (2), hexamethyldisilazane (HMDS) may be added to the compound represented by [Chemical Formula 3], and then stirring under reflux may be performed.

In another embodiment of the present disclosure, in step (3), the compound represented by [Chemical Formula 4] may be dissolved in tetrahydrofuran (THF), and then n-butyllithium (n-BuLi) and diethyl chlorophosphate may be sequentially added.

### EFFECTS OF THE INVENTION

The present disclosure relates to a sulfanone derivative or a pharmaceutically acceptable salt thereof, and a composition containing the derivative as an active ingredient for the prevention or treatment of cancer, and the sulfanone derivative of the present disclosure or a pharmaceutically acceptable salt thereof exhibits an excellent Nrf2 activation effect and a high solubility and pharmaco-metabolic stability, has anti-inflammatory and antioxidant effects, and improves motor performance and cognitive ability, and thus can be used for the prevention or treatment of diseases induced by a decrease in Nrf2 activity, preferably liver diseases, kidney diseases, lung diseases, neurodegenerative diseases, mitochondrial myopathy, Friedreich's ataxia, corneal endothelial cell loss, psoriasis, and the like.

The effects of the present disclosure are not limited to those mentioned above, and other effects not mentioned may be clearly understood by a person skilled in the art from the following descriptions.

### BRIEF DESCRIPTION OF THE DRAWINGS

Figure 1 shows the Nrf2 activation activity and cytotoxicity according to the concentration of Compound 1-4 of the present disclosure; and the positive control group, DMF and Bardoxolone-methyl (CDDO-Me).
Figure 2 shows the NO inhibitory effect of Compounds 1-1 to 1-10, 1-12 to 1-20 of the present disclosure, the negative control group, and DMF (the positive control group).
Figure 3 shows the expression of antioxidant enzymes when cells were treated with Compound 1-4 of the present disclosure at different concentrations.
Figure 4 shows the effect of protecting cells in the Parkinson's disease model MPTP mouse when cells were treated with Compound 1-4 of the present disclosure. Figure 4a shows the timeline of the animal experiment; Figure 4b is a diagram illustrating specific test methods; Figure 4c shows the results of a vertical grid test; and Figure 4d shows the results of a coat hanger test.
Figure 5 shows the results of immunohistochemistry (IHC) staining to confirm the effect on dopaminergic neurons in a Parkinson's disease model MPTP mouse when tissues were treated with Compound 1-4 of the present disclosure.
Figures 6a-6c show the results of a Morris water maze test in an Alzheimer's disease model APP/PS1 mouse when the mouse was treated with Compound 1-4 of the present disclosure. Figure 6a shows the results of an acquisition/reversal test; Figure 6b shows the results of a probe test; and Figure 6c shows the results of a reversal probe test.
Figure 7a and 7b show the effects of enhancing memory and cognitive function in an Alzheimer's disease model APP/PS1 mouse when the mouse was treated with Compound 1-4 of the present disclosure. Figure 7a shows the results of a Y-maze test; and Figure 7b shows the results of a passive avoidance test.

### BEST MODE FOR CARRYING OUT THE INVENTION

As a result of intensive research on a sulfanone derivative or a pharmaceutically acceptable salt thereof, the present inventors confirmed that the sulfanone derivative or a pharmaceutically acceptable salt thereof exhibits an excellent Nrf2 activation effect and a high solubility and pharmaco-metabolic stability, has anti-inflammatory and antioxidant effects, and improves motor performance and cognitive ability.

As shown by the above mentioned results, the present disclosure may provide a sulfanone derivative represented by the following [Chemical Formula 1] or a pharmaceutically acceptable salt thereof, wherein the sulfanone derivative may include a racemate, an isomer, or a solvate thereof:

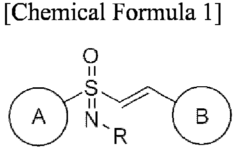

In the above [Chemical Formula 1],
A and B are each independently phenyl or pyridine;
R is hydrogen, a cyano group, a trimethylsilyl group (Trimethylsilane, TMS), or a methyl group;
wherein each of A and B is unsubstituted or substituted with any one or more substituents selected from the group consisting of a hydroxy group, a halogen group, a cyano group, a nitro group, a C₁-C₇ alkyl group, and a C₁-C₇ alkoxy group;
wherein one or more hydrogen of the alkyl group or alkoxy group may be unsubstituted or substituted with any one or more substituent selected from the group consisting of a hydroxy group, a cyano group, a nitro group, and a morpholine group.

As used herein, the term "substitution" refers to a reaction in which an atom or atomic group contained in a molecule of a compound is replaced with another atom or atomic group.

As used herein, the term "alkyl group" means a monovalent linear or branched or cyclic saturated hydrocarbon residue having 1 to 20 carbon atoms and consisting only of carbon and hydrogen atoms. Examples of such alkyl groups include, but are not limited to, methyl, ethyl, propyl, isopropyl, butyl, isobutyl, 2-butyl, 3-butyl, pentyl, n-hexyl, cyclobutyl, cyclopentyl, cyclohexyl, etc.

As used herein, the term "alkoxy group" means an alkyl group bonded to oxygen (-OR). Examples of such alkoxy groups include, but are not limited to, a methoxy group, an ethoxy group, a propoxy group, a butoxy group, and the like.

As used herein, the "halogen group" may mean fluorine (F), chloride (Cl), bromine (Br), or iodine (I), etc.

As used herein, the term "pharmaceutically acceptable salt" means a formulation of a compound that does not cause serious irritation to the organism to which the compound is administered and does not impair the biological activity and properties of the compound. The pharmaceutically acceptable salt of the compound of the present disclosure may be obtained by reacting the compound of the present disclosure with inorganic acids such as hydrochloric acid, hydrobromic acid, sulfuric acid, nitric acid, and phosphoric acid; sulfonic acids such as methanesulfonic acid, ethanesulfonic acid, and p-toluenesulfonic acid; organic carboxylic acids such as tartaric acid, formic acid, citric acid, acetic acid, trichloroacetic acid, trifluoroacetic acid, capric acid, isobutanoic acid, malonic acid, succinic acid, phthalic acid, gluconic acid, benzoic acid, lactic acid, fumaric acid, maleic acid, and salicylic acid. Also, the pharmaceutically acceptable salt of the compound of the present disclosure may be obtained by reacting the compound of the present disclosure with a base to form salts such as ammonium salt, alkali metal salt such as sodium or potassium salt, alkaline earth metal salt such as calcium or magnesium salt, salts of organic bases such as dicyclohexylamine, N-methyl-D-glucamine, tris(hydroxymethyl)methylamine and salts of amino acids such as arginine and lysine. Furthermore, the sulfanone derivative or a pharmaceutically acceptable salt thereof may include not only pharmaceutically acceptable salts, but also all salts, hydrates and solvates that may be prepared by conventional methods.

The sulfanone derivative of the present disclosure or a pharmaceutically acceptable salt thereof has an effect of activating Nrf2, and thus it may be used as a pharmaceutical composition for preventing or treating a disease induced by decreased Nrf2 activity, including the sulfanone derivative or a pharmaceutically acceptable salt thereof as an active ingredient.

The term "Nuclear factor erythroid-derived 2-related factor 2 (Nrf2)" of the present disclosure, refers to a transcription factor encoded by the NFE2L2 gene in humans, which is a basic leucine zipper protein (bZIP) that regulates the expression of antioxidant proteins that protect against oxidative damage caused by damage or inflammation. Accordingly, drugs that activate the Nrf2 pathway are being studied to treat diseases caused by oxidative stress.

As used herein, the term "prevention" refers to all actions that inhibit or delay the occurrence, spread, or recurrence of disease by the administration of the composition of the present disclosure, and the term "treatment" refers to all actions by which the symptoms of the disease are ameliorated or beneficially changed by the administration of a composition of the present disclosure.

As described above, Nrf2, which is targeted by the compound of the present disclosure, is reportedly involved in the prevention and treatment of various diseases through an antioxidant defense mechanism by its activation. For example, non-limiting examples of diseases induced by decreased Nrf2 activity that may be prevented or treated by administering the composition of the present disclosure include liver diseases such as alcoholic liver disease, non-alcoholic liver disease, non-alcoholic fatty liver disease (NAFLD), chronic liver injury, viral hepatitis, and hepatocellular carcinoma; kidney diseases such as diabetic nephropathy, focal segmental glomerulosclerosis, renal fibrosis, lupus-like autoimmune nephritis, chronic kidney disease (CKD), and hypertensive kidney disease; pulmonary diseases such as chronic obstructive pulmonary disease (COPD), pulmonary emphysema, ventilation-associated lung injury, acute lung injury (ALI), acute respiratory distress syndrome (ARDS), pulmonary artery hypertension (PAH), and right heart failure induced by the pulmonary artery hypertension; Neurodegenerative diseases such as Parkinson's disease (PD), Alzheimer's disease (AD), Huntington's disease, Lou Gehrig's disease, epilepsy, depression, insomnia, anxiety, and multiple sclerosis (MS); Mitochondrial myopathy; Friedreich's ataxia; Corneal endothelial cell loss; Psoriasis, etc.

As used herein, the term "pharmaceutical composition" means a composition manufactured for the prevention or treatment of a disease, which may be formulated and used in various forms according to conventional methods. For example, the composition may be formulated in oral dosage forms such as powders, granules, tablets, capsules, suspensions, emulsions, syrups, and the like, and may be formulated and used in the form of external preparations, suppositories, and sterile injectable solutions.

As used herein, the phrase "including as an active ingredient" means that the ingredient is included in an amount necessary or sufficient to realize a desired biological effect. In practical application, the amount to be included as an active ingredient may be determined to be an amount to treat the disease of interest and not cause other toxicities. For example, the amount may vary depending on a variety of factors, such as the disease or condition being treated, the form of the composition being administered, the size of the individual, or the severity of the disease or condition. A person of ordinary skill in the art to which the present disclosure pertains will be able to determine empirically the effective amount of an individual composition without undue experimentation.

In addition, the pharmaceutical composition of the present disclosure may, depending on each formulation, further include one or more pharmaceutically acceptable carriers, in addition to the active ingredients described above.

The pharmaceutically acceptable carrier may be saline, sterile water, Ringer's solution, buffered saline, dextrose solution, maltodextrin solution, glycerol, ethanol, and a mixture of one or more of these components, and may further include other conventional additives, such as antioxidants, buffers, bacteriostatic agents, and the like. In addition, the composition may be formulated in injectable forms, such as aqueous solutions, suspensions, emulsions, etc., pills, capsules, granules or tablets by additionally adding diluents, dispersants, surfactants, binders and lubricants. Further, the composition may be preferably formulated depending on the disease or ingredient, by any suitable method in the art, or as disclosed in Remington's Pharmaceutical Science (Mack Publishing Company, Easton PA).

In addition, the present disclosure may provide a method for preventing, treating, and/or diagnosing a disease induced by decreased Nrf2 activity, including a step of administering the sulfanone derivative or a pharmaceutically acceptable salt thereof to a subject.

As used herein, the term "individual" refers to, but is not limited to, any mammal, such as a domestic animal or human, preferably a human, in need of prevention or treatment of a cancer.

As used herein, the term "individual" refers to, but is not limited to, any mammal, such as a domestic animal or human, preferably a human, that has developed or may develop a disease induced by decreased Nrf2 activity and requires prevention or treatment of the disease. By administering the pharmaceutical composition of the present disclosure to a subject, the disease may be effectively prevented or treated. In addition, the pharmaceutical composition of the present disclosure exhibits a therapeutic effect through Nrf2 activation, and thus it may exhibit a synergistic effect when administered in combination with an existing therapeutic agent.

As used herein, the term "administration" means providing a predetermined substance to a patient by any appropriate method. The pharmaceutical composition of the present disclosure may be formulated in various forms for administration to an individual, and a representative of the form for parenteral administration is an injectable form, preferably an isotonic aqueous solution or suspension. The injectable form may be prepared by techniques known in the art using a suitable dispersing or wetting agent and a suspending agent. For example, each component may be dissolved in saline or a buffer solution and the mixture may be formulated for injection. The forms for oral administration include, for example, swallowable tablets, buccal tablets, troches, capsules, elixirs, suspensions, syrups, and wafers, which may include, in addition to an active ingredient, a diluent (e.g., lactose, dextrose, sucrose, mannitol, sorbitol, cellulose, and/or glycine) and a lubricant (e.g., silica, talc, stearic acid and its magnesium or calcium salt, and/or polyethylene glycol). The tablets may include a binder such as magnesium aluminum silicate, starch paste, gelatin, tragacanth, methylcellulose, sodium carboxymethylcellulose, and/or polyvinylpyrrolidine, and in some instances, may further include disintegrating agents such as starch, agar, alginic acid or its sodium salts, absorbents, colorants, flavoring agents, and/or sweeteners. The formulations may be prepared by conventional mixing, granulating, or coating methods.

The pharmaceutical composition of the present disclosure may additionally contain adjuvants such as preservatives, hydrating agents, emulsification accelerators, salts or buffers for osmotic pressure control, and other therapeutically useful substances, and may be formulated according to a conventional method.

The pharmaceutical composition of the present disclosure may be administered via several routes, including oral, transdermal, subcutaneous, intravenous, and intramuscular, and the dosage of the active ingredient may be adequately selected depending on several factors, including the route of administration, the age, gender, and weight of the patient, and the severity of the patient's disease. The pharmaceutical composition of the present disclosure may be administered in combination with known compounds, which may enhance the desired effect.

The routes of administration of the pharmaceutical composition of the present disclosure can be administered to humans and animals orally or parenterally, for example, intravenously, subcutaneously, intranasally or intraperitoneally. Oral administration also includes sublingual application. Parenteral administration includes injection and drop methods such as subcutaneous injection, intramuscular injection, and intravenous injection.

The pharmaceutical composition of the present disclosure may be administered orally or parenterally in a pharmaceutically effective amount, depending on the intended method, and the term "pharmaceutically effective amount" as used herein means an amount sufficient to treat a disease where a benefit/risk ratio applicable to medical treatment is reasonable and not cause adverse effects. The effective dose level may be determined based on factors including the patient's health condition, severity, activity of the drug, sensitivity to the drug, method of administration, time of administration, route of administration and rate of elimination, duration of treatment, drugs used in combination or concurrently, and other factors well known in the medical field.

In other words, the total effective dose of the sulfanone derivative or a pharmaceutically acceptable salt thereof according to the present disclosure may be administered to a patient in a single dose, or may be administered to a patient in multiple doses over a longer period of time in accordance with a fractionated treatment protocol. The pharmaceutical composition of the present disclosure may vary in the amount of active ingredients depending on the severity of the disease. Further, the effective dose for the patient may be determined considering various factors such as the patient's age, weight, health status, sex, the severity of the disease, and the patient's diet and excretion rate.

The terminology used in the Examples section is used only to describe a specific embodiment and is not intended to limit the invention. The singular form of a word may also have the meaning of the plural form of the word unless the context clearly dictates otherwise. In the present application, the terms "comprise" or "having" and the like refer to a feature, a number, a step, an operation, an element, a component or a combination thereof described in the specification, but do not preclude the possibility that other features, numbers, steps, operations, elements, components, or a combination thereof may be present or added.

Unless defined otherwise, all terms used herein, including technical or scientific terms, have the same meaning as is commonly understood by one of ordinary skill in the art to which this invention belongs. Terms defined in commonly used dictionaries should be interpreted as having a meaning consistent with the meaning that may be understood from the context of the relevant art and are not to be interpreted in an ideal or overly formal sense unless they are explicitly defined in the present application.

Hereinafter, embodiments will be described in detail with reference to the accompanying drawings. However, since various changes may be made to the embodiments, it will be understood that the descriptions of the embodiments do not limit or otherwise restrict the scope of the patent application. With respect to the embodiments, it should be understood that the scope of the rights shall include all modifications, equivalents and substitutes.

In addition, in the description with reference to the accompanying drawings, the same components are given the same reference numerals regardless of the drawing numerals, and any overlapping description of a component has been omitted. With respect to the description of an embodiment, in case where it was determined that including a detailed description of a related known technology may unnecessarily obscure the gist of the embodiment, such detailed description was omitted.

### MODE FOR CARRYING OUT THE INVENTION

### Example 1. Synthesis of cyanosulfanone derivatives

In an embodiment of the present disclosure, the synthesis process of the sulfanone derivative, wherein R in Chemical Formula 1 is cyano, is shown by Reaction Scheme 1 presented below.

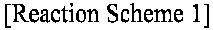

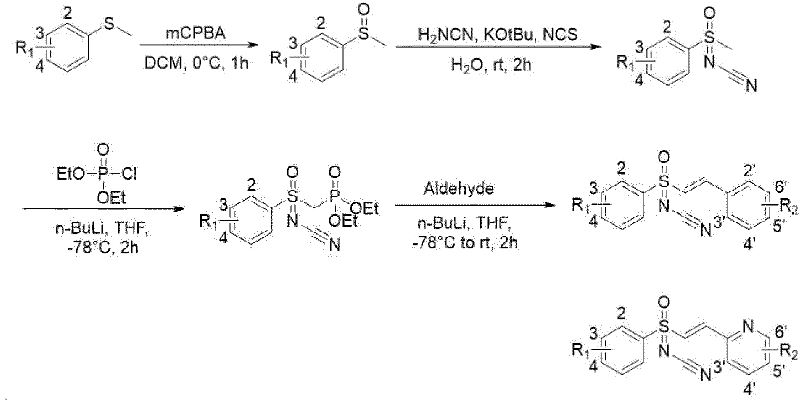

### (1) Synthesis of substituted (methylsulfinyl)benzene by reaction with metachloroperoxybenzoic acid

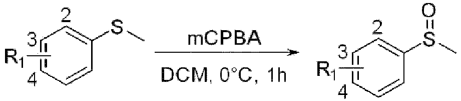

According to the above shown reaction scheme, substituted (methylsulfinyl)benzene was synthesized. Substituted methyl(phenyl)sulfane (1.0 eq) was dissolved in dichloromethane (DCM), then metachloroperoxybenzoic acid (mCPBA, 1.0 eq) was added thereto at 0°C. The reaction mixture was stirred at the same temperature for 1 hour. After the reaction was completed, the reactant was diluted with ethyl acetate (EtOAc), and then was washed with water and brine. The organic layer was dried over anhydrous Na₂SO₄ and was filtered. The solvent was distilled off under reduced pressure. The resulting residue was purified by column chromatography to obtain 2-, 3-, or 4-substituted (methylsulfinyl)benzene.

### (2) Synthesis of substituted N-(methyl(oxo)(phenyl)-λ⁶-sulfaneylidene)cyanamide by reaction with cyanamide

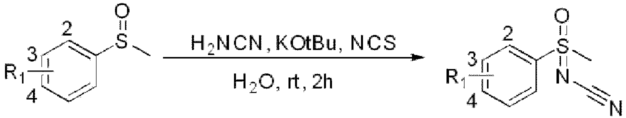

According to the above shown reaction scheme, substituted N-(methyl(oxo)(phenyl)-λ⁶-sulfaneylidene)cyanamide was synthesized. The compound (1.0 eq) synthesized in reaction (1) was fully dissolved in water, and cyanamide (2.0 eq), potassium tert-butoxide (KOtBu, 2.0 eq), and N-chlorosuccinimide (NCS, 2.0 eq) were added thereto. The reaction mixture was stirred at room temperature for 2 hours. After the reaction was completed, the reactant was diluted with ethyl acetate (EtOAc), and then was washed with water and brine. The organic layer was dried over anhydrous Na₂SO₄ and was filtered. The solvent was distilled off under reduced pressure. The resulting residue was purified by column chromatography to obtain 2-, 3-, or 4-substituted *N*-(methyl(oxo)(phenyl)-λ⁶-sulfaneylidene)cyanamide.

### (3) Synthesis of substituted diethyl((N-cyanophenylsulfonimidoyl)methyl)phosphonate by reaction with diethylchlorophosphate

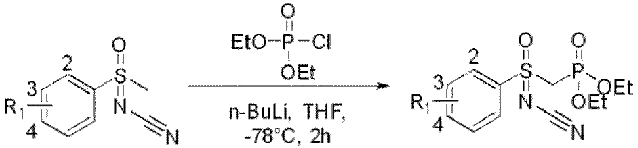

According to the above shown reaction scheme, substituted diethyl((*N-*cyanophenylsulfonimidoyl)methyl)phosphonate was synthesized. The compound (1.0 eq) synthesized in reaction (2) was dissolved in anhydrous tetrahydrofuran (THF), and then the reaction mixture was cooled to -78°C using acetone and dry ice. Then, n-butyllithium (2.2 eq, 2.0 M cyclohexane solution) was added dropwise using acetone and dry ice at -78°C. The reaction solution was stirred at the same temperature for 30 minutes. Then, diethyl chlorophosphate (1.5 eq) was added at the same temperature. Then, the reaction mixture was stirred at room temperature for 2 hours. After the reaction was completed, the reactant was diluted with ethyl acetate (EtOAc), and then was washed with water and brine. The organic layer was dried over anhydrous Na₂SO₄ and was filtered. The solvent was distilled off under reduced pressure. The resulting residue was purified by column chromatography to obtain 2-, 3-, or 4-substituted diethyl((*N*-cyanophenylsulfonimidoyl)methyl)phosphonate.

### (4) Synthesis of substituted (E)-N-(oxo(phenyl)(2-(pyridin-2-yl)vinyl)-λ⁶-sulfaneylidene)cyanamide by reaction with picolinaldehyde derivatives

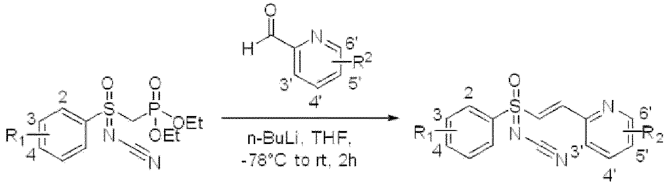

According to the above shown reaction scheme, substituted (*E*)-*N*-(oxo(phenyl)(2-(pyridin-2-yl)vinyl)-λ⁶-sulfaneylidene)cyanamide was synthesized. The compound (1.0 eq) synthesized in reaction (3) was dissolved in anhydrous THF, and then the reaction mixture was cooled to -78°C using acetone and dry ice. Then, n-BuLi (1.2 eq, 2.0 M cyclohexane solution) was slowly added dropwise to the solution, and then the mixture was stirred for 1 hour. To this mixture, benzaldehyde derivatives (1.2 eq) were added and reacted for another 1 hour. If the reaction was not completed as confirmed by TLC, the reaction was allowed to proceed for another 30 minutes at room temperature. The reaction mixture was quenched with a small amount of water. The mixture was extracted with water and 10% MeOH/MC. The organic layer was dried over anhydrous Na₂SO₄ to remove a small amount of water. The solvent was distilled off under reduced pressure. The resulting residue was separated and purified by column chromatography to obtain 2-, 3-, or 4-substituted (*E*)-imino(phenyl)(styryl)- λ⁶-sulfanone derivatives.

### Example 1.1. Synthesis of (E)-N-((2-(3-chloropyridin-2-yl)vinyl)(2-methoxyphenyl)(oxo)-λ⁶-sulfaneylidene)cyanamide (Chemical Formula 1-57)

### 1.1.1. Synthesis of 1-methoxy-2-(methylsulfinyl)benzene

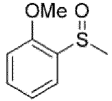

Using reaction (1), (2-methoxyphenyl)(methyl)sulfane (1.00 g, 6.48 mmol) was fully dissolved in DCM and the reaction mixture was reacted with 70-75% mCPBA (1.60 g, 6.48 mmol) to synthesize 1-methoxy-2-(methylsulfinyl)benzene as a pale yellow oil (0.99 g, 90%); *R_{f}* = 0.44 (EtOAc 100%); ¹H NMR (400 MHz, CDCl₃) *δ* 7.83 (dd, 1.6, 7.7 Hz, Ar**H**), 7.46 (td, 1.7, 7.9 Hz, ArH), 7.20 (td, 0.8, 7.6 Hz, Ar**H**), 6.93 (d, 8.1 Hz, Ar**H**), 3.89 (s, OC**H₃**), 2.78 (s, **SCH₃**).

### 1.1.2. Synthesis of N-((2-methoxyphenyl)(methyl)(oxo)-λ⁶-sulfaneylidene)cyanamide

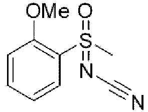

Using reaction (2), 1-methoxy-2-(methylsulfinyl)benzene (0.99 g, 5.81 mmol) was fully dissolved in water, and cyanamide (0.49 g, 11.63 mmol), KOtBu (1.31 g, 11.63 mmol), and NCS (1.55 g, 11.63 mmol) were added thereto to synthesize *N*-((2-methoxyphenyl)(methyl)(oxo)-λ⁶-sulfaneylidene)cyanamide as a transparent oil (1.03 g, 84%); R*_{f}* = 0.30 (*n*-hexane 1 : EtOAc 3); ¹H NMR (400 MHz, DMSO-*d*₆) *δ* 7.90-7.84 (m, 2Ar**H**), 7.44 (d, 8.4 Hz, Ar**H**), 7.29 (t, 7.6 Hz, Ar**H**), 4.00 (s, OC**H₃**), 3.67 (s, SC**H₃**).

### 1.1.3. Synthesis of diethyl ((N-cyano-2-methoxyphenylsulfonimidoyl)methyl)phosphonate

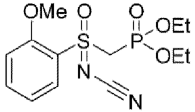

Using reaction (3), *N*-((2-methoxyphenyl)(methyl)(oxo)-λ⁶-sulfaneylidene)cyanamide (1.03 g, 4.90 mmol) was fully dissolved in THF, and then the solution was reacted with diethyl chlorophosphate (1.06 ml, 7.35 mmol) and 2.0 M *n* -BuLi cyclohexane solution (5.39 ml, 10.78 mmol) to synthesize diethyl ((*N*-cyano-2-methoxyphenylsulfonimidoyl)methyl)phosphonate (0.91 g, 53%) as an yellow solid; R*_{f}* = 0.29 (EtOAc 100%); ¹H NMR (400 MHz, CDCl₃) *δ 8.00* (dd, 1.5, 8.0 Hz, Ar**H**), 7.74 (td, 1.6, 8.1 Hz, Ar**H**), 7.22 (t, 7.4 Hz, Ar**H**), 7.14 (d, 8.3 Hz, Ar**H**), 4.37- 4.21 (m, SC**H₂**P), 4.16-4.06 (m, (POC**H₂**CH₃)**₂**, OC**H₃**), 1.27 (dt, 7.2, 36.9 Hz, P(OCH₂C**H₃**)**₂**).

### 1.1.4. Synthesis of (E)-N-((2-chlorostyryl)(2-methoxyphenyl)(oxo)-λ⁶-sulfanylidene)cyanamide (Chemical Formula 1-57)

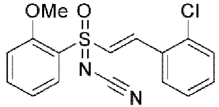

Using reaction (4), diethyl ((*N*-cyano-2-methoxyphenylsulfonimidoyl)methyl)phosphonate (0.20 g, 0.58 mmol), 2.0 M n-BuLi solution in cyclohexane (0.32 mL, 0.64 mmol), and 3-chloropicolinaldehyde (0.07 ml, 0.64 mmol) were reacted to synthesize compound 1-57 as a white solid (0.08 g, 39%): R*_{f}* = 0.46 (n-hexane 1 : EtOAc 3); ¹H NMR (DMSO-*d*₆, 400 MHz) *δ* 8.12-8.02 (m, (E)-isomeric **H**, 2Ar**H**), 7.92-7.86 (m, (*E*)-isomeric **H**, Ar**H**), 7.63 (d, *J =* 8.0 Hz, Ar**H**), 7.56 (t, *J =* 7.5 Hz, Ar**H**), 7.48-7.41 (m, 2Ar**H**) 7.32 (t, *J =* 7.7 Hz, Ar**H**), 4.00 (s, O**CH₃**); ¹³C NMR (DMSO-*d*₆, 100 MHz) *δ* 157.7, 142.0, 138.5, 135.0, 134.0, 130.7, 130.1, 130.0, 129.7, 128.5, 126.5, 122.7, 121.8, 114.6, 112.3, 57.4.

### Example 1.2. Synthesis of (E)-N-((2-(3-chloropyridin-2-yl)vinyl)(2-methoxyphenyl)(oxo)-λ⁶-sulfaneylidene)cyanamide (Chemical Formula 1-58)

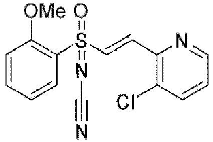

The method used for Examples 1.1.1 to 1.1.3 was used to synthesize Diethyl ((*N*-cyano-2-methoxyphenylsulfonimidoyl)methyl)phosphonate.

Using reaction (4), Diethyl ((N-cyano-2-methoxyphenylsulfonimidoyl)methyl)phosphonate (0.10 g, 0.29 mmol), 2.0 M n-BuLi solution in cyclohexane (0.17 mL, 0.35 mmol), and 3-chloropicolinaldehyde (0.05 g, 0.35 mmol) were reacted to synthesize compound 1-58 as a white solid (0.03 g, 30%): R*_{f}* = 0.23 (n-hexane 1 : EtOAc 2); ¹H NMR (CDCl₃, 400 MHz) *δ* 8.54 (dd, *J* = 1.3, 4.5 Hz, Ar**H**), 8.25 (d, *J* = 14.6 Hz, (E)-isomeric **H**), 8.06 (dd, *J =* 1.6, 8.0 Hz, Ar**H**), 7.85 (d, *J =* 14.6 Hz, (E)-isomeric **H**), 7.77 (dd, *J =* 1.4, 8.2 Hz, Ar**H**) 7.70 (td, *J =* 1.6, 8.0 Hz, Ar**H**), 7.32 (dd, *J =* 4.5, 8.2 Hz, Ar**H**), 7.19 (td, *J =* 0.7, 7.8 Hz, Ar**H**), 7.12 (td, *J* = 8.4 Hz, Ar**H**), 4.05 (s, OC**H₃**); ¹³C NMR (CDCl₃, 100 MHz) *δ* 157.5, 148.2, 147.4, 139.9, 138.1, 137.4, 133.6, 130.3, 129.9, 126.6, 123.2, 121.4, 113.1, 112.0, 56.9

### Example 2. Synthesis of sulfanone derivative in which A is phenyl and B is pyridine

In an embodiment of the present disclosure, the synthesis process of a sulfanone derivative, in which A of chemical formula 1 is phenyl and B of chemical formula 1 is pyridine, is shown by Reaction Scheme 2 presented below.

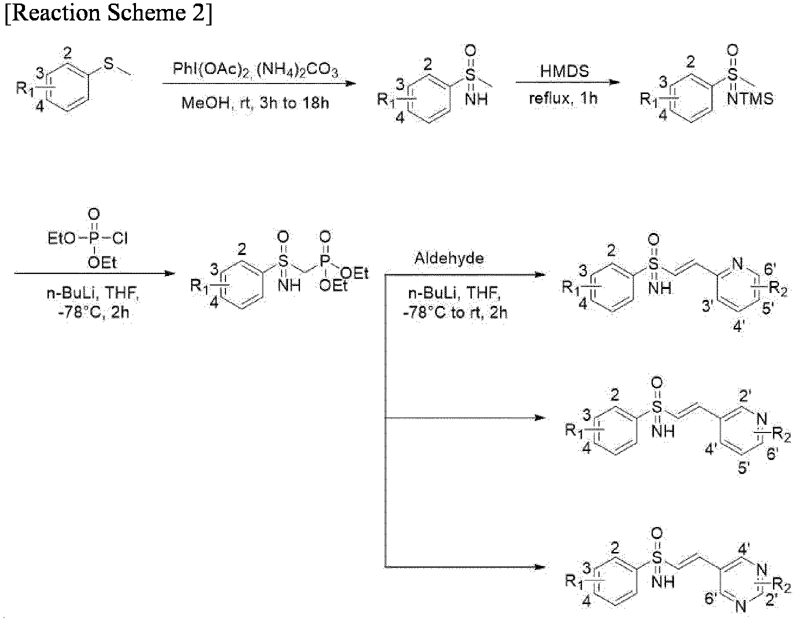

### (1) Synthesis of substituted imino(methyl)(phenyl)-λ⁶-sulfanone by reaction of ammonium carbonate and diacetoxyiodobenzene

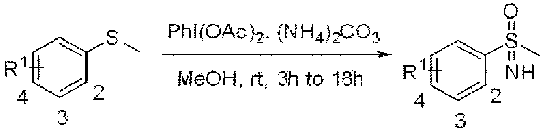

According to the above shown reaction scheme, substituted imino(methyl)(phenyl)-λ⁶-sulfanone was synthesized. The compound (1.0 eq) synthesized from reaction (1) was dissolved in methyl alcohol (Methanol; MeOH), and ammonium carbonate ((NH₄)₂CO₃, 1.5 eq) was added thereto. Diacetoxyiodobenzene (PhI(OAc)₂, 2.3 eq) was added sequentially, and then the mixture was stirred at room temperature for 3 to 18 hours. After the reaction was completed, the solvent was removed under reduced pressure from the reaction solution. The obtained residue was purified by column chromatography to obtain 2-, 3-, or 4-substituted imino(methyl)(phenyl)-λ⁶-sulfanone.

### (2) Synthesis of substituted methyl(phenyl)((trimethylsilyl)imino)-λ⁶-sulfanone using HMDS

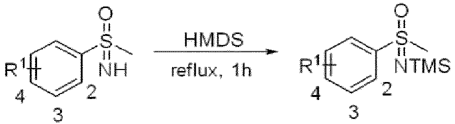

According to the above shown reaction scheme, substituted methyl(phenyl)((trimethylsilyl)imino)-λ⁶-sulfanone was synthesized. Hexamethyldisilazane (HMDS, 1.5 eq) was added to the compound (1.0 eq) synthesized in reaction (2), and the mixture was stirred at 80°C with reflux for 1 hour. After the reaction was completed, HMDS was removed under reduced pressure to obtain 2-, 3-, or 4-substituted methyl(phenyl)((trimethylsilyl)imino)-λ⁶-sulfanone.

### (3) Synthesis of substituted diethyl(phenylsulfonimidoylmethyl)phosphonate by reaction with diethylchlorophosphate

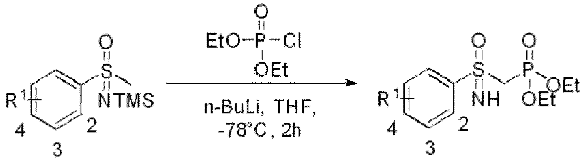

According to the above shown reaction scheme, substituted diethyl (phenylsulfonimidoylmethyl)phosphonate was synthesized. The compound (1.0 eq) synthesized in reaction (3) was dissolved in anhydrous tetrahydrofuran (THF), and then the mixed solution was cooled to -78°C with acetone and dry ice. Then, n-butyllithium (2.0 eq, 2.0 M cyclohexane solution) was added dropwise thereto at -78°C. The reaction solution was stirred at the same temperature for 30 minutes. Then, diethyl chlorophosphate (1.5 eq) was added thereto at the same temperature. The solution was stirred at room temperature for 2 hours. After the reaction was completed, the reactant was diluted with ethyl acetate (EtOAc), and was washed with water and brine. The organic layer was dried over anhydrous Na₂SO₄ and was filtered. The solvent was distilled off under reduced pressure. The residue obtained was purified by column chromatography to obtain 2-, 3-, or 4-substituted diethyl(phenylsulfonimidoylmethyl)phosphonate.

### (4) Synthesis of substituted (E)-imino(phenyl)(2-(pyridin-2-yl)vinyl)-λ⁶-sulfanone derivative by reaction with picolinaldehyde derivative

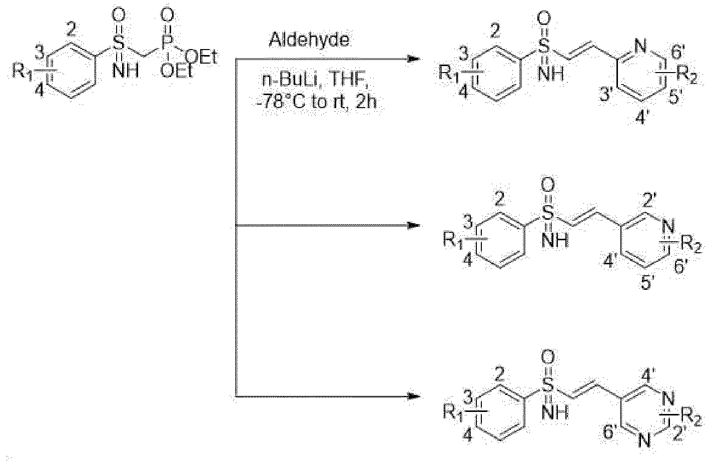

The substituted diethyl(phenylsulfonimidoylmethyl)phosphonate (1.0 eq) synthesized in reaction (3) was dissolved in anhydrous THF, and then the mixed solution was cooled to - 78°C with dry ice and acetone. To this solution, n-BuLi (1.2 eq, 2.0 M cyclohexane solution) was slowly added dropwise, and the mixed solution was stirred for 1 hour. Picolinaldehyde derivatives (1.2 eq) were added thereto and the solution was reacted for another 1 hour. If the reaction was not completed as confirmed by TLC, the reaction was allowed to proceed for another 30 minutes at room temperature. The reaction mixture was quenched with a small amount of water. The mixture was extracted with water and 10% MeOH/MC. Then, the organic layer was dried over anhydrous Na₂SO₄ to remove a small amount of water. The solvent was distilled off under reduced pressure and the mixture was dried under vacuum. Thereafter, the residue obtained was separated and purified by column chromatography to obtain the 2-, 3- or 4-substituted (E)-imino(phenyl)(2-(pyridin-2-yl)vinyl)-λ⁶-sulfanone derivative.

### Example 2.1. Synthesis of (E)-(2-fluorophenyl)(imino)(2-(pyridin-2-yl)vinyl)-λ⁶-sulfanone (Chemical Formula 1-1)

### 2.1.1. Synthesis of imino(2-fluorophenyl)(methyl)-λ⁶-sulfanone

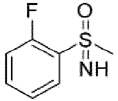

Using reaction (1), 1-fluoro-2-(methylsulfinyl)benzene (17.9 ml, 126.3 mmol) was fully dissolved in MeOH, and then diacetoxyiodobenzene (53.1 g, 189.4 mmol) and ammonium carbonate (14.0 g, 290.4 mmol) were reacted therewith to synthesize imino(2-fluorophenyl)(methyl)-λ⁶-sulfanone as a pale yellow oil (21.00 g, 96%); *R*_{f} = 0.40 (acetone/CH₂Cl₂ 1/9); ¹H NMR (300 MHz, DMSO) *δ* 7.88 (td, *J* = 1.3, 7.6 Hz, Ar**H**), 7.39-7.74 (m, 3Ar**H**), 4.71 (s, N**H**), 3.18 (s, C**H₃**); ¹³C NMR (75 MHz, DMSO) *δ* 159.0 (d, *J*_{C-F} = 249.8 Hz, Ar**C**-F), 135.6 (d, *J*_{C-F} = 8.4 Hz, Ar**C**), 132.4 (d, *J*_{C-F} = 15.6 Hz, Ar**C**), 129.8, 125.2 (d, *J*_{C*-*F} = 3.7 Hz, ArC), 117.5 (d, *J*_{C-F} = 21.9 Hz, 2ArC-CF), 45.3 (d, *J*_{C-F} = 2.7 Hz, O**C**H₃).

### 2.1.2. Synthesis of (2-Fluorophenyl)(methyl)(trimethylsilyl)imino)-λ⁶-sulfanone

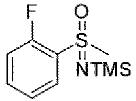

Using reaction (2), hexamethyldisilazane (6.64 ml, 31.7 mmol) was added to imino(2-fluorophenyl)(methyl)-λ⁶-sulfanone (5.00 g, 28.8 mmol) to synthesize (2-fluorophenyl)(methyl)((trimethylsilyl)imino)-λ⁶-sulfanone as a clear oil (7.0 g, 100%); R_{f} = 0.25 (acetone/CH₂Cl₂ 1/9); ¹H NMR (300 MHz, DMSO) *δ* 7.86 (td, *J* = 1.6, 7.7 Hz, Ar**H**), 7.41-7.76 (m, 3Ar**H**), 3.22 (s, C**H**₃), 0.00 (s, Si(C**H₃**)₃); ¹³C NMR (75 MHz, DMSO) *δ* 156.4 (d, *J*_{C*-*F} = 250.0 Hz, Ar**C**-F), 133.1 (dd, *J*_{C-F} = 5.0, 8.3 Hz, **C**SOCH₃), 130.0 (t, *J*_{C-F} = 14.9 Hz, Ar**C**), 127.2, 126.7, 122.7 (dd, *J*_{C-F} = 3.6, 14.4 Hz, Ar**C**), 115.0 (d, *J*_{C-F} = 4.8, 21.8 Hz, Ar**C**), 42.6 (d, *J*_{C-F} = 2.8 Hz, O**C**H₃), 0.0 (Si(**C**H₃)₃).

### 2.1.3. Synthesis of diethyl ((2-fluoro-N-(trimethylsilyl)phenylsulfonimidoyl)methyl)phosphonate

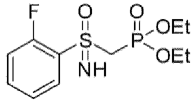

Using reaction (3), (2-Fluorophenyl)(methyl)((trimethylsilyl)imino)-λ ⁶ -sulfanone (1.00 g, 5.77 mmol) was fully dissolved in THF, and then diethyl chlorophosphate (0.99 ml, 6.93 mmol) and 2.0 M *n* -BuLi cyclohexane solution (3.43 ml, 6.93 mmol) were reacted therewith to synthesize diethyl ((2-fluoro-N-(trimethylsilyl)phenylsulfonimidoyl)methyl)phosphonate as a white solid (0.75 g, 42%); R*_{f}* = 0.20 (*n*-hexane 1 : EtOAc 1); ¹H NMR (400 MHz, DMSO-*d*₆) *δ* 7.37-7.90 (m, 4Ar**H**), 4.16 (d, *J= 21.9* Hz, SC**H₂**P), 3.83-4.02 (m, P(OC**H₂**CH₃)**₂**), 1.08-1.18 (m, P(OCH₂C**H₃**)**₂**).

### 2.1.4. Synthesis of (E)-(2-Fluorophenyl)(imino)(2-(pyridin-2-yl)vinyl)-λ⁶-sulfanone (Chemical Formula 1-1)

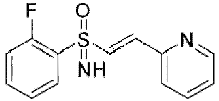

Using reaction (4)-2, diethyl ((2-fluoro-N-(trimethylsilyl)phenylsulfonimidoyl)methyl)phosphonate (0.68 mL, 2.19 mmol), 2.0 M n-BuLi solution in cyclohexane (1.70 mL, 2.63 mmol), and picolinaldehyde (0.25 mL, 2.63 mmol) were reacted to synthesize compound 1-1 as a pale yellow oil (0.26 g, 45%): R*_{f}* = 0.30 (n-hexane 1 : EtOAc 1); mp: 139.2-141.2°C; ¹H NMR (300 MHz, DMSO-*d*₆) *δ* 8.64 (d, *J* = 4.6 Hz, ArH), 7.97 (td, *J =* 1.6, 8.0 Hz, Ar**H**), 7.88 (td, *J =* 1.6, 7.6 Hz, Ar**H**), 7.81 (d, *J* = 7.4 Hz, Ar**H**), 7.63-7.74 (m, 2Ar**H**, (*E*)-isomeric **H**), 7.58 (d, *J =* 14.9 Hz, (*E*)-isomeric 2**H**), 7.37-7.46 (m, 3Ar**H**), 5.27 (s, N**H**); ¹³C NMR (75 MHz, DMSO-*d*₆) *δ* 158.6 (d, *J*_{C-F} = 251.8 Hz), 151.0, 150.1, 140.6, 137.4, 135.5 (d, *J*_{C-F} = 8.3 Hz), 133.1, 131.1 (d, *J*_{C-F} =14.0 Hz), 129.6, 125.1, 124.9 (d, *J*_{C-F} = 3.7 Hz), 117.2 (d, *J*_{C-F} = 21.5 Hz).

### Example 2.2. Synthesis of (E)-imino(4-methoxyphenyl)(2-(pyridin-2-yl)vinyl)-λ⁶-sulfanone (Chemical Formula 1-2)

### 2.2.1. Synthesis of imino(4-methoxyphenyl)(methyl)-λ⁶-sulfanone

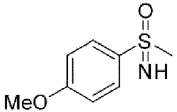

Using reaction (2), (4-methoxyphenyl)(methyl)sulfane (1.6 ml, 12.42 mmol) was fully dissolved in MeOH, and then diacetoxyiodobenzene (8.00 g, 28.53 mmol) and ammonium carbonate (1.20 g, 24.82 mmol) were reacted therewith to synthesize imino(4-methoxyphenyl)(methyl)-λ⁶-sulfanone as a pale yellow oil (2.96 g, 77%); R_{f} = 0.38 (acetone/CH₂Cl₂ 1/9); ¹H NMR (300 MHz, DMSO) δ 7.84 (d, J = 8.7 Hz, 2Ar**H**), 7.11 (d, J = 8.7 Hz, 2Ar**H**), 4.05 (s, N**H**), 3.84 (s, OC**H₃**), 3.02 (s, C**H₃**).

### 2.2.2. Synthesis of (4-methoxyphenyl)(methyl)((trimethylsilyl)imino)-λ⁶-sulfanone

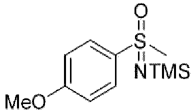

Using reaction (3), hexamethyldisilazane (2.70 ml, 12.9 mmol) was added to imino(4-methoxyphenyl)(methyl)-λ⁶-sulfanone (2.18 g, 11.7 mmol) to synthesize (4-methoxyphenyl)(methyl)((trimethylsilyl)imino)-λ⁶-sulfanone as a clear oil (3.40 g, 100%); R_{f} = 0.25 (acetone/CH₂Cl₂ 1/9); ¹H NMR (300 MHz, DMSO) δ 7.79 (d, J = 8.8 Hz, Ar**H**), 7.09 (d, J = 6.9 Hz, Ar**H**), 3.82 (s, OC**H₃**), 3.01 (s, C**H₃**), 0.00 (s, Si(C**H₃**)₃).

### 2.2.3. Synthesis of diethyl ((4-methoxyphenylsulfonimidoyl)methyl)phosphonate

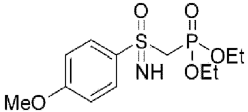

Using reaction (3), imino(4-methoxyphenyl)(methyl)((trimethylsilyl)imino)-λ⁶-sulfanone (0.22 g, 0.72 mmol) was fully dissolved in THF, and then diethyl chlorophosphate (0.22 ml, 1.50 mmol) and 2.0 M *n*-BuLi cyclohexane solution (0.74 ml, 1.50 mmol) were reacted therewith to synthesize diethyl ((4-methoxy-N-(trimethylsilyl)phenylsulfonimidoyl)methyl)phosphonate as a white solid (0.12 g, 52%); R*_{f}* = 0.29 (*n*-hexane 1 : EtOAc 4); ¹H NMR (300 MHz, DMSO-*d*₆) *δ* 7.90 (d, *J =* 8.9 Hz, 2Ar**H**), 7.11 (d, *J =* 8.9 Hz, 2Ar**H**), 4.37 (s, N**H**), 3.95-4.08 (m, P(OC**H₂**CH₃)**₂**), 3.85 (s, OC**H₃**), 1.14-1.20 (m, P(OCH₂C**H₃**)**₂**).

### 2.2.4. Synthesis of (E)-Imino(4-methoxyphenyl)(2-(pyridin-2-yl)vinyl)-λ⁶-sulfanone (Chemical Formula 1-2)

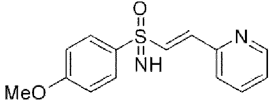

Using reaction (4)-2, diethyl ((4-methoxyphenylsulfonimidoyl)methyl)phosphonate (0.15 mL, 0.46 mmol), 2.0 M n-BuLi solution in cyclohexane (0.30 mL, 0.55 mmol), and picolinaldehyde (0.06 mL, 0.55 mmol) were reacted to synthesize compound 1-2 as a pale yellow oil (0.14 g, 51%): R*_{f}* = 0.30 (*n*-hexane 1 : EtOAc 1); ¹H NMR (300 MHz, DMSO-*d*₆) *δ* 8.59 (d, *J =* 4.7 Hz, Ar**H**), 7.82-7.39 (m, 3Ar**H**), 7.73 (d, *J* = 7.8, 8.2 Hz, Ar**H**), 7.14-7.52 (m, Ar**H**, (*E*)-isomeric 2**H**), 7.12 (d, *J* = 7.3 Hz, 2Ar**H**), 4.67 (s, N**H**), 3.83 (s, OC**H₃**); ¹³C NMR (75 MHz, DMSO-*d*₆) *δ* 162.5, 151.4, 150.0, 137.6, 137.2, 135.7, 134.0, 129.9, 124.7, 124.6, 114.4, 55.7 (O**C**H₃).

### Example 2.3. Synthesis of (E)-(2-chlorophenyl)(2-(3-fluoropyridin-2-yl)vinyl)(imino)-λ⁶-sulfanone (Chemical Formula 1-3)

### 2.3.1. Synthesis of imino(2-chlorophenyl)(methyl)-λ⁶-sulfanone

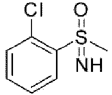

Using reaction (1), 1-chloro-2-(methylsulfinyl)benzene (6.45 ml, 50.43 mmol) was fully dissolved in MeOH, and then diacetoxyiodobenzene (37.36 g, 115.99 mmol) and ammonium carbonate (7.27 g, 75.64 mmol) were reacted therewith to synthesize imino(2-chlorophenyl)(methyl)-λ⁶-sulfanone as a pale yellow oil (5.0 g, 52%); R_{f} = 0.58 (EtOAc 100%); ¹H NMR (400 MHz, DMSO-d₆) δ 8.08-8.10 (m, Ar**H**), 7.62-7.68 (m, 2 Ar**H**), 7.55-7.59 (m, Ar**H**), 4.60 (brs, N**H**), 3.22 (s, SC**H₃**).

### 2.3.2. Synthesis of (2-chlorophenyl)(methyl)((trimethylsilyl)imino)-λ⁶-sulfanone

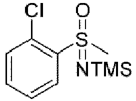

Using reaction (2), hexamethyldisilazane (1.66 ml, 7.91 mmol) was added to imino(2-chlorophenyl)(methyl)-λ⁶-sulfanone (1.00 g, 5.27 mmol) to synthesize (2-chlorophenyl)(methyl)((trimethylsilyl)imino)-λ⁶-sulfanone as a pale yellow oil (1.35 g, 98%); R_{f} = 0.81 (n-hexane 1 : EtOAc 1); ¹H NMR (400 MHz, DMSO-d₆) δ 8.08 (dd, J = 1.5, 7.8 Hz, Ar**H**), 7.63-7.68 (m, 2 Ar**H**), 7.56-7.60 (m, Ar**H**), 3.25 (s, SC**H₃**), 0.00 (s, Si(C**H₃**)**₃**).

### 2.3.3. Synthesis of diethyl ((2-chlorophenylsulfonimidoyl)methyl)phosphonate

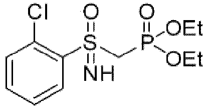

Using reaction (3), (2-chlorophenyl)(methyl)((trimethylsilyl)imino)-λ⁶-sulfanone (1.35 g, 5.16 mmol) was fully dissolved in THF, and then diethyl chlorophosphate (0.89 ml, 6.19 mmol) and 2.0 M n-BuLi cyclohexane solution (3.09 ml, 6.19 mmol) were reacted therewith to synthesize diethyl ((2-chlorophenylsulfonimidoyl)methyl)phosphonate as a white solid (0.67 g, 40%); R*_{f}* = 0.18 (n-hexane 1 : EtOAc 1); ¹H NMR (400 MHz, DMSO-*d*₆) *δ* 8.10 (d, *J =* 7.9 Hz, ArH), 7.66 (d, *J* = 3.7 Hz, 2 Ar**H**), 7.56-7.60 (m, Ar**H**), 4.93 (s, N**H**), 4.27 (q, *J* = 15.8 Hz, SC**H₂**P), 3.87-4.01 (m, P(OC**H₂**CH₃)**₂**), 1.11-1.16 (m, P(OCH₂C**H₃**)**₂**).

### 2.3.4. Synthesis of (E)-(2-chlorophenyl)(2-(3-fluoropyridin-2-yl)vinyl)(imino)-λ⁶-sulfanone (Chemical Formula 1-3)

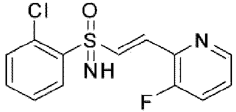

Using reaction (4)-2, diethyl ((2-chlorophenylsulfonimidoyl)methyl)phosphonate (0.20 mL, 0.62 mmol), 2.0 M n-BuLi solution in cyclohexane (0.40 mL, 0.74 mmol), and 3-fluoropicolinaldehyde (0.10 g, 0.74 mmol) were reacted to synthesize compound 1-3 as a pale yellow powder (0.11 g, 61%); R*_{f}* = 0.51 (*n*-hexane 1 : EtOAc 4); mp: 94.3-96.0°C ¹H NMR (400 MHz, DMSO-*d*₆) *δ* 8.52 (d, *J =* 4.4 Hz, Ar**H**), 8.21 (d, *J =* 7.4 Hz, Ar**H**), 7.87 (t, *J =* 9.4 Hz, Ar**H**), 7.75 (d, *J=* 14.8 Hz, (*E*)-isomeric **H**), 7.52-7.69 (m, 4Ar**H**, (*E*)-isomeric **H**), 5.31 (s, N**H**); ¹³C NMR (100 MHz, DMSO-*d*₆) *δ* 157.8 (d, *J*_{C-F} = 261.1 Hz), 146.4 (d, *J*_{C-F} = 4.9 Hz), 139.9, 138.8 (d, *J*_{C-F} = 11.0 Hz), 134.4, 133.5 (d, *J*_{C-F} = 4.4 Hz), 132.8, 131.8, 131.3, 130.6, 127.8, 127.5 (d, *J*_{C-F} = 4.6 Hz), 124.7 (d, *J*_{C-F} = 18.9 Hz).

### Example 2.4. Synthesis of (E)-(2-chlorophenyl)(2-(3-chloropyridin-2-yl)vinyl)(imino)-λ⁶-sulfanone (Chemical Formula 1-4)

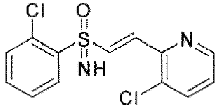

The method used for Examples 2.3.1 to 2.3.3 was used to synthesize diethyl ((2-chlorophenylsulfonimidoyl)methyl)phosphonate.

Using reaction (4)-2, diethyl ((2-chlorophenylsulfonimidoyl)methyl)phosphonate (1.00 g, 3.07 mmol), 2.0 M n-BuLi solution in cyclohexane (1.84 mL, 3.68 mmol), and 3-chloropicolinaldehyde (0.44 g, 3.07 mmol) were reacted to synthesize compound 1-4 as a white powder (0.48 g, 50%); R*_{f}* = 0.30 (n-hexane 1 : EtOAc 2); mp: 144.8-146.5°C ¹H NMR (400 MHz, CDCl₃) *δ* 8.61 (dd, *J =* 1.3, 4.5 Hz, Ar**H**), 8.20-8.22 (m, Ar**H**), 8.06 (dd, *J* = 1.4, 8.2 Hz, Ar**H**), 7.97 (d, *J =* 14.6 Hz, (E)-isomeric **H**), 7.59-7.68 (m, (*E*)-isomeric **H**, 3 Ar**H**), 7.51 (dd, *J =* 4.5, 8.2 Hz, Ar**H**), 7), 5.32 (brs, N**H**). ¹³C NMR (75 MHz, DMSO-*d*₆) *δ* 148.6, 147.4, 140.0, 138.3, 135.4, 134.6, 134.5, 131.9, 131.8, 131.4, 130.7, 127.9, 126.7.

### Example 2.5. Synthesis of (E)-(2-fluorophenyl)(2-(3-fluoropyridin-2-yl)vinyl)(imino)-λ⁶-sulfanone (Chemical Formula 1-5)

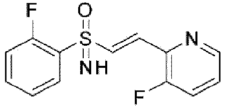

The method used for Examples 2.1.1 to 2.1.3 was used to synthesize diethyl ((2-fluoro-N-(trimethylsilyl)phenylsulfonimidoyl)methyl)phosphonate.

Using reaction (4)-2, diethyl ((2-fluorophenylsulfonimidoyl)methyl)phosphate (0.24 mL, 0.78 mmol), 2.0 M n-BuLi solution in cyclohexane (0.50 mL, 0.94 mmol), and 3-fluoropicolinaldehyde (0.12 g, 0.94 mmol) were reacted to synthesize compound 1-5 as a pale yellow oil (0.11 g, 51%): R*_{f}* = 0.30 (n-hexane 1 : EtOAc 2); mp: 107.8-109.8°C ¹H NMR (300 MHz, DMSO-*d*₆) *δ* 8.51 (d, *J =* 4.4 Hz, Ar**H**), 7.98 (t, *J* = 7.8 Hz, Ar**H**), 7.87 (t, *J =* 8.6 Hz, Ar**H**), 7.38-7.76 (m, 4Ar**H**, (E)-isomeric 2**H**), 5.39 (s, N**H**); ¹³C NMR (75 MHz, DMSO-*d*₆) *δ* 158.6 (d, *J*_{C-F} = 251.4 Hz), 157.8 (d, *J*_{C-F} = 260.9 Hz), 146.4 (d, *J*_{C-F} = 4.9 Hz), 138.8 (d, *J*_{C-F} = 11.0 Hz), 135.8 (d, *J*_{C-F} =8.4 Hz), 134.7 (d, *J*_{C-F} = 3.1 Hz), 132.3, 130.6 (d, *J*_{C-F} = 14.1 Hz), 129.7, 127.6 (d, *J*_{C-F} = 4.6 Hz), 125.0 (d, *J*_{C-F} = 3.6 Hz), 124.7 (d, *J*_{C-F} = 18.9 Hz), 117.2 (d, *J*_{C-F} = 21.6 Hz).

### Example 2.6. Synthesis of (E)-(2-(3-chloropyridin-2-yl)vinyl)(2-fluorophenyl)(imino)-λ⁶-sulfanone (Chemical Formula 1-6)

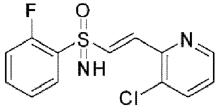

The method used for Examples 2.1.1 to 2.1.3 was used to synthesize diethyl ((2-fluoro-N-(trimethylsilyl)phenylsulfonimidoyl)methyl)phosphonate.

Using reaction (4)-2, diethyl ((2-fluoro-N-(trimethylsilyl)phenylsulfonimidoyl)methyl)phosphonate (0.24 mL, 0.78 mmol), 2.0 M n-BuLi solution in cyclohexane (0.50 mL, 0.94 mmol), and 3-chloropicolinaldehyde (0.12 g, 0.94 mmol) were reacted to synthesize compound 1-6 as a white powder (0.14 g, 59%): *R_{f}* = 0.30 (n-hexane 1 : EtOAc 2); mp: 89.8-91.8°C ¹H NMR (300 MHz, DMSO-*d*₆) *δ* 8.60 (d, *J =* 4.4 Hz, ArH), 8.06 (d, *J* = 8.2 Hz, Ar**H**), 7.70-8.00 (m, 2Ar**H**, (*E*)-isomeric **H**), 7.61 (d, *J* = 14.9 Hz, (*E*)-isomeric **H**), 7.29-7.54 (m, 3Ar**H**) 5.41 (s, N**H**); ¹³C NMR (75 MHz, DMSO-*d*₆) *δ* 158.6 (d, *J*_{C-F} = 251.4 Hz), 148.6, 147.3, 138.3, 135.9, 135.8, 135.7, 134.7, 131.8, 130.6 (d, *J*_{C-F} = 14.0 Hz), 129.7, 126.7, 125.0 (d, *J*_{C-F} = 3.6 Hz), 117.2 (d, *J*_{C-F} = 21.5 Hz).

### Example 2.7. Synthesis of (E)-(2-(3-fluoropyridin-2-yl)vinyl)(imino)(3-methoxyphenyl)-λ⁶-sulfanone (Chemical Formula 1-7)

### 2.7.1. Synthesis of imino(3-methoxyphenyl)methyl-λ⁶-sulfanone

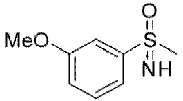

Using reaction (1), (3-methoxyphenyl)(methyl)sulfane (1.4 ml, 10.35 mmol) was fully dissolved in MeOH, and then diacetoxyiodobenzene (6.67 g, 23.78 mmol) and ammonium carbonate (1.00 g, 20.69 mmol) were reacted therewith to synthesize imino(3-methoxyphenyl)methyl-λ⁶-sulfanone as a pale yellow oil (2.96 g, 77%); *R*_{f} = 0.38 (acetone/CH₂Cl₂ 1/9); ¹H NMR (300 MHz, DMSO) δ 7.19-7.54 (m, 4Ar**H**), 4.19 (s, N**H**), 3.84 (s, OC**H₃**), 3.06 (s, C**H₃**).

### 2.7.2. Synthesis of (3-methoxyphenyl)(methyl)((trimethylsilyl)imino)-λ⁶-sulfanone

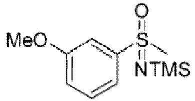

Using reaction(2), hexamethyldisilazane (0.74 ml, 3.52 mmol) was added to imino(3-methoxyphenyl)methyl-λ⁶-sulfanone (0.50 g, 2.93 mmol) to synthesize (3-methoxyphenyl)(methyl)((trimethylsilyl)imino)-λ⁶-sulfanone as a clear oil (0.52 g, 70%); *R*_{f} = 0.25 (acetone/CH₂Cl₂ 1/9); ¹H NMR (300 MHz, DMSO) *δ* 7.35-7.51 (m, 3Ar**H**), 7.18 (d, J = 4.4 Hz, Ar**H**), 3.81 (s, OC**H₃**), 3.05 (s, C**H₃**), 0.00 (s, Si(C**H₃**)₃).

### 2.7.3. Synthesis of diethyl ((3-methoxyphenylsulfonimidoyl)methyl)phosphonate

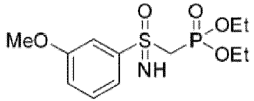

Using reaction (3), imino(3-methoxyphenyl)(methyl)((trimethylsilyl)imino)-λ⁶-sulfanone (0.34 g, 1.12 mmol) was fully dissolved in THF, and then diethyl chlorophosphate (0.25 ml, 1.68 mmol) and 2.0 M *n*-BuLi cyclohexane solution (0.83 ml, 1.68 mmol) were reacted therewith to synthesize diethyl ((3-methoxy-N-(trimethylsilyl)phenylsulfonimidoyl)methyl)phosphonate as a white solid (0.12 g, 51%); R*_{f}* = 0.27 (*n*-hexane 1 : EtOAc 3); ¹H NMR (400 MHz, DMSO-*d*₆) *δ* 7.48-7.55 (m, 4Ar**H**), 4.48 (s, N**H**), 3.95-4.06 (m, P(OC**H₂**CH₃)**₂**), 3.84 (s, OC**H₃**), 1.14-1.20 (m, P(OCH₂C**H₃**)**₂**).

### 2.7.4. Synthesis of (E)-(2-(3-fluoropyridin-2-yl)vinyl)(imino)(3-methoxyphenyl)-λ⁶-sulfanone (Chemical Formula 1-7)

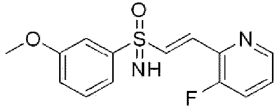

Using reaction (4)-2, diethyl ((3-methoxyphenylsulfonimidoyl)methyl)phosphonate (0.51 mL, 1.58 mmol), 2.0 M n-BuLi solution in cyclohexane (0.95 mL, 1.89 mmol), and 3-fluoropicolinaldehyde (0.24 g, 1.89 mmol) were reacted to synthesize compound 1-7 as a transparent oil (0.24 g, 52%): R*_{f}* = 0.34 (n-hexane 1 : EtOAc 2); ¹H NMR (400 MHz, DMSO-*d*₆) *δ* 8.48 (d, *J* = 4.4 Hz, Ar**H**), 7.85 (t, *J =* 9.1 Hz, Ar**H**), 7.62 (d, *J =* 14.9 Hz, (*E*)-isomeric **H**), 7.48-7.58 (m, 4Ar**H**, (*E*)-isomeric **H**), 7.23 (d, *J* = 7.3 Hz, Ar**H**), 4.95 (s, N**H**); ¹³C NMR (75 MHz, DMSO-*d*₆) *δ* 159.5, 157.7 (d, *J*_{C-F} = 261.8 Hz), 146.3 (d, *J*_{C-F} = 4.8 Hz), 143.6, 139.1 (d, *J*_{C-F} = 11.1 Hz), 136.4 (d, *J*_{C-F} = 4.3 Hz), 130.4 (d, *J*_{C-F} = 11.4 Hz), 127.2 (d, *J*_{C-F} = 2.4 Hz), 124.6 (d, *J*_{C-F} = 18.9 Hz), 119.4 (d, *J*_{C-F} = 83.1 Hz), 112.5, 55.6 (O**C**H₃).

### Example 2.8. Synthesis of (E)-(2-(3-chloropyridin-2-yl)vinyl)(imino)(2-methoxyphenyl)-λ⁶- sulfanone (Chemical Formula 1-8)

### 2.8.1. Synthesis of imino(2-methoxyphenyl)methyl)-λ⁶-sulfanone

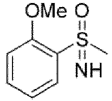

Using reaction (1), (2-methoxyphenyl)(methyl)sulfane (2.8 ml, 20.70 mmol) was fully dissolved in MeOH, and then diacetoxyiodobenzene (15.33 g, 47.57 mmol) and ammonium carbonate (3.98 g, 41.39 mmol) were reacted therewith to synthesize imino(2-methoxyphenyl)methyl)-λ⁶-sulfanone as a pale yellow oil (3.08 g, 80%); R*_{f}* = 0.30 (EtOAc 100%); ¹H NMR (400 MHz, DMSO-*d*₆) *δ* 7.84 (dd, 1.7, 7.8 Hz, Ar**H**), 7.60 (m, Ar**H**), 7.23 (d, 8.2 Hz, Ar**H**), 7.11 (m, Ar**H**), 4.23 (brs, N**H**), 3.92 (s, OC**H₃**), 3.14 (s, SC**H₃**).

### 2.8.2. Synthesis of (2-methoxyphenyl)methyl((trimethylsilyl)imino)-λ⁶-sulfanone

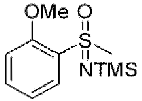

Using reaction (2), hexamethyldisilazane (5.1 ml, 24.30 mmol) was added to imino(2-methoxyphenyl)methyl)-λ⁶-sulfanone (3.08 g, 16.63 mmol) to synthesize (2-methoxyphenyl)methyl((trimethylsilyl)imino)-λ⁶-sulfanone as a clear oil (4.28 g, 100%); R*_{f}* = 0.81 (EtOAc 100%); ¹H NMR (400 MHz, DMSO-*d*₆) *δ* 7.88 (dd, 1.7, 7.8 Hz, Ar**H**), 7.68 (m, Ar**H**), 7.30 (d, 8.2 Hz, Ar**H**), 7.18 (m, Ar**H**), 3.99 (s, OC**H₃**), 3.22 (s, SC**H₃**), 0.00 (s, Si(C**H₃**)**₃**).

### 2.8.3. Synthesis of diethyl ((2-methoxyphenylsulfonimidoyl)methyl)phosphonate

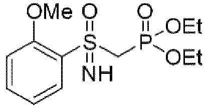

Using reaction (3), (2-methoxyphenyl)methyl((trimethylsilyl)imino)-λ⁶-sulfanone (4.28 g, 16.63 mmol) was fully dissolved in THF, and then diethyl chlorophosphate (2.88 ml, 19.95 mmol) and 2.0 M *n*-BuLi cyclohexane solution (18.29 ml, 36.58 mmol) were reacted therewith to synthesize diethyl ((2-methoxyphenylsulfonimidoyl)methyl)phosphonate a pale yellow solid (3.48 g, 53%); R*_{f}* = 0.28 (EtOAc 100%); ¹H NMR (400 MHz, DMSO-*d*₆) *δ* 7.88 (dd, 7.8, 29.7 Hz, Ar**H**), 7.62 (m, Ar**H**), 7.23 (dd, 4.3, 8.2 Hz, Ar**H**), 7.18 (t, 7.5 Hz, Ar**H**), 4.02-3.88 (m, SC**H₂**P, (POC**H₂**CH₃)**₂**, OC**H₃**), 1.16-1.10 (m, P(OCH₂**CH₃**)**₂**), 0.01 (s, Si(C**H₃**)**₃**).

### 2.8.4. Synthesis of (E)-(2-(3-chloropyridin-2-yl)vinyl)(imino)(2-methoxyphenyl)-λ⁶-sulfanone (Chemical Formula 1-8)

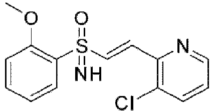

The method used for Examples 1.1.1 to 1.1.3 was used to synthesize diethyl ((2-methoxyphenylsulfonimidoyl)methyl)phosphonate.

Using reaction (4)-2, diethyl ((2-methoxyphenylsulfonimidoyl)methyl)phosphonate (0.61 mL, 1.91 mmol), 2.0 M n-BuLi solution in cyclohexane (1.20 mL, 2.29 mmol), and 3-chloropicolinaldehyde (0.33 g, 2.29 mmol) were reacted to synthesize compound 1-8 as a pale yellow powder (0.40 g, 68%): R*_{f}* = 0.35 (*n*-hexane 1 : EtOAc 7), mp: 113.0-114.1°C ¹H NMR (400 MHz, DMSO-*d*₆) *δ* 8.60 (d, *J* = 4.4 Hz, Ar**H**), 8.04 (dd, *J=* 1.4, 8.2 Hz, Ar**H**), 7.95 (dd, *J* = 1.6, 7.8 Hz, Ar**H**), 7.90 (d, *J* = 14.7 Hz, (E)-isomeric **H**), 7.67 (d, *J* = 14.6 Hz, (E)-isomeric **H**), 7.48-7.64 (m, 2Ar**H**), 7.22 (d, *J =* 8.2 Hz, Ar**H**), 7.15 (t, *J =* 7.4 Hz, Ar**H**), 4.84 (s, N**H**), 3.91 (OC**H₃**); ¹³C NMR (75 MHz, DMSO-*d*₆) *δ* 156.7, 148.6, 147.7, 138.2, 136.4, 134.9, 133.7, 131.6, 130.2, 128.8, 126.4, 120.4, 113.3, 56.2 (O**C**H₃).

### Example 2.9. Synthesis of (E)-(2-(3-chloropyridin-2-yl)vinyl)(imino)(3-methoxyphenyl)-λ⁶-sulfanone (Chemical Formula 1-9)

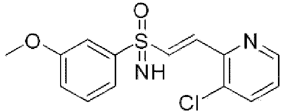

The method used for Examples 2.7.1 to 2.7.3 was used to synthesize diethyl ((3-methoxyphenylsulfonimidoyl)methyl)phosphonate.

Using reaction (4)-2, diethyl ((3- methoxyphenylsulfonimidoyl)methyl)phosphonate (0.62 mL, 1.93 mmol), 2.0 M n-BuLi solution in cyclohexane (1.2 mL, 2.32 mmol), and 3-chloropicolinaldehyde (0.33 g, 2.32 mmol) were reacted to synthesize compound 1-9 as a pale yellow oil (0.24 g, 44%): R*_{f}* = 0.37 (*n*-hexane 1 : EtOAc 2); ¹H NMR (300 MHz, DMSO-*d*₆) *δ* 8.57 (d, *J=* 4.5 Hz, Ar**H**), 8.02-8.05 (m, Ar**H**), 7.84 (d, *J =* 14.6 Hz, (*E*)-isomeric **H**), 7.22-7.65 (m, 5Ar**H**, (*E*)-isomeric 2**H**), 4.98 (s, N**H**), 3.84 (OC**H₃**); ¹³C NMR (75 MHz, DMSO-*d*₆) *δ* 160.1, 148.5, 147.9, 143.1, 137.8, 136.7, 134.4, 130.8, 125.5, 120.8, 119.7, 113.0, 55.7 (O**C**H₃).

### Example 2.10. Synthesis of (E)-(2-(3-chloropyridin-2-yl)vinyl)(imino)(4-methoxyphenyl)-λ⁶-sulfanone (Chemical Formula 1-10)

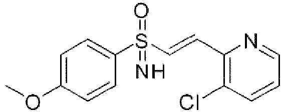

The method used for Examples 2.2.1 to 2.2.3 was used to synthesize diethyl ((4-methoxyphenylsulfonimidoyl)methyl)phosphonate.

Using reaction (4)-2, diethyl ((4-methoxyphenylsulfonimidoyl)methyl)phosphonate (0.15 mL, 0.46 mmol), 2.0 M n-BuLi solution in cyclohexane (0.30 mL, 0.55 mmol), and 3-chloropicolinaldehyde (0.07 mL, 0.55 mmol) were reacted to synthesize compound 1-10 as a pale yellow oil (0.15 g, 53%): R*_{f}* = 0.32 (n-hexane 1 : EtOAc 2); mp: 103.6-105.6°C ¹H NMR (300 MHz, DMSO-*d*₆) *δ* 8.56 (d, *J* = 4.5 Hz, Ar**H**), 7.11-7.89 (m, 7Ar**H**, (*E*)-isomeric 2**H**), 4.67 (s, N**H**), 3.83 (OC**H₃**); ¹³C NMR (75 MHz, DMSO-*d*₆) *δ* 162.7, 148.4, 147.6, 138.4, 138.2, 133.3, 131.5, 130.1, 126.3, 114.5, 55.7 (O**C**H₃).

### Example 2.11. Synthesis of (E)-imino(2-methoxyphenyl)(2-(3-(trifluoromethyl)pyridin-2-yl)vinyl)-λ⁶-sulfanone (Chemical Formula 1-11)

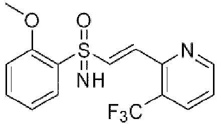

Using the same method as in Examples 2.8.1 to 2.8.3, diethyl ((2-methoxyphenylsulfonimidoyl)methyl)phosphonate .

Using reaction (4)-2, diethyl ((2-methoxyphenylsulfonimidoyl)methyl)phosphonate (0.30 g, 0.76 mmol) was fully dissolved in THF, and then 3-(trifluoromethyl)picolinaldehyde (0.11 ml, 0.84 mmol) and 2.0 M *n*-BuLi cyclohexane solution (0.42 ml, 0.84 mmol) were reacted therewith to synthesize compound 1-11 as a white solid (0.07 g, 28%): R*_{f}* = 0.22 (n-hexane 1 : EtOAc 3); ¹H NMR (400 MHz, CDCl₃) *δ* 8.76 (d, 4.0 Hz, Ar**H**), 8.09 (dd, 1.7, 7.9, Ar**H**), 8.00-7.96 (m, 2Ar**H**), 7.92 (d, *J =* 14.4 Hz, (*E*)-isomeric **H**), 7.55 (td, *J* = 1.7, 7.6 Hz, Ar**H**), 7.40 (dd, *J =* 4.7, 8.0 Hz, Ar**H**), 7.10 (t, *J =* 7.3 Hz, Ar**H**), 7.01 (d, *J =* 8.3 Hz, Ar**H**), 3.95 (s, OC**H₃**), 3.14 (brs, N**H**); ¹³C NMR (100 MHz, DMSO-*d*₆) *δ* 157.2, 153.8, 149.0, 138.3, 135.5 (2Ar**C)**, 133.9, 130.4, 129.5, 125.3, 124.0 (q, *J*_{C-F} = 271.8 Hz), 124.4 (q, *J*_{C-F} = 31.4 Hz), 120.9, 113.7, 56.7.

### Example 2.12. Synthesis of (E)-(2-chlorophenyl)(imino)(2-(pyridin-3-yl)vinyl)-λ⁶-sulfanone (Chemical Formula 1-12)

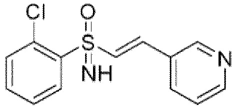

The method used for Examples 2.3.1 to 2.3.3 was used to synthesize diethyl ((2-chlorophenylsulfonimidoyl)methyl)phosphonate.

Using reaction (4)-2, diethyl ((2-chlorophenylsulfonimidoyl)methyl)phosphonate (0.70 mL, 2.14 mmol), 2.0 M n-BuLi solution in cyclohexane (1.20 mL, 2.14 mmol), and nicotinaldehyde (0.14 mL, 1.43 mmol) were reacted to synthesize compound 1-12 as a pale yellow oil (0.43 g, 71%); R*_{f}* = 0.52 (*n*-hexane 1 : EtOAc 4); ¹H NMR (400 MHz, DMSO-*d*₆) *δ* 8.90 (s, Ar**H**), 8.60 (d, *J =* 4.6 Hz, Ar**H**), 8.21 (t*, J =* 6.1 Hz, 2Ar**H**), 7.44-7.70 (m, 4Ar**H**, (*E*)-isomeric 2**H**), 5.11 (s, N**H**).

### Example 2.13. Synthesis of (E)-(2-chlorophenyl)(2-(2-chloropyridin-3-yl)vinyl)(imino)-λ ⁶ -sulfanone (Chemical Formula 1-13)

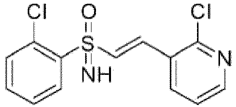

The method used for Examples 2.3.1 to 2.3.3 was used to synthesize diethyl ((2-chlorophenylsulfonimidoyl)methyl)phosphonate.

Using reaction (4)-2, diethyl ((2-chlorophenylsulfonimidoyl)methyl)phosphonate (0.70 mL, 2.14 mmol), 2.0 M n-BuLi solution in cyclohexane (1.20 mL, 2.14 mmol), and 2-chloronicotinaldehyde (0.21 g, 1.43 mmol) were reacted to synthesize compound 1-13 as a white powder (0.48 g, 60%); R*_{f}* = 0.50 (*n*-hexane 1 : EtOAc 9); ¹H NMR (400 MHz, DMSO-*d*₆) *δ* 8.47 (dd, *J* = 1.8, 4.7 Hz, Ar**H**), 8.37 (dd, *J* = 1.8, 7.8 Hz, Ar**H**), 8.22 (dd, *J* = 1.5, 7.7 Hz, Ar**H**), 7.86 (d, *J =* 15.2 Hz, (*E*)-isomeric **H**), 7.49-7.69 (m, 4Ar**H**, (*E*)-isomeric **H**), 5.26 (s, N**H**); ¹³C NMR (100 MHz, DMSO-*d*₆) *δ* 151.8, 150.6, 140.6, 138.4, 136.8, 134.9, 133.9, 132.4, 131.9, 131.2, 128.3, 127.9, 124.3.

### Example 2.14. Synthesis of (E)-(2-chlorophenyl)(imino)(2-(2,4,6-trichloropyrimidin-5-yl)vinyl)-λ⁶-sulfanone (Chemical Formula 1-14)

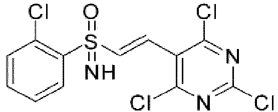

The method used for Examples 2.3.1 to 2.3.3 was used to synthesize diethyl ((2-chlorophenylsulfonimidoyl)methyl)phosphonate.

Using reaction (4)-2, diethyl ((2-chlorophenylsulfonimidoyl)methyl)phosphonate (0.60 mL, 1.84 mmol), 2.0 M n-BuLi solution in cyclohexane (2.76 mL, 2.76 mmol), and 2,4,6-trichloropyrimidine-5-carbaldehyde (0.39 g, 1.84 mmol) were reacted to synthesize compound 1-14 as a white powder (0.46 g, 66%); R*_{f}* = 0.10 (*n*-hexane 3 : EtOAc 1); ¹H NMR (400 MHz, DMSO-*d*₆) *δ* 8.90 (s, Ar**H**), 8.60 (d, *J =* 4.6 Hz, Ar**H**), 8.21 (t, *J =* 6.1 Hz, 2Ar**H**), 7.44-7.70 (m, 4Ar**H**, (*E*)-isomeric 2**H**), 5.11 (s, N**H**).

### Example 3. Synthesis of sulfanone derivative in which A is pyridine and B is phenyl

In an example of the present disclosure, the synthesis process of a sulfanone derivative, in which A of Chemical Formula 1 is pyridine and B of Chemical Formula 1 is phenyl, is shown by Reaction Scheme 3 presented below.

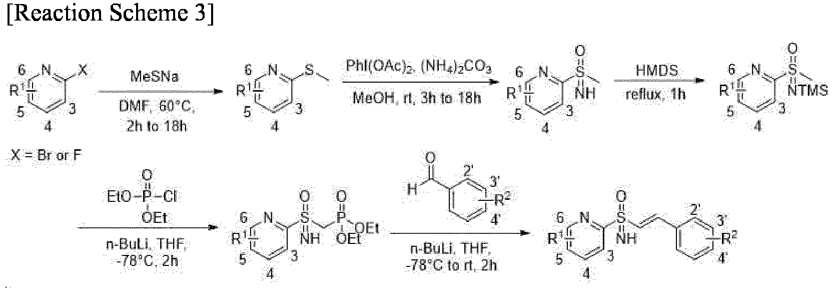

### (1) Synthesis of methylthiopyridine derivative from halogen pyridine derivative

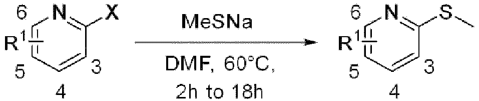

According to the above shown Reaction Scheme, 2-(methylthio)pyridine derivatives were synthesized. A bromo or fluoro pyridine starting material (1.0 eq) substituted with a methoxy group, fluorine or chlorine at the 3-, 4-, 5- or 6-position was dissolved in dimethyl formamide (DMF). Then, sodium thiomethoxide (2.0 eq) was added, and the mixture was stirred at 60°C for 2 to 18 hours. After the reaction was completed, the reactant was diluted with ethyl acetate (EtOAc), and was washed with water and brine. The organic layer was dried over anhydrous Na₂SO₄ and was filtered. The solvent was distilled under reduced pressure. The residue obtained thereby was purified by column chromatography using a mixed developing solvent of ethyl acetate/n-hexane to obtain 3-, 4-, 5- or 6-substituted 2-(methylthio)pyridine derivatives.

### (2) Synthesis of substituted imino(methyl)(pyridin-2-yl)-λ⁶-sulfanone by reaction of ammonium carbonate and diacetoxyiodobenzene

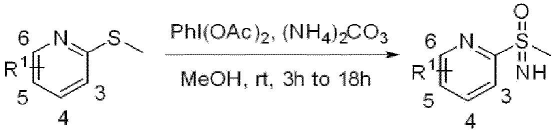

According to the above shown Reaction Scheme, substituted imino(methyl)(pyridine-2-yl)-λ⁶-sulfanone was synthesized. The compound (1.0 eq) synthesized from reaction (1) was dissolved in methyl alcohol (Methanol; MeOH), and then ammonium carbonate ((NH₄)₂CO₃, 1.5 eq) was added thereto. Next, diacetoxyiodobenzene (PhI(OAc)₂, 2.3 eq) was added, and the mixture was stirred at room temperature for 3 to 18 hours. After the reaction was completed, the solvent was removed under reduced pressure from the reaction solution. The obtained residue was purified by column chromatography to obtain 3-, 4-, 5- or 6-substituted imino(methyl)(pyridin-2-yl)- λ⁶-sulfanone.

### (3) Synthesis of substituted methyl(pyridin-2-yl)((trimethylsilyl)imino)-λ⁶-sulfanone using HMDS

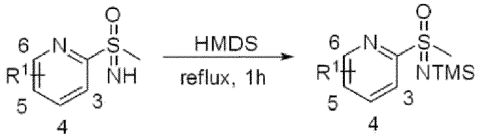

According to the above shown Reaction Scheme, substituted methyl(pyridine-2-yl)((trimethylsilyl)imino)-λ⁶-sulfanone was synthesized. Hexamethyldisilazane (HMDS, 1.5 eq) was added to the compound (1.0 eq) synthesized in reaction (2), and the mixture was stirred at 80°C with reflux for 1 hour. After the reaction was completed, HMDS was removed under reduced pressure to obtain 3-, 4-, 5-, or 6-substituted methyl(pyridine-2-yl)((trimethylsilyl)imino)-λ⁶-sulfanone.

### (4) Synthesis of substituted methyl(pyridin-2-yl)((trimethylsilyl)imino)-λ⁶-sulfanone using HMDS

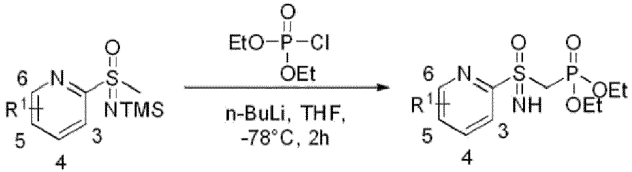

According to the above shown Reaction Scheme, substituted diethyl ((pyridine-2-sulfonimidoyl)methyl)phosphonate was synthesized. The compound (1.0 eq) synthesized in reaction (3) was dissolved in anhydrous tetrahydrofuran (THF), and then the mixed solution was cooled to -78°C with acetone and dry ice. Then, n-butyllithium (2.0 eq, 2.0 M cyclohexane solution) was added dropwise thereto. The solution was stirred at the same temperature for 30 minutes. Then, diethyl chlorophosphate (1.5 eq) was added thereto at the same temperature. The mixture was stirred at room temperature for 2 hours. After the reaction was completed, the reactant was diluted with ethyl acetate (EtOAc), and was washed with water and brine. The organic layer was dried over anhydrous Na₂SO₄ and was filtered. The solvent was distilled off under reduced pressure, and the residue obtained was purified by column chromatography to obtain 3-, 4-, 5-, or 6-substituted diethyl((pyridine-2-sulfonimidoyl)methyl)phosphonates.

### (5)-1. Synthesis of substituted (E)-imino(pyridin-2-yl)(styryl)-λ⁶-sulfanone derivative by reaction with benzaldehyde derivative

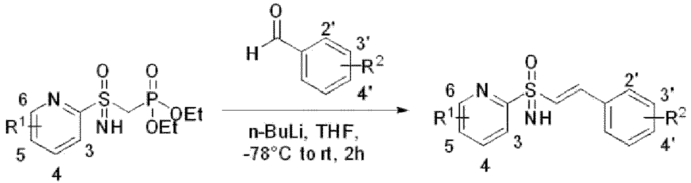

According to the above shown Reaction Scheme, substituted (E)-imino(pyridin-2-yl)(styryl)-λ⁶-sulfanone derivatives were synthesized. The compound (1.0 eq) synthesized in step 2-4 was dissolved in anhydrous THF, and then the mixed solution was cooled to -78°C using dry ice and acetone. n-BuLi (1.2 eq, 2.0 M cyclohexane solution) was slowly added dropwise thereto, and the solution was stirred for 1 hour. To this solution, benzaldehyde derivatives (1.2 eq) were added and then were reacted for another hour. If the reaction was not completed as confirmed by TLC, the reaction was allowed to proceed for another 30 minutes at room temperature. The reaction was quenched with a small amount of water. Then, extraction was performed with water and 10% MeOH/MC. The organic layer was dried over anhydrous Na₂SO₄ to remove a small amount of water. The solvent was distilled off under reduced pressure, and the mixture was dried under vacuum. Thereafter, the residue obtained was separated and purified by column chromatography to obtain 3-, 4-, 5-, or 6-substituted (E)-imino(pyridin-2-yl)(styryl)-λ⁶-sulfanone derivative.

### Example 3.1. Synthesis of (E)-(2-chlorostyryl)(imino)(pyridin-2-yl)-λ⁶-sulfanone (Chemical Formula 1-15)

### 3.1.1. Synthesis of imino(methyl)(pyridin-2-yl)-λ⁶-sulfanone

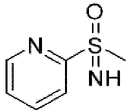

Using reaction (2), 2-(methylthio)pyridine (0.18 mL, 1.60 mmol) was fully dissolved in MeOH, and then the solution was reacted with diacetoxyiodobenzene (1.18 g, 3.67 mmol) and ammonium carbonate (0.23 g, 2.40 mmol) to synthesize imino(methyl)(pyridin-2-yl)-λ⁶-sulfanone as a white oil (0.21 g, 83%): R*_{f}* = 0.18 (EtOAc 100%); ¹H NMR (400 MHz, DMSO-*d*₆) *δ* 8.70-8.72 (m, Ar**H**), 8.05-8.12 (m, 2 Ar**H**), 7.63-7.66 (m, Ar**H**), 4.41 (s, N**H**), 3.15 (s, C**H₃**).

### 3.1.2. Synthesis of methyl(pyridin-2-yl)((trimethylsilyl)imino)-λ⁶-sulfanone

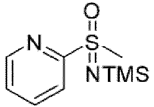

Using reaction (3), hexamethyldisilazane (0.46 mL, 2.18 mmol) was added to imino(methyl)(pyridin-2-yl)-λ⁶-sulfanone (0.23 g, 1.45 mmol) to synthesize methyl(pyridin-2-yl)((trimethylsilyl)imino)-λ⁶-sulfanone as a clear oil (0.35 g, 100%): R*_{f}* = 0.85 (EtOAc 100%); ¹H NMR (400 MHz, DMSO-*d*₆) *δ* 8.72-8.74 (m, Ar**H**), 8.11 (td, *J =* 1.7, 7.8 Hz, ArH), 8.01 (d, *J =* 7.8 Hz, Ar**H**), 7.66 (ddd, *J* = 1.2, 2.8, 7.7 Hz, Ar**H**), 3.16 (s, C**H₃**), 0.00 (s, Si(C**H₃**)₃).

### 3.1.3. Synthesis of diethyl ((N-(trimethylsilyl)pyridine-2-sulfonimidoyl)methyl)phosphonate

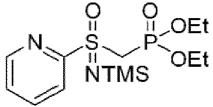

Using reaction (4), methyl(pyridin-2-yl)((trimethylsilyl)imino)-λ⁶-sulfanone (0.24 g, 1.04 mmol) was fully dissolved in THF, and then diethyl chlorophosphate (0.18 mL, 1.25 mmol) and 2.0 M n-BuLi cyclohexane solution (1.14 mL, 2.28 mmol) were reacted therewith to synthesize diethyl ((N-(trimethylsilyl)pyridine-2-sulfonimidoyl)methyl)phosphonate as a pale yellow transparent oil (0.15 g, 48%): R*_{f}* = 0.43 (n-hexane 1 : EtOAc 1); ¹H NMR (400 MHz, DMSO-*d*₆) *δ* 8.71-8.73 (m, Ar**H**), 8.11 (td, *J* = 1.7, 7.8 Hz, Ar**H**), 7.99 (d, *J =* 7.9 Hz, Ar**H**), 7.66 (ddd, *J* = 1.1, 2.9, 7.7 Hz, Ar**H**), 4.23 (d, *J* = 16.0 Hz, SC**H₂P**), 3.89-4.05 (m, P(OC**H₂**CH₃)**₂**), 1.13 (q, *J =* 7.0 Hz, P(OCH₂C**H₃**)**₂**), 0.02 (s, Si(C**H₃**)**₃**).

### 3.1.4. Synthesis of (E)-(2-chlorostyryl)(imino)(pyridin-2-yl)-λ⁶-sulfanone (Chemical Formula 1-15)

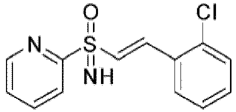

Using reaction (5)-1, diethyl ((N-(trimethylsilyl)pyridine-2-sulfonimidoyl)methyl)phosphonate (0.85 g, 2.91 mmol) was fully dissolved in THF, and then 2-chlorobenzaldehyde (0.41 g, 2.91 mmol) and 2.0 M n-BuLi cyclohexane solution (2.18 mL, 4.36 mmol) were reacted therewith to synthesize compound 1-15 as a white powder (0.55 g, 68%): R*_{f}* = 0.38 (EtOAc 100%); ¹H NMR (400 MHz, DMSO-*d*₆) *δ* 8.72 (d, *J* = 4.4 Hz, Ar**H**), 8.09-8.17 (m, 3 Ar**H**), 7.98 (d, *J* = 7.8 Hz, Ar**H**), 7.90 (d, *J* = 15.3 Hz, (*E*)-isomeric **H**), 7.56-7.67 (m, 2Ar**H**, (*E*)-isomeric **H**), 7.47 (t, *J* = 7.3 Hz, Ar**H**), 7.39 (d, *J* = 7.6 Hz, Ar**H**), 4.97 (s, N**H**); ¹³C NMR (400 MHz, DMSO-*d*₆) *δ* 160.8, 150.3, 139.2, 137.0, 134.3, 132.7, 132.5, 130.8, 130.6, 129.4, 128.3, 127.3, 121.8.

### Example 3.2. Synthesis of (E)-(3-chlorostyryl)(imino)(pyridin-2-yl)-λ⁶-sulfanone (Chemical Formula 1-16)

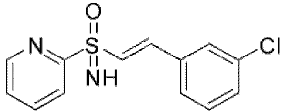

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.85 g, 2.91 mmol) was fully dissolved in THF, and then 3-chlorobenzaldehyde (0.50 g, 3.49 mmol) and 2.0 M n-BuLi cyclohexane solution (2.62 mL, 5.23 mmol) were reacted therewith to synthesize compound 1-16 as a white powder (0.68 g, 70%): R*_{f}* = 0.40 (EtOAc 100%); ¹H NMR (400 MHz, DMSO-*d*₆) *δ* 8.70-8.71 (m, Ar**H**), 8.10-8.16 (m, 3 Ar**H**), 7.43-7.90 (m, 4Ar**H**, (*E*)-isomeric 2**H**), 4.86 (s, N**H**).

### Example 3.3. Synthesis of (E)-imino(pyridin-2-yl)(2-(trifluoromethyl)styryl)-λ⁶-sulfanone (Chemical Formula 1-17)

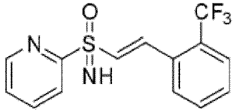

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.45 g, 1.54 mmol) was fully dissolved in THF, and then 3-(trifluoromethyl)benzaldehyde (0.25 g, 1.84 mmol) and 2.0 M *n*-BuLi cyclohexane solution (1.15 mL, 2.31 mmol) were reacted therewith to synthesize compound 1-17 as a white powder (0.24 g, 50%): R*_{f}* = 0.38 (EtOAc 4/ n-hexane 1); ¹H NMR (400 MHz, DMSO-*d*₆) *δ* 8.71 (d, *J =* 4.5 Hz, Ar**H**), 8.05-8.16 (m, 3Ar**H**), 7.64-7.84 (m, 4Ar**H**, (*E*)-isomeric 2**H**), 4.50 (s, N**H**).

### Example 3.4. Synthesis of (E)-imino(pyridin-2-yl)(2-(trifluoromethoxy)styryl)-λ ⁶ -sulfanone (Chemical Formula 1-18)

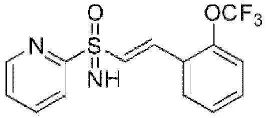

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.60 g, 2.05 mmol) was fully dissolved in THF, and then 2-(trifluoromethoxy)benzaldehyde (0.36 g, 2.46 mmol) and 2.0 M *n*-BuLi cyclohexane solution (1.60 mL, 3.07 mmol) were reacted therewith to synthesize compound 1-18 as a white powder (0.38 g, 57%): R*_{f}* = 0.28 (EtOAc 4/ n-hexane 1); ¹H NMR (400 MHz, DMSO-*d*₆) *δ* 8.03-8.16 (m, 4ArH), 7.44-7.74 (m, 4ArH, (E)-isomeric 2H), 4.97 (s, NH); ¹³C NMR (400 MHz, DMSO-*d*₆) *δ* 160.2, 149.7, 146.4, 138.6, 133.4, 132.5, 132.4, 129.0, 128.0, 126.7, 125.7, 121.6, 121.3.

### Example 3.5. Synthesis of (E)-(2,6-dichlorostyryl)(imino)(pyridin-2-yl)-λ⁶-sulfanone (Chemical Formula 1-19)

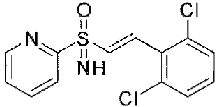

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.60 g, 2.05 mmol) was fully dissolved in THF, and then 2,6-dichlorobenzaldehyde (0.43 g, 2.46 mmol) and 2.0 M n-BuLi cyclohexane solution (1.60 mL, 3.07 mmol) were reacted therewith to synthesize compound 1-19 as a white powder (0.30 g, 47%): R*_{f}* = 0.51 (EtOAc 4/ *n*-hexane 1); ¹H NMR (400 MHz, DMSO-*d*₆) *δ* 8.73 (dd, *J =* 0.9, 4.1 Hz, Ar**H**), 8.11-8.16 (m, 2Ar**H**), 7.64-7.70 (m, Ar**H**, (*E*)-isomeric **H**), 7.58 (d, *J* = 8.0 Hz, Ar**H**), 7.42-7.50 (m, Ar**H**, (*E*)-isomeric **H**), 5.01 (s, N**H**).

### Example 3.6. Synthesis of (E)-(4-chlorostyryl)(imino)(pyridin-2-yl)-λ⁶-sulfanone (Chemical Formula 1-20)

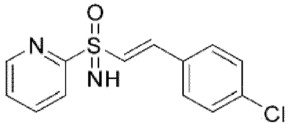

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.40 g, 1.37 mmol) was fully dissolved in THF, and then 4-chlorobenzaldehyde (0.23 g, 1.64 mmol) and 2.0 M *n*-BuLi cyclohexane solution (1.10 mL, 2.05 mmol) were reacted therewith to synthesize compound 1-20 as a white powder (0.22 g, 58%): R*_{f}* = 0.52 (EtOAc 4/ *n*-hexane 1); ¹H NMR (400 MHz, DMSO-*d*₆) *δ* 8.70 (d, *J =* 3.9 Hz, Ar**H**), 8.07-8.15 (m, 2Ar**H**), 7.78 (d, *J =* 7.2 Hz, 2Ar**H**), 7.48-7.65 (m, 3Ar**H**, (*E*)-isomeric 2**H**), 4.84 (s, N**H**).

### Example 3.7. Synthesis of (E)-(5-chloropyridin-2-yl)(2-chlorostyryl)(imino)-λ⁶-sulfanone (Chemical Formula 1-21)

### 3.7.1. Synthesis of 5-chloro-2-(methylthio)pyridine

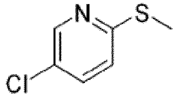

Using reaction (1), 5-chloro-2-fluoropyridine (0.20 g, 1.52 mmol) was fully dissolved in DMF and then sodium thiomethoxide (0.11 g, 1.52 mmol) was reacted therewith to synthesize 5-chloro-2-(methylthio)pyridine as a transparent oil (0.18 g, 75%): R*_{f}* = 0.67 (*n-*hexane 5 : EtOAc 1); ¹H NMR (400 MHz, DMSO-*d*₆) *δ* 8.39 (d, *J =* 2.4 Hz, ArH), 7.45 (dd, *J* = 2.5, 8.6 Hz, Ar**H**), 7.12 (d, *J =* 8.6 Hz, Ar**H**), 2.55 (s, C**H₃**).

### 3.7.2. Synthesis of (5-chloropyridin-2-yl)(imino)(methyl)-λ⁶-sulfanone

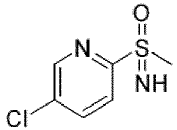

Using reaction (2), 5-chloro-2-(methylthio)pyridine (0.18 g, 1.13 mmol) was fully dissolved in MeOH, and then diacetoxyiodobenzene (0.84 g, 2.61 mmol) and ammonium carbonate (0.16 g, 1.70 mmol) were reacted therewith to synthesize (5-chloropyridin-2-yl)(imino)(methyl)-λ⁶-sulfanone (0.18 g, 83%) as a white powder: R*_{f}* = 0.41 (EtOAc 100%); ¹H NMR (400 MHz, DMSO-*d*₆) *δ* 8.79 (d, *J =* 2.1 Hz, Ar**H**), 8.24 (dd, *J =* 2.4, 8.4 Hz, Ar**H**), 8.08 (d, *J* = 8.3 Hz, Ar**H**), 4.53 (s, N**H**), 3.17 (s, C**H₃**).

### 3.7.3. (5-chloropyridin-2-yl)(methyl)((trimethylsilyl)imino)-λ ⁶ -sulfanone

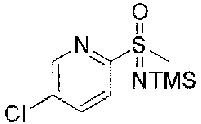

Using reaction (3), hexamethyldisilazane (0.28 mL, 1.34 mmol) was added to (5-chloropyridin-2-yl)(imino)(methyl)-λ⁶-sulfanone (0.17 g, 0.89 mmol) to synthesize a transparent (5-chloropyridin-2-yl)(methyl)((trimethylsilyl)imino)-λ⁶-sulfanone (0.24 g, 100%): R*_{f}* = 0.96 (EtOAc 100%); ¹H NMR (400 MHz, DMSO-*d*₆) *δ* 8.82 (d, *J =* 2.3 Hz, ArH), 8.24 (dd, *J* = 2.4, 8.4 Hz, ArH), 8.02 (d, *J* = 8.4 Hz), 3.17 (s, C**H₃**), 0.00 (s, Si(OC**H₃**)**₃**).

### 3.7.4. Synthesis of diethyl ((5-chloropyridine-2-sulfonimidoyl)methyl)phosphonate

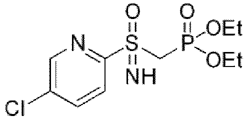

Using reaction (4), (5-chloropyridin-2-yl)(methyl)((trimethylsilyl)imino)-λ⁶-sulfanone (0.24 g, 0.92 mmol) was fully dissolved in THF, and then diethyl chlorophosphate (0.19 mL, 1.29 mmol) and 2.0 M *n*-BuLi cyclohexane solution (1.01 mL, 2.03 mmol) were reacted therewith to synthesize diethyl ((5-chloropyridine-2-sulfonimidoyl)methyl)phosphonate as a yellow oil (0.067 g, 22%): R*_{f}* = 0.19 (EtOAc 100%); ¹H NMR (400 MHz, DMSO-*d*₆) *δ* 8.81 (d, *J =* 2.0 Hz, Ar**H**), 8.25 (dd, *J =* 2.4, 8.5 Hz, Ar**H**), 8.10 (d, *J =* 8.4 Hz, Ar**H**), 4.86 (s, N**H**), 4.20-4.34 (m, SC**H₂**P), 3.96-4.02 (m, P(OC**H₂**CH₃)**₂**), 1.15 (q, *J* = 5.9 Hz, P(OCH₂C**H₃**)**₂**).

### 3.7.5. Synthesis of (E)-(5-chloropyridin-2-yl)(2-chlorostyryl)(imino)-λ⁶-sulfanone (Chemical Formula 1-21)

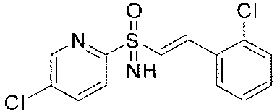

Using reaction (5)-1, diethyl ((5-chloropyridine-2-sulfonimidoyl)methyl)phosphonate (0.65 g, 1.99 mmol) was fully dissolved in THF, and then 2-chlorobenzaldehyde (0.34 g, 2.39 mmol) and 2.0 M *n*-BuLi cyclohexane solution (1.50 mL, 2.98 mmol) were reacted therewith to synthesize compound 1-21 as a white powder (0.45 g, 62%): R*_{f}* = 0.56 (EtOAc 2/ *n*-hexane 1); ¹H NMR (400 MHz, DMSO-*d*₆) *δ* 8.79 (d, *J =* 1.8 Hz, Ar**H**), 7.95-8.18 (m, 2Ar**H**), 7.91 (d, *J* = 15.3 Hz, (*E*)-isomeric **H**), 7.38-7.63 (m, 3Ar**H**, (*E*)-isomeric **H**), 5.10 (s, N**H**).

### Example 3.8. Synthesis of (E)-(2,6-difluorostyryl)(imino)(pyridin-2-yl)-λ⁶-sulfanone (Chemical Formula 1-22)

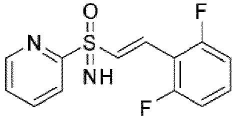

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.50 g, 1.71 mmol) was fully dissolved in THF, and then 2,6-difluorobenzaldehyde (0.23 g, 2.05 mmol) and 2.0 M *n*-BuLi cyclohexane solution (1.30 mL, 2.56 mmol) were reacted therewith to synthesize compound 1-22 as a white powder (0.47 g, 98%): R*_{f}* = 0.34 (EtOAc 4/ *n*-hexane 1); ¹H NMR (400 MHz, DMSO-*d*₆) *δ* 8.72-8.73 (m, ArH), 8.09-8.17 (m, 2Ar**H**), 7.56-7.67 (m, 2Ar**H**, (*E*)-isomeric **H**), 7.42 (d, *J =* 15.7 Hz, (*E*)-isomeric **H**), 7.26 (t, *J* = 9.0 Hz, 2Ar**H**), 5.07 (s, N**H**).

### Example 3.9. Synthesis of (E)-imino(pyridin-2-yl)(2,4,6-trifluorostyryl)-λ⁶-sulfanone (Chemical Formula 1-23)

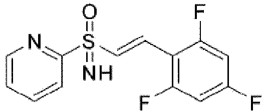

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.50 g, 1.71 mmol) was fully dissolved in THF, and then 2,4,6-trifluorobenzaldehyde (0.33 g, 2.05 mmol) and 2.0 M *n*-BuLi cyclohexane solution (1.30 mL, 2.56 mmol) were reacted therewith to synthesize compound 1-23 as a white powder (0.44 g, 86%): R*_{f}* = 0.34 (EtOAc 4/ *n*-hexane 1); ¹H NMR (400 MHz, DMSO-*d*₆) *δ* 8.72 (d, *J* = 4.7 Hz, Ar**H**), 8.08-8.16 (m, 2Ar**H**), 7.64-7.67 (m, Ar**H**), 7.53 (d, *J =* 15.7 Hz, (*E*)-isomeric **H**), 7.36-7.42 (m, 2Ar**H**, (*E*)-isomeric **H**), 5.06 (s, N**H**).

### Example 3.10. Synthesis of (E)-(2-fluorostyryl)(imino)(pyridin-2-yl)-λ⁶-sulfanone (Chemical Formula 1-24)

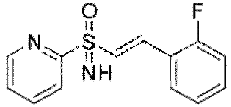

The method used for Examples 3.1.1 to 3.1.3 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.90 g, 3.08 mmol) was fully dissolved in THF, and then 2-fluorobenzaldehyde (0.39 g, 3.69 mmol) and 2.0 M *n*-BuLi cyclohexane solution (2.30 mL, 4.62 mmol) were reacted therewith to synthesize compound 1-24 as a white powder (0.47 g, 98%): R*_{f}* = 0.34 (EtOAc 4/ n-hexane 1); ¹H NMR (400 MHz, DMSO-*d*₆) *δ* 8.71 (d, *J =* 4.6 Hz, Ar**H**), 8.08-8.17 (m, 2Ar**H**), 7.87-7.92 (m, Ar**H**), 7.49-7.69 (m, 2Ar**H**, (*E*)-isomeric 2**H**), 7.25-7.35 (m, 2Ar**H**), 4.94 (s, N**H**).

### Example 3.11. Synthesis of (E)-imino(3-methoxypyridin-2-yl)(2-(trifluoromethyl)styryl)-λ⁶-sulfanone (Chemical Formula 1-25)

### 3.11.1. Synthesis of 3-methoxy-2-(methylthio)pyridine

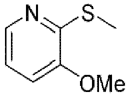

Using reaction (1), 2-bromo-3-methoxypyridine (0.20 g, 1.06 mmol) was fully dissolved in DMF, and then the solution was reacted with sodium thiomethoxide (0.15 g, 2.13 mmol) to synthesize 3-methoxy-2-(methylthio)pyridine as a transparent oil (0.096 g, 58%): R*_{f}* = 0.50 (n-hexane 9 : EtOAc 1); ¹H NMR (400 MHz, DMSO-*d*₆) *δ* 8.06 (dd, *J =* 1.2, 4.8 Hz, Ar**H**), 7.26 (dd, *J =* 1.2, 8.1 Hz, Ar**H**), 7.10 (dd, *J =* 4.8, 8.1 Hz, Ar**H**), 3.84 (s, OC**H₃**), 2.43 (s, C**H₃**).

### 3.11.2. Synthesis of imino(3-methoxypyridin-2-yl)(methyl)-λ ⁶ -sulfanone

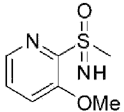

Using reaction (2), 3-methoxy-2-(methylthio)pyridine (1.23 g, 7.92 mmol) was fully dissolved in MeOH, and then diacetoxyiodobenzene (5.87 g, 18.23 mmol) and ammonium carbonate (1.14 g, 11.89 mmol) were reacted therewith to synthesize imino(3-methoxypyridin-2-yl)(methyl)-λ⁶-sulfanone as a transparent oil (0.97 g, 66%): R*_{f}* = 0.21 (DCM 4 : Acetone 1); ¹H NMR (400 MHz, DMSO-*d*₆) *δ* 8.17 (dd, *J* = 1.1, 4.5 Hz, Ar**H**), 7.76 (dd, *J =* 1.0, 8.5 Hz, Ar**H**), 7.63 (dd, *J =* 4.5, 8.5 Hz, Ar**H**), 4.16 (s, N**H**), 3.96 (s, OC**H₃**), 3.24 (s, C**H₃**).

### 3.11.3. Synthesis of (3-methoxypyridin-2-yl)(methyl)((trimethylsilyl)imino)-λ⁶-sulfanone

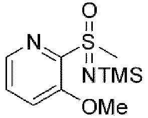

Using reaction (3), hexamethyldisilazane (1.23 mL, 5.85 mmol) was added to imino(3-methoxypyridin-2-yl)(methyl)-λ⁶-sulfanone (0.22 g, 1.17 mmol) to synthesize (3-methoxypyridin-2-yl)(methyl)((trimethylsilyl)imino)-λ⁶-sulfanone as a yellow oil (0.25 g, 81%): R*_{f}* = 0.69 (DCM 4 : Acetone 1); ¹H NMR (400 MHz, DMSO-*d*₆) *δ* 8.18 (dd, *J =* 1.2, 4.5 Hz, Ar**H**), 7.74 (dd, *J =* 1.0, 8.4 Hz, Ar**H**), 7.63 (dd, *J =* 4.4, 8.4 Hz, Ar**H**), 3.93 (s, OC**H₃**), 3.19 (s, C**H₃**), 0.00 (s, Si(OC**H₃**)**₃**).

### 3.11.4. Synthesis of diethyl ((3-methoxypyridine-2-sulfonimidoyl)methyl)phosphonate

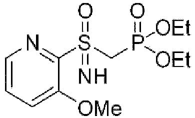

Using reaction (4), (3-methoxypyridin-2-yl)(methyl)((trimethylsilyl)imino)-λ⁶-sulfanone (0.24 g, 0.95 mmol) was fully dissolved in THF, and then diethyl chlorophosphate (0.19 mL, 1.33 mmol) and 2.0 M n-BuLi cyclohexane solution (1.04 mL, 2.09 mmol) were reacted therewith to synthesize diethyl ((3-methoxypyridine-2-sulfonimidoyl)methyl)phosphonate (0.19 g, 60%) as a yellow transparent oil: R*_{f}* = 0.20 (DCM 4 : Acetone 1); ¹H NMR (400 MHz, DMSO-*d*₆) *δ* 8.17-8.21 (m, Ar**H**), 7.76 (d, *J =* 8.4 Hz, Ar**H**), 7.64 (td, *J =* 4.6, 9.20 Hz, Ar**H**), 4.60 (s, N**H**), 4.24-4.40 (m, SC**H₂**P), 3.97-4.06 (m, P(OC**H₂**CH₃)**₂**), 3.96 (s, OC**H₃**), 1.14 (td, *J =* 1.0, 7.0 Hz, P(OCH₂C**H₃**)**₂**).

### 3.11.5. Synthesis of (E)-imino(3-methoxypyridin-2-yl)(2-(trifluoromethyl)styryl)-λ⁶-sulfanone (Chemical Formula 1-25)

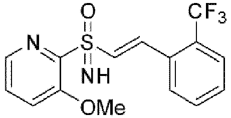

Using reaction (5)-1, diethyl ((3-methoxypyridine-2-sulfonimidoyl)methyl)phosphonate (0.13 g, 0.39 mmol) was fully dissolved in THF, and then 2-(trifluoromethyl)benzaldehyde (0.082 g, 0.47 mmol) and 2.0 M n-BuLi cyclohexane solution (0.24 mL, 1.2 mmol) were reacted therewith to synthesize compound 1-25 as a white powder (0.044 g, 33%): R*_{f}* = 0.69 (DCM 4 : Acetone 1); ¹H NMR (400 MHz, DMSO-*d*₆) *δ* 8.20 (dd, *J* = 1.2, 4.5 Hz, Ar**H**), 8.12 (d, *J* = 7.8 Hz, Ar**H**), 7.84-7.89 (m, Ar**H**, (*E*)-isomeric **H**), 7.74-7.78 (m, 2 Ar**H**), 7.62-7.69 (m, 2 Ar**H**, (*E*)-isomeric **H**), 4.81 (s, N**H**), 3.92 (s, OC**H₃**).

### Example 3.12. Synthesis of (E)-imino(4-methoxypyridin-2-yl)(2-(trifluoromethyl)styryl)-λ⁶-sulfanone (Chemical Formula 1-26)

### 3.12.1. Synthesis of 4-methoxy-2-(methylthio)pyridine

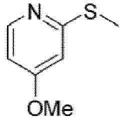

Using reaction (1), 2-bromo-4-methoxypyridine (1.00 g, 5.32 mmol) was well dissolved in DMF, and then sodium thiomethoxide (0.75 g, 10.6 mmol) was reacted therewith to synthesize 4-methoxy-2-(methylthio)pyridine as a clear oil (0.36 g, 44%): R*_{f}* = 0.20 (n-hexane 9 : EtOAc 1); ¹H NMR (400 MHz, DMSO-*d*₆) *δ* 8.22 (d, *J* = 5.8 Hz, ArH), 6.83 (d, *J* = 2.2 Hz, Ar**H**), 6.71 (dd, *J* = 2.2, 5.8, ArH), 3.81 (s, OC**H₃**), 2.49 (s, C**H₃**).

### 3.12.2. Synthesis of imino(4-methoxypyridin-2-yl)(methyl)-λ⁶-sulfanone

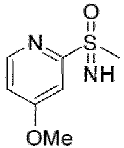

Using reaction (2), 4-methoxy-2-(methylthio)pyridine (0.36 g, 2.33 mmol) was fully dissolved in MeOH, and then diacetoxyiodobenzene (1.72 g, 5.35 mmol) and ammonium carbonate (0.34 g, 3.49 mmol) were reacted therewith to synthesize imino(4-methoxypyridin-2-yl)(methyl)-λ⁶-sulfanone as a white solid (0.33 g, 75%): R*_{f}* = 0.32 (EtOAc 10 : MeOH 1);¹H NMR (400 MHz, DMSO-*d*₆) *δ* 8.51 (d, *J =* 5.6 Hz, ArH), 7.59 (d, *J* = 2.4 Hz, Ar**H**), 7.19 (dd, *J =* 2.5, 5.6 Hz, Ar**H**), 4.41 (s, N**H**), 3.92 (s, OC**H₃**), 3.14 (s, C**H₃**).

### 3.12.3. Synthesis of (4-methoxypyridin-2-yl)(methyl)((trimethylsilyl)imino)-λ⁶-sulfanone

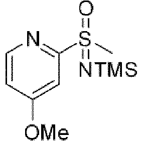

Using reaction (3), hexamethyldisilazane (0.66 mL, 3.14 mmol) was added to imino(4-methoxypyridin-2-yl)(methyl)-λ⁶-sulfanone (0.33 g, 2.09 mmol) to synthesize (4-methoxypyridin-2-yl)(methyl)((trimethylsilyl)imino)-λ⁶-sulfanone as a pale yellow (0.54 g, 100%): R*_{f}* = 0.70 (EtOAc 10 : MeOH 1); ¹H NMR (400 MHz, DMSO-*d*₆) *δ* 8.54 (d, *J =* 5.6 Hz, ArH), 7.47 (d, *J =* 2.4 Hz, Ar**H**), 7.21 (dd, *J* = 2.5, 5.6 Hz, Ar**H**), 3.93 (s, OC**H₃**), 3.14 (s, C**H₃**) 0.00 (s, Si(OC**H₃**)**₃**).

### 3.12.4. Synthesis of diethyl ((4-methoxy-N-(trimethylsilyl)pyridine-2-sulfonimidoyl)methyl)phosphonate

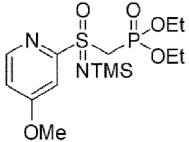

Using reaction (4), (4-methoxypyridin-2-yl)(methyl)((trimethylsilyl)imino)-λ⁶-sulfanone (0.54 g, 2.10 mmol) was fully dissolved in THF, and then diethyl chlorophosphate (0.36 mL, 2.52 mmol) and 2.0 M *n* -BuLi cyclohexane solution (2.31 mL, 4.62 mmol) were reacted therewith to synthesize diethyl ((4-methoxy-N-(trimethylsilyl)pyridine-2-sulfonimidoyl)methyl)phosphonate as a transparent oil (0.56 g, 67%): R*_{f}* = 0.42 (EtOAc 10 : MeOH 1); ¹H NMR (400 MHz, DMSO-*d*₆) *δ* 8.53-8.55 (m, Ar**H**), 7.60 (d, *J* = 2.4 Hz, Ar**H**), 7.45 (d, *J =* 2.4 Hz, Ar**H**), 7.22 (dd, *J =* 2.5, 5.6 Hz, Ar**H**), 4.16-4.29 (m, SC**H₂**P), 3.94-4.04 (m, P(OC**H₂**CH₃)**₂**), 3.93 (s, OC**H₃**), 1.12-1.18 (m, P(OCH₂C**H₃**)**₂**), 0.03 (s, Si(C**H₃**)**₃**).

### 3.12.5. Synthesis of (E)-imino(4-methoxypyridin-2-yl)(2-(trifluoromethyl)styryl)-λ⁶-sulfanone (Chemical Formula 1-26)

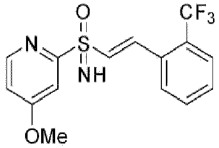

Using reaction (5)-1, diethyl ((4-methoxy-N-(trimethylsilyl)pyridine-2-sulfonimidoyl)methyl)phosphonate (0.38 g, 0.96 mmol) was fully dissolved in THF, and then 2-(trifluoromethyl)benzaldehyde (0.15 mL, 1.16 mmol) and 2.0 M *n*-BuLi cyclohexane solution (0.58 mL, 1.16 mmol) were reacted therewith to synthesize compound 1-26 as a white powder (0.17 g, 51%): R*_{f}* = 0.34 (EtOAc 100%); ¹H NMR (400 MHz, DMSO-*d*₆) *δ* 8.51 (d, *J* = 5.6 Hz, Ar**H**), 8.06 (d, *J =* 7.8 Hz, ArH), 7.80-7.84 (m, Ar**H**, (*E*)-isomeric **H**), 7.73 (t, *J =* 7.5 Hz, Ar**H**), 7.62-7.67 (m, 2 Ar**H**, (*E*)-isomeric **H**), 7.21 (dd, *J =* 2.5, 5.6 Hz, Ar**H**), 4.99 (s, N**H**), 3.93 (s, OC**H₃**); ¹³C NMR (100 MHz, DMSO-*d*₆) *δ* 167.2, 162.5, 151.6, 136.5, 133.8, 133.6, 131.2, 129.5, 127.7 (q, *J*_{C-F} = 29.6 Hz), 126.7 (q, *J*_{C-F} = 5.6 Hz), 124.5 (q, *J*_{C-F} = 272.1 Hz, 113.1, 107.8, 56.6.

### Example 3.13. Synthesis of (E)-(2-chlorostyryl)(imino)(4-methoxypyridin-2-yl)-λ⁶-sulfanone (Chemical Formula 1-30)

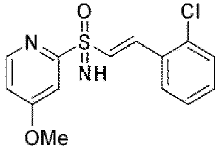

The method used for Examples 3.12.1 to 3.12.4 was used to synthesize diethyl ((N-(trimethylsilyl)pyridine-2-sulfonimidoyl)methyl)phosphonate.

Using reaction (5)-1, diethyl ((N-(trimethylsilyl)pyridine-2-sulfonimidoyl)methyl)phosphonate (0.091 g, 0.23 mmol) was fully dissolved in THF, and then 2-chlorobenzaldehyde (0.03 mL, 0.28 mmol) and 2.0 M n-BuLi cyclohexane solution (0.14 mL, 0.28 mmol) were reacted therewith to synthesize compound 1-30 as a white powder (0.030 g, 42%): R*_{f}* = 0.21 (EtOAc 100%); ¹H NMR (400 MHz, DMSO-*d*₆) *δ* 8.51 (d, *J =* 5.6 Hz, Ar**H**), 7.96 (dd, *J* = 1.5, 7.8 Hz, Ar**H**), 7.89 (d, *J* = 15.8 Hz, (*E*)-isomeric **H**), 7.67 (d, *J* = 2.4 Hz, Ar**H**), 7.56-7.63 (m, Ar**H**, (*E*)-isomeric **H**), 7.47 (td, *J =* 1.6, 7.4 Hz, Ar**H**), 7.39 (t, *J* = 7.5 Hz, Ar**H**), 7.20 (dd, *J* = 2.5, 5.6 Hz, Ar**H**), 4.96 (s, N**H**), 3.93 (s, OC**H₃**); ¹³C NMR (100 MHz, DMSO-*d*₆) *δ* 167.3, 162.3, 151.7, 149.1, 147.9, 138.9, 135.8, 134.9, 132.2, 127.2, 113.3, 107.8, 56.6.

### Example 3.14. Synthesis of (E)-(2-fluorostyryl)(imino)(4-methoxypyridin-2-yl)-λ⁶-sulfanone (Chemical Formula 1-31)

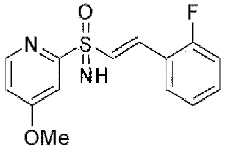

The method used for Examples 3.12.1 to 3.12.4 was used to synthesize diethyl ((N-(trimethylsilyl)pyridine-2-sulfonimidoyl)methyl)phosphonate .

Using reaction (5)-1, diethyl ((N-(trimethylsilyl)pyridine-2-sulfonimidoyl)methyl)phosphonate (0.31 g, 0.78 mmol) was fully dissolved in THF, and then 2-fluorobenzaldehyde (0.10 mL, 0.94 mmol) and 2.0 M n-BuLi cyclohexane solution (0.47 mL, 0.94 mmol) were reacted therewith to synthesize 1-31 as a white powder (0.057 g, 25%): R*_{f}* = 0.27 (EtOAc 100%); ¹H NMR (400 MHz, DMSO-*d*₆) *δ* 8.51 (d, *J =* 5.6 Hz, ArH), 7.89 (t, *J =* 7.6 Hz, Ar**H**), 7.64-7.68 (m, Ar**H**, (*E*)-isomeric **H**), 7.48-7.54 (m, Ar**H**, (*E*)-isomeric **H**), 7.25-7.34 (m, 2 Ar**H**), 7.19 (dd, *J* = 2.4, 5.6 Hz, Ar**H**), 4.93 (s, N**H**), 3.93 (s, OC**H₃**); ¹³C NMR (100 MHz, DMSO-*d*₆) *δ* 167.2, 162.6, 162.2, 159.7, 151.6, 134.0 (d, *J*_{C-F} = 3.9 Hz), 133.4 (d, *J*_{C-F} = 8.8 Hz), 131.8 (d, *J*_{C-F} = 5.4 Hz), 130.2 (d, *J*_{C-F} = 1.9 Hz), 125.6 (d, *J*_{C-F} = 3.4 Hz), 120.9 (d, *J*_{C-F} = 11.1 Hz), 116.5 (d, *J*_{C-F} = 21.4 Hz), 113.0, 107.8, 56.6.

### Example 3.15. Synthesis of (E)-(3-chloropyridin-2-yl)(2-chlorostyryl)(imino)-λ⁶-sulfanone (Chemical Formula 1-38)

### 3.15.1. Synthesis of 3-chloro-2-(methylthio)pyridine

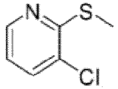

Using reaction (1), 3-chloro-2-fluoropyridine (1.00 g, 3.60 mmol) was fully dissolved in DMF, and then sodium thiomethoxide (0.53 g, 7.60 mmol) was reacted therewith to synthesize 3-chloro-2-(methylthio)pyridine as a transparent oil (0.85 g, 70%): R*_{f}* = 0.69 (n-hexane 5 : EtOAc 1); ¹H NMR (400 MHz, DMSO-*d*₆) *δ* 8.46 (dd, *J* = 1.3, 4.7 Hz, Ar**H**), 7.84 (dd, *J=* 1.3, 7.9 Hz, Ar**H**), 7.18 (dd, *J* = 4.7, 7.9 Hz, Ar**H**), 2.52 (s, C**H₃**).

### 3.15.2. Synthesis of (3-chloropyridin-2-yl)(imino)(methyl)-λ⁶-sulfanone

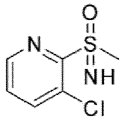

Using reaction (2), 3-chloro-2-(methylthio)pyridine (0.20 g, 1.23 mmol) was fully dissolved in MeOH, and then diacetoxyiodobenzene (0.91 g, 2.82 mmol) and ammonium carbonate (0.18 g, 1.84 mmol) were reacted therewith to synthesize (3-chloropyridin-2-yl)(imino)(methyl)-λ⁶-sulfanone as a white oil (0.15 g, 63%): R*_{f}* = 0.37 (EtOAc 100%); ¹H NMR (400 MHz, DMSO-*d*₆) *δ* 8.60 (dd, *J =* 1.2, 4.6 Hz, Ar**H**), 8.19 (dd, *J =* 1.2, 8.1 Hz, Ar**H**), 7.67 (dd, *J* = 4.5*,* 8.1 Hz, Ar**H**), 4.64 (s, N**H**), 3.35 (s, C**H₃**).

### 3.15.3. Synthesis of (3-chloropyridin-2-yl)(methyl)((trimethylsilyl)imino)-λ⁶-sulfanone

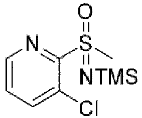

Using reaction (3), hexamethyldisilazane (0.24 mL, 1.16 mmol) was added to (3-chloropyridin-2-yl)(imino)(methyl)-λ⁶-sulfanone (0.15 g, 0.77 mmol) to synthesize (3-chloropyridin-2-yl)(methyl)((trimethylsilyl)imino)-λ⁶-sulfanone as a clear oil (0.19 g, 95%): R*_{f}* = 0.67 (*n*-hexane 1 : EtOAc 1); ¹H NMR (400 MHz, DMSO-*d*₆) *δ* 8.60 (dd, *J* = 1.1, 4.5 Hz, Ar**H**), 8.17 (dd, *J* = 1.2, 8.1 Hz, Ar**H**), 7.67 (dd, *J =* 4.6, 8.1 Hz, Ar**H**), 3.31 (s, C**H₃**), 0.02 (s, Si(OC**H₃**)**₃**).

### 3.15.4. Synthesis of diethyl ((3-chloropyridine-2-sulfonimidoyl)methyl)phosphonate

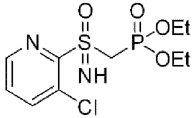

Using reaction (4), (3-chloropyridin-2-yl)(methyl)((trimethylsilyl)imino)-λ⁶-sulfanone (0.20 g, 0.73 mmol) was fully dissolved in THF, and then diethyl chlorophosphate (0.15 mL, 1.02 mmol) and 2.0 M n-BuLi cyclohexane solution (0.80 mL, 1.61 mmol) were reacted therewith to synthesize diethyl ((3-chloropyridine-2-sulfonimidoyl)methyl)phosphonate as a yellow oil (0.061 g, 26%): R*_{f}* = 0.35 (EtOAc 10 : MeOH 1); ¹H NMR (400 MHz, DMSO-*d*₆) *δ* 8.59 (dd, *J* = 1.2, 4.6 Hz, Ar**H**), 8.18 (dd, *J* = 1.2, 8.1 Hz, Ar**H**), 7.67 (dd, *J =* 4.6, 8.1 Hz, Ar**H**), 4.62 (s, N**H**), 4.11-4.19 (m, SC**H₂**P), 3.91-4.04 (m, P(OC**H**₂CH₃)**₂**), 1.20-1.30 (m, P(OCH₂C**H₃**)**₂**).

### 3.15.5. Synthesis of (E)-(3-chloropyridin-2-yl)(2-chlorostyryl)(imino)-λ⁶-sulfanone (Chemical Formula 1-38)

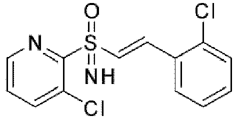

Using reaction (5)-1, diethyl ((3-chloropyridine-2-sulfonimidoyl)methyl)phosphonate (0.16 g, 0.48 mmol) was fully dissolved in THF, and then 2-chlorobenzaldehyde (0.06 mL, 0.58 mmol) and 2.0 M n-BuLi cyclohexane solution (0.29 mL, 0.58 mmol) were reacted therewith to synthesize compound 1-38 as a white powder (0.017 g, 11%): R*_{f}* = 0.46 (EtOAc 100%); ¹H NMR (400 MHz, DMSO-*d*₆) *δ* 8.61 (dd, *J =* 1.3, 4.5 Hz, Ar**H**), 8.19 (dd, *J =* 1.3, 8.1 Hz, Ar**H**), 8.03 (dd, *J* = 1.6, 7,7 Hz, Ar**H**), 7.95 (d, *J* = 15.3 Hz, (*E*)-isomeric **H**), 7.66-7.73 (m, Ar**H**, (*E*)-isomeric **H**), 7.59 (dd, *J* = 1.2, 8.0 Hz, Ar**H**), 7.42-7.52 (m, 2 Ar**H**), 5.26 (s, N**H**); ¹³C NMR (100 MHz, DMSO-*d*₆) *δ* 157.0, 147.4, 141.5, 137.8, 134.4, 132.8, 131.2, 130.9, 130.6, 129.5, 128.6, 128.4, 128.3.

### Example 3.16. Synthesis of (E)-(2-chlorostyryl)(imino)(5-methoxypyridin-2-yl)-λ⁶-sulfanone (Chemical Formula 1-39)

### 3.16.1. Synthesis of 5-methoxy-2-(methylthio)pyridine

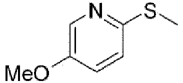

Using reaction (1), 2-bromo-5-methoxypyridine (3.00 g, 15.9 mmol) was fully dissolved in DMF, and then sodium thiomethoxide (4.47 g, 63.8 mmol) was reacted therewith to synthesize 5-methoxy-2-(methylthio)pyridine as a clear oil (0.84 g, 34%): R*_{f}* = 0.58 (*n-*hexane 3 : EtOAc 1); ¹H NMR (400 MHz, DMSO-*d*₆) *δ* 8.19 (d, *J =* 3.0 Hz, Ar**H**), 7.32 (dd, *J* = 3.0, 8.8 Hz, Ar**H**), 7.23 (d, *J* = 8.8 Hz, Ar**H**), 3.79 (s, OC**H₃**), 2.47 (s, C**H₃**).

### 3.16.2. Synthesis of imino(5-methoxypyridin-2-yl)(methyl)-λ ⁶ -sulfanone

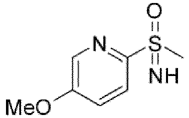

Using reaction (2), 5-methoxy-2-(methylthio)pyridine (0.84 g, 5.41 mmol) was fully dissolved in MeOH, and then diacetoxyiodobenzene (4.00 g, 12.43 mmol) and ammonium carbonate (0.78 g, 8.11 mmol) were reacted therewith to synthesize imino(5-methoxypyridin-2-yl)(methyl)-λ⁶-sulfanone as a white solid (0.92 g, 91%): R*_{f}* = 0.32 (EtOAc 10 : MeOH 1); ¹H NMR (400 MHz, DMSO-*d*₆) *δ* 8.40 (d, *J* = 2.8 Hz, Ar**H**), 8.03 (d, *J* = 8.7 Hz, Ar**H**), 7.62 (dd, *J* = 2.9, 8.7 Hz, Ar**H**), 4.25 (s, N**H**), 3.91 (s, OC**H₃**), 3.10 (s, C**H₃**).

### 3.16.3. Synthesis of (5-methoxypyridin-2-yl)(methyl)((trimethylsilyl)imino)-λ⁶-sulfanone

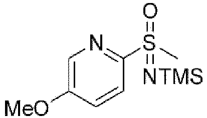

Using reaction (3), hexamethyldisilazane (0.34 mL, 1.61 mmol) was added to imino(5-methoxypyridin-2-yl)(methyl)-λ⁶-sulfanone (0.20 g, 1.07 mmol) to synthesize a yellow (5-methoxypyridin-2-yl)(methyl)((trimethylsilyl)imino)-λ⁶-sulfanone (0.31 g, 100%): R*_{f}* = 0.90 (EtOAc 10 : MeOH 1); ¹H NMR (400 MHz, DMSO-*d*₆) *δ* 8.41 (d, *J =* 2.7 Hz, Ar**H**), 7.96 (d, *J* = 8.7 Hz, Ar**H**), 7.62 (dd, *J* = 2.8, 8.7 Hz, Ar**H**), 3.92 (s, OC**H₃**), 3.10 (s, C**H₃**), 0.00 (s, Si(OC**H₃**)**₃**)-

### 3.16.4. Synthesis of diethyl ((5-methoxypyridine-2-sulfonimidoyl)methyl)phosphonate

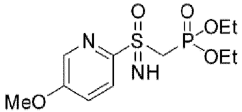

Using reaction (4), (5-methoxypyridin-2-yl)(methyl)((trimethylsilyl)imino)-λ⁶ - sulfanone (0.31 g, 1.21 mmol) was fully dissolved in THF, and then diethyl chlorophosphate (0.24 mL, 1.69 mmol) and 2.0 M n-BuLi cyclohexane solution (0.64 mL, 1.33 mmol) were reacted therewith to synthesize diethyl ((5-methoxypyridine-2-sulfonimidoyl)methyl)phosphonate as a transparent oil (0.17 g, 44%): R*_{f}* = 0.61 (EtOAc 10 : MeOH 1); ¹H NMR (400 MHz, DMSO-*d*₆) *δ* 8.40 (t, *J =* 3.0 Hz, Ar**H**), 8.04 (dd, *J =* 8.8, 12.1 Hz, Ar**H**), 7.62 (dd, *J =* 1.9, 5.8 Hz, Ar**H**), 4.56 (s, N**H**), 4.10-4.25 (m, SC**H₂**P), 3.94-4.04 (m, P(OC**H₂**CH₃)₂), 3.93 (s, OC**H**₃), 1.15 (q, *J =* 7.2 Hz, P(OCH₂C**H₃**)₂).

### 3.16.5. Synthesis of (E)-(2-chlorostyryl)(imino)(5-methoxypyridin-2-yl)-λ⁶-sulfanone (Chemical Formula 1-39)

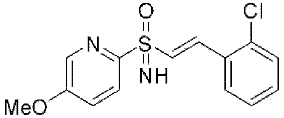

Using reaction (5)-1, diethyl ((5-methoxypyridine-2-sulfonimidoyl)methyl)phosphonate (0.19 g, 0.48 mmol) was fully dissolved in THF, and then 2-chlorobenzaldehyde (0.09 mL, 0.73 mmol) and 2.0 M *n*-BuLi cyclohexane solution (0.29 mL, 0.58 mmol) were reacted therewith to synthesize compound 1-39 as a white powder (0.030 g, 18%): R*_{f}* = 0.46 (EtOAc 100%); ¹H NMR (400 MHz, DMSO-*d*₆) *δ* 8.40 (d, *J =* 2.8 Hz, Ar**H**), 8.12 (d, *J =* 8.8 Hz, Ar**H**), 7.94 (d, *J =* 7.8 Hz, Ar**H**), 7.85 (d, *J =* 15.3 Hz, (E)-isomeric **H**), 7.62 (dd, *J =* 2.8, 8.8 Hz, Ar**H**), 7.53-7.57 (m, Ar**H**, (*E*)-isomeric **H**), 7.43-7.47 (m, Ar**H**), 7.38 (t, *J =* 7.5 Hz, Ar**H**), 4.80 (s, N**H**), 3.91 (s, OC**H₃**); ¹³C NMR (100 MHz, DMSO-*d*₆) *δ* 157.9, 152.0, 138.4, 136.1, 134.2, 133.2, 132.6, 130.9, 130.6, 129.3, 128.3, 123.9, 121.9, 56.7.

### Example 3.17. Synthesis of (E)-(2-chlorostyryl)(3-fluoropyridin-2-yl)(imino)-λ⁶-sulfanone (Chemical Formula 1-40)

### 3.17.1. Synthesis of 3-fluoro-2-(methylthio)pyridine

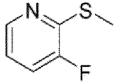

Using reaction (1), 2,3-difluoropyridine (0.20 g, 1.74 mmol) was fully dissolved in DMF, and then sodium thiomethoxide (0.12 g, 1.74 mmol) was reacted therewith to synthesize 3-fluoro-2-(methylthio)pyridine as a transparent oil (0.11 g, 45%): R*_{f}* = 0.64 (*n*-hexane 5 : EtOAc 1); ¹H NMR (400 MHz, DMSO-*d*₆) *δ* 8.28 (d, *J =* 4.8 Hz, Ar**H**), 7.20-7.26 (m, Ar**H**), 6.98-7.02 (m, Ar**H**), 2.59 (s, C**H₃**).

### 3.17.2. Synthesis of (3-fluoropyridin-2-yl)(imino)(methyl)-λ⁶-sulfanone

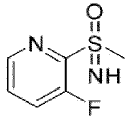

Using reaction (2), 3-fluoro-2-(methylthio)pyridine (0.32 g, 2.25 mmol) was fully dissolved in MeOH, and then diacetoxyiodobenzene (1.67 g, 5.17 mmol) and ammonium carbonate (0.32 g, 3.37 mmol) were reacted therewith to synthesize (3-fluoropyridin-2-yl)(imino)(methyl)-λ⁶-sulfanone as a white solid (0.25 g, 64%): R*_{f}* = 0.26 (EtOAc 100%); ¹H NMR (400 MHz, DMSO-*d*₆) *δ* 8.51 (d, *J =* 4.5 Hz, Ar**H**), 8.01 (t, *J =* 9.0 Hz, Ar**H**), 7.74-7.78 (m, Ar**H**), 4.75 (s, N**H**), 3.27 (s, C**H₃**).

### 3.17.3. Synthesis of (3-fluoropyridin-2-yl)(methyl)((trimethylsilyl)imino)-λ⁶-sulfanone

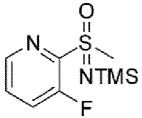

Using reaction (3), hexamethyldisilazane (0.45 mL, 2.17 mmol) was added to (3-fluoropyridin-2-yl)(imino)(methyl)-λ⁶-sulfanone (0.25 g, 1.45 mmol) to synthesize (3-fluoropyridin-2-yl)(methyl)((trimethylsilyl)imino)-λ⁶-sulfanone as a yellow oil (0.33 g, 93%): R*_{f}* = 0.77 (EtOAc 100%); ¹H NMR (400 MHz, DMSO-*d*₆) *δ* 8.52 (d, *J =* 4.4 Hz, Ar**H**), 7.99 (t, *J* = 9.3 Hz, ArH), 7.74-7.78 (m, Ar**H**), 3.26 (s, C**H₃**), 0.02 (s, Si(OC**H₃**)₃).

### 3.17.4. Synthesis of diethyl ((3-fluoro-N-(trimethylsilyl)pyridine-2-sulfonimidoyl)methyl)phosphonate

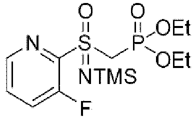

Using reaction (4), (3-fluoropyridin-2-yl)(methyl)((trimethylsilyl)imino)-λ⁶-sulfanone (0.33 g, 1.35 mmol) was fully dissolved in THF, and then diethyl chlorophosphate (0.27 mL, 1.89 mmol) and 2.0 M *n*-BuLi cyclohexane solution (1.49 mL, 2.97 mmol) were reacted therewith to synthesize diethyl ((3-fluoro-N-(trimethylsilyl)pyridine-2-sulfonimidoyl)methyl)phosphonate as a translucent oil (0.28 g, 54%): R*_{f}* = 0.44 (EtOAc 100%); ¹H NMR (400 MHz, DMSO-*d*₆) *δ* 8.53 (dt, *J* = 1.3, 4.4 Hz, Ar**H**), 7.97-8.02 (m, Ar**H**), 7.75-7.79 (m, Ar**H**), 4.38 (t, *J =* 15.6 Hz, SCHHP), 4.21 (t, *J =* 16.1 Hz, SCH**H**P), 3.92-4.04 (m, P(OC**H₂**CH₃)₂), 1.16 (t, *J =* 7.0 Hz, P(OCH₂C**H₃**)₂), 0.04 (s, Si(OC**H₃**)₃).

### 3.17.5. Synthesis of (E)-(2-chlorostyryl)(3-fluoropyridin-2-yl)(imino)-λ⁶-sulfanone (Chemical Formula 1-40)

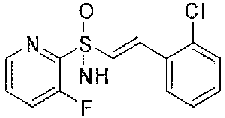

Using reaction (5)-1, diethyl ((3-fluoro-N-(trimethylsilyl)pyridine-2-sulfonimidoyl)methyl)phosphonate (0.28 g, 0.73 mmol) was fully dissolved in THF, and then 2-chlorobenzaldehyde (0.14 mL, 1.10 mmol) and 2.0 M *n*-BuLi cyclohexane solution (0.44 mL, 0.88 mmol) were reacted therewith to synthesize compound 1-40 as a white powder (0.038 g, 18%): R*_{f}* = 0.54 (EtOAc 100%); ¹H NMR (400 MHz, DMSO-*d*₆) *δ* 8.53 (d, *J =* 4.5 Hz, Ar**H**), 7.96-8.02 (m, 2 Ar**H**, (*E*)-isomeric **H**), 7.77-7.80 (m, Ar**H**), 7.65 (d, *J =* 15.3 Hz, (*E*)-isomeric **H**), 7.58-7.60 (m, Ar**H**), 7.49 (td, *J =* 1.4, 7.4 Hz, Ar**H**), 7.42 (t, *J =* 7.2 Hz, Ar**H**), 5.34 (s, N**H**).

### Example 3.18. Synthesis of (E)-(3-chloropyridin-4-yl)(2-chlorostyryl)(imino)-λ⁶-sulfanone (Chemical Formula 1-41)

### 3.18.1. Synthesis of 3-chloro-2-(methylthio)pyridine

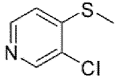

Using reaction (1), 4-bromo-3-chloropyridine (0.80 g, 4.16 mmol) was fully dissolved in DMF, and then sodium thiomethoxide (0.29 g, 4.16 mmol) was reacted therewith to synthesize 3-chloro-2-(methylthio)pyridine as a transparent oil (0.53 g, 79%): R*_{f}* = 0.15 *(n-*hexane 5 : EtOAc 1); ¹H NMR (400 MHz, DMSO-*d*₆) *δ* 8.46 (s, Ar**H**), 8.40 (d, *J =* 5.3 Hz, Ar**H**), 7.34 (d, *J =* 5.3 Hz, Ar**H**), 2.55 (s, C**H₃**).

### 3.18.2. Synthesis of (3-chloropyridin-2-yl)(imino)(methyl)-λ⁶-sulfanone

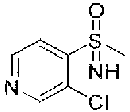

Using reaction (2), 3-chloro-2-(methylthio)pyridine (0.53 g, 3.30 mmol) was fully dissolved in MeOH, and then diacetoxyiodobenzene (2.44 g, 7.58 mmol) and ammonium carbonate (0.48 g, 4.94 mmol) were reacted therewith to synthesize (3-chloropyridin-2-yl)(imino)(methyl)-λ⁶-sulfanone as a white solid (0.47 g, 100%): R*_{f}* = 0.24 (EtOAc 100%); ¹H NMR (400 MHz, DMSO-*d*₆) *δ* 8.86 (s, Ar**H**), 8.81 (d, *J =* 5.0 Hz, Ar**H**), 7.97 (d, *J =* 5.0 Hz, Ar**H**), 4.96 (s, N**H**), 3.27 (s, C**H₃**).

### 3.18.3. Synthesis of (3-chloropyridin-2-yl)(methyl)((trimethylsilyl)imino)-λ⁶-sulfanone

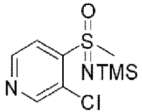

Using reaction (3), hexamethyldisilazane (0.16 mL, 0.79 mmol) was added to (3-chloropyridin-2-yl)(imino)(methyl)-λ⁶-sulfanone (0.10 g, 0.12 mmol) to synthesize (3-chloropyridin-2-yl)(methyl)((trimethylsilyl)imino)-λ⁶-sulfanone as a clear oil (0.15 g, 100%): R*_{f}* = 0.73 (EtOAc 100%); ¹H NMR (400 MHz, DMSO-*d*₆) *δ* 8.88 (s, Ar**H**), 8.82 (d, *J =* 5.0 Hz, Ar**H**), 7.96 (d, *J =* 5.0 Hz, Ar**H**), 3.30 (s, C**H₃**), 0.00 (s, Si(C**H₃**)₃).

### 3.18.4. Synthesis of diethyl ((3-chloro-N-(trimethylsilyl)pyridine-2-sulfonimidoyl)methyl)phosphonate

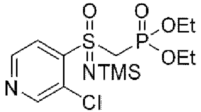

Using reaction (4), (3-chloropyridin-2-yl)(methyl)((trimethylsilyl)imino)-λ⁶-sulfanone (0.50 g, 1.91 mmol) was fully dissolved in THF, and then diethyl chlorophosphate (0.39 mL, 2.67 mmol) and 2.0 M *n*-BuLi cyclohexane solution (2.10 mL, 4.19 mmol) were reacted therewith to synthesize diethyl ((3-chloro-N-(trimethylsilyl)pyridine-2-sulfonimidoyl)methyl)phosphonate as a yellow oil (0.62 g, 81%): R*_{f}* = 0.53 (EtOAc 100%); ¹H NMR (400 MHz, DMSO-*d*₆) *δ* 8.87 (s, Ar**H**), 8.82 (d, *J =* 5.0 Hz, Ar**H**), 7.93 (d, *J =* 4.9 Hz, Ar**H**), 4.27-4.43 (m, SC**H₂**P), 3.95-4.04 (m, P(OC**H₂**CH₃)₂), 1.22 (t, *J* = 7.0 Hz, P(OCH₂C**H₃**)₂), 0.03 (s, Si(C**H₃**)₃).

### 3.18.5. Synthesis of (E)-(3-chloropyridin-4-yl)(2-chlorostyryl)(imino)-λ⁶-sulfanone (Chemical Formula 1-41)

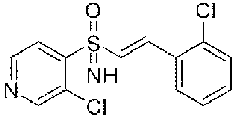

Using reaction (5)-1, the compound of Reaction Scheme 3-4 (0.30 g, 0.74 mmol) was fully dissolved in THF, and then 2-chlorobenzaldehyde (0.11 mL, 1.11 mmol) and 2.0 M *n-*BuLi cyclohexane solution (0.44 mL, 0.89 mmol) were reacted therewith to synthesize compound 1-41 as a white powder (0.025 g, 11%): R*_{f}* = 0.54 (EtOAc 100%); ¹H NMR (400 MHz, DMSO-*d*₆) *δ* 8.82-8.83 (m, 2 Ar**H**), 8.08 (d, *J =* 5.0, Ar**H**), 8.02 (d, *J =* 15.2, (E)-isomeric **H**), 7.94 (d, *J =* 7.7 Hz, Ar**H**), 7.59 (d, *J =* 8.0 Hz, Ar**H**), 7.54 (d, *J =* 15.2 Hz, (E)-isomeric **H**), 7.49 (t, *J =* 7.8 Hz, Ar**H**), 7.41 (t, *J =* 7.6 Hz, Ar**H**), 5.56 (s, N**H**).

### Example 3.19. Synthesis of (E)-(5-chloropyridin-2-yl)(2-chlorostyryl)(imino)-λ⁶-sulfanone (Chemical Formula 1-42)

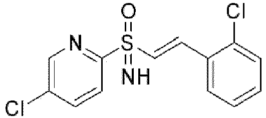

The method used for Examples 3.7.1 to 3.7.4 was used to synthesize diethyl ((5-chloropyridine-2-sulfonimidoyl)methyl)phosphonate .

Using reaction (5)-1, diethyl ((5-chloropyridine-2-sulfonimidoyl)methyl)phosphonate (0.16 g, 0.50 mmol) was fully dissolved in THF, and then 2-chlorobenzaldehyde (0.08 mL, 0.74 mmol) and 2.0 M *n*-BuLi cyclohexane solution (0.30 mL, 0.59 mmol) were reacted therewith to synthesize compound 1-42 as a white powder (0.070 g, 45%): R*_{f}* = 0.69 (EtOAc 100%); ¹H NMR (400 MHz, DMSO-*d*₆) *δ* 8.79 (d, *J* = 2.1 Hz, Ar**H**), 8.24 (dd, *J =* 2.4, 8.4 Hz, Ar**H**), 8.17 (d, *J =* 8.5 Hz, Ar**H**), 7.96 (dd, *J* = 1.4, 7.8 Hz, ArH), 7.91 (d, *J* = 15.3 Hz, (*E*)-isomeric **H**), 7.56-7.62 (m, Ar**H**, (*E*)-isomeric **H**), 7.47 (td, *J=* 1.4, 7.5 Hz, Ar**H**), 7.39 (t, *J =* 7.2 Hz, Ar**H)**, 5.10 (s, N**H**); ¹³C NMR (100 MHz, DMSO-*d*₆) *δ* 159.2, 148.9, 138.8, 137.5, 134.6, 134.3, 132.8, 132.1, 130.7, 130.6, 129.4, 128.3, 123.5.

### Example 3.20. Synthesis of (E)-(2-chlorostyryl)(imino)(3-methoxypyridin-2-yl)-λ⁶-sulfanone (Chemical Formula 1-43)

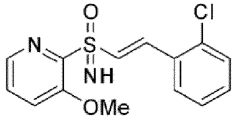

The method used for Examples 3.11.1 to 3.11.4 was used to synthesize diethyl ((3-methoxypyridine-2-sulfonimidoyl)methyl)phosphonate .

Using reaction (5)-1, diethyl ((3-methoxypyridine-2-sulfonimidoyl)methyl)phosphonate (0.19 g, 0.57 mmol) was fully dissolved in THF, and then 2-chlorobenzaldehyde (0.10 mL, 0.86 mmol) and 2.0 M *n*-BuLi cyclohexane solution (0.34 mL, 0.69 mmol) were reacted therewith to synthesize 1-43 as a white powder (0.044 g, 25%): R*_{f}* = 0.35 (DCM 4 : Acetone 1); ¹H NMR (400 MHz, DMSO-*d*₆) *δ* 8.19 (dd, *J =* 1.0, 4.5 Hz, Ar**H**), 8.01 (dd, *J* = 1.6, 7.7 Hz, Ar**H**), 7.93 (d, *J =* 15.2 Hz, (*E*)-isomeric **H**), 7.74-7.76 (m, Ar**H**), 7.64 (dd, *J =* 4.5, 8.4 Hz, Ar**H**), 7.56-7.60 (m, Ar**H**, (*E*)-isomeric **H**), 7.48 (td, *J* = 1.6, 7.4 Hz, Ar**H**), 7.42 (t, *J =* 7.5, Ar**H**), 4.77 (s, N**H**), 3.95 (s, OC**H₃**); ¹³C NMR (100 MHz, DMSO-*d*₆) *δ* 153.0, 148.5, 140.1, 137.7, 134.3, 132.6, 131.4, 131.1, 130.6, 129.4, 128.9, 128.4, 122.7, 56.8.

### Example 3.21. Synthesis of (E)-(2-fluorostyryl)(imino)(3-methoxypyridin-2-yl)-λ⁶-sulfanone (Chemical Formula 1-44)

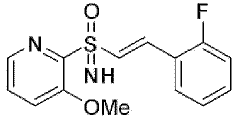

The method used for Examples 3.11.1 to 3.11.4 was used to synthesize diethyl ((3-methoxypyridine-2-sulfonimidoyl)methyl)phosphonate .

Using reaction (5)-1, diethyl ((3-methoxypyridine-2-sulfonimidoyl)methyl)phosphonate (0.20 g, 0.61 mmol) was fully dissolved in THF, and then 2-fluorobenzaldehyde (0.10 mL, 0.92 mmol) and 2.0 M n-BuLi cyclohexane solution (0.37 mL, 0.74 mmol) were reacted therewith to synthesize 1-44 as a white powder (0.18 g, 65%): R*_{f}* = 0.56 (DCM 4 : Acetone 1); ¹H NMR (400 MHz, DMSO-*d*₆) *δ* 8.19 (dd, *J =* 1.1, 4.5 Hz, Ar**H**), 7.93 (td, *J =* 1.5, 7.8 Hz, Ar**H**), 7.75 (dd, *J =* 1.0, 8.5 Hz, Ar**H**), 7.69 (d, *J =* 15.4 Hz, (E)-isomeric **H**), 7.64 (dd, *J* = 4.5, 8.4 Hz, Ar**H**), 7.50-7.55 (m, Ar**H**, (E)-isomeric **H**), 7.28-7.36 (m, 2 Ar**H**), 4.75 (s, N**H**), 3.94 (s, OC**H₃**); ¹³C NMR (100 MHz, DMSO-*d*₆) *δ* 152.9, 148.6, 140.0, 134.7 (d, *J*_{C-F} = 3.3 Hz), 133.3 (d, *J*_{C-F} = 8.8 Hz), 130.9 (d, *J*_{C-F} = 5.7 Hz), 130.3, 128.9, 125.6 (d, *J*_{C-F} = 3.4 Hz), 122.7, 121.1 (d, *J*_{C-F} = 10.9 Hz), 116.8, 116.5, 56.8.

### Example 3.22. Synthesis of (E)-(4-chloropyridin-2-yl)(2-chlorostyryl)(imino)-λ⁶-sulfanone (Chemical Formula 1-45)

### 3.22.1. Synthesis of (4-Chloropyridin-2-yl)(imino)(methyl)-λ⁶-sulfanone

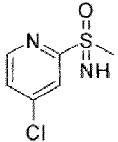

Using reaction (2), 4-Chloro-2-(methylthio)pyridine (0.20 g, 1.25 mmol) was fully dissolved in MeOH, and then diacetoxyiodobenzene (0.93 g, 2.88 mmol) and ammonium carbonate (0.18 g, 1.88 mmol) were reacted therewith to synthesize (4-chloropyridin-2-yl)(imino)(methyl)-λ⁶-sulfanone as a white solid (0.18 g, 77%): R*_{f}* = 0.58 (EtOAc 10 : MeOH 1); ¹H NMR (400 MHz, DMSO-*d*₆) *δ* 8.59 (dd, *J =* 1.0, 4.5 Hz, Ar**H**), 8.18 (dd, *J* = 1.1, 8.1 Hz, Ar**H**), 7.65-7.68 (m, Ar**H**), 4.62 (s, N**H**), 3.35 (s, C**H₃**).

### 3.22.2. Synthesis of (4-Chloropyridin-2-yl)(methyl)((trimethylsilyl)imino)-λ⁶-sulfanone

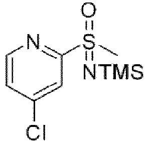

Using reaction (3), hexamethyldisilazane (0.30 mL, 1.44 mmol) was added to (4-Chloropyridin-2-yl)(imino)(methyl)-λ⁶-sulfanone (0.18 g, 0.96 mmol) to synthesize (4-Chloropyridin-2-yl)(methyl)((trimethylsilyl)imino)-λ⁶-sulfanone as a transparent oil (0.29 g, *100%):* R*_{f}* = 0.75 (EtOAc 100%); ¹H NMR (400 MHz, DMSO-*d*₆) *δ* 8.73 (d, *J =* 5.2 Hz, Ar**H**), 8.02 (d, *J* = 2.0 Hz, Ar**H**), 7.84 (dd, *J* = 2.0, 5.2 Hz, Ar**H**), 3.19 (s, C**H₃**), 0.01 (s, Si(OC**H₃**)₃).

### 3.22.3. Synthesis of diethyl ((4-chloro-N-(trimethylsilyl)pyridine-2-sulfonimidoyl)methyl)phosphonate

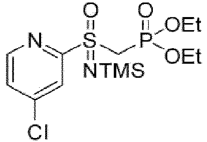

Using reaction (4), (4-Chloropyridin-2-yl)(methyl)((trimethylsilyl)imino)-λ⁶-sulfanone (0.29 g, 1.10 mmol) was fully dissolved in THF, and then diethyl chlorophosphate (0.22 mL, 1.53 mmol) and 2.0 M *n*-BuLi cyclohexane solution (1.21 mL, 2.41 mmol) were reacted therewith to synthesize diethyl ((4-chloro-N-(trimethylsilyl)pyridine-2-sulfonimidoyl)methyl)phosphonate as a yellow oil (0.28 g, 65%): R*_{f}* = 0.52 (EtOAc 100%); ¹H NMR (400 MHz, DMSO-*d*₆) *δ* 8.73 (d, *J =* 5.2 Hz, Ar**H**), 8.01 (d, *J =* 1.6 Hz, Ar**H**), 7.84 (dd, *J =* 2.0, 5.2 Hz, Ar**H**), 4.27 (d, *J* = 16.0 Hz, SC**H₂**P), 3.93-4.01 (m, P(OC**H₂**CH₃)₂), 1.12-1.17 (m, P(OCH₂C**H₃**)₂), 0.04 (s, Si(C**H₃**)₃).

### 3.22.4. Synthesis of (E)-(4-chloropyridin-2-yl)(2-chlorostyryl)(imino)-λ⁶-sulfanone (Chemical Formula 1-45)

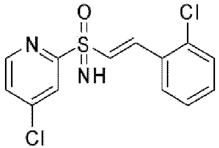

Using reaction (5)-1, diethyl ((4-chloro-N-(trimethylsilyl)pyridine-2-sulfonimidoyl)methyl)phosphonate (0.28 g, 0.71 mmol) was fully dissolved in THF, and then 2-chlorobenzaldehyde (0.12 mL, 1.07 mmol) and 2.0 M *n*-BuLi cyclohexane solution (0.43 mL, 0.85 mmol) were reacted therewith to synthesize compound 1-45 as a white solid (0.15 g, 53%): R*_{f}* = 0.26 (*n*-hexane 1 : EtOAc 1); ¹H NMR (400 MHz, DMSO-*d*₆) *δ* 8.70 (d, *J* = 5.2 Hz, Ar**H**), 8.21 (d, *J =* 1.9 Hz, Ar**H**), 7.91-7.97 (m, Ar**H**, (*E*)-isomeric **H**), 7.82 (dd, *J* = 1.9, 5.2 Hz, Ar**H**), 7.56-7.64 (m, Ar**H**, (*E*)-isomeric **H**), 7.47 (td, *J =* 1.4, 7.4 Hz, Ar**H**), 7.40 (t, *J* = 7.4 Hz, Ar**H**), 5.17 (s, N**H**); ¹³C NMR (100 MHz, DMSO-*d*₆) *δ* 162.5, 151.8, 145.4, 137.9, 134.4, 132.9, 131.6, 130.7, 130.6, 129.4, 128.3, 127.3, 121.9.

### Example 3.23. Synthesis of (E)-(2-chlorostyryl)(3-fluoropyridin-4-yl)((trimethylsilyl)imino)-λ⁶-sulfanone (Chemical Formula 1-46)

### 3.23.1. 3-fluoro-4-(methylthio)pyridine

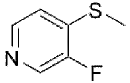

Using reaction (1), 4-bromo-3-fluoropyridine (1.00 g, 7.60 mmol) was fully dissolved in DMF, and then sodium thiomethoxide (0.53 g, 7.60 mmol) was reacted therewith to synthesize 3-fluoro-2-(methylthio)pyridine as a transparent oil (0.72 g, 59%): R*_{f}* = 0.60 (*n-*hexane 1 : EtOAc 1); ¹H NMR (400 MHz, DMSO-*d*₆) *δ* 8.39 (d, *J=* 2.1 Hz, ArH), 8.33 (dd, *J* = 1.0, 5.2 Hz, ArH), 7.39 (dd, *J =* 5.2, 6.8 Hz, ArH), 2.55 (s, CH₃).

### 3.23.2. Synthesis of (3-fluoropyridin-4-yl)(imino)(methyl)-λ⁶-sulfanone

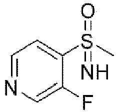

Using reaction (2), 3-fluoro-4-(methylthio)pyridine (0.64 g, 4.43 mmol) was fully dissolved in MeOH, and then diacetoxyiodobenzene (3.29 g, 10.20 mmol) and ammonium carbonate (0.48 g, 4.94 mmol) were reacted therewith to synthesize (3-fluoropyridin-2-yl)(imino)(methyl)-λ⁶-sulfanone as a pale yellow oil (0.47 g, 74%): R*_{f}* = 0.24 (EtOAc 100%); ¹H NMR (400 MHz, DMSO-*d*₆) *δ* 8.83 (d, *J* = 1.7 Hz, ArH), 8.70 (d, *J* = 4.8 Hz, ArH), 7.82 (dd, *J=* 5.0, 5.9 Hz, ArH), 5.11 (s, NH), 3.26 (s, CH₃).

### 3.23.3. Synthesis of (3-fluoropyridin-4-yl)(methyl)((trimethylsilyl)imino)-λ⁶-sulfanone

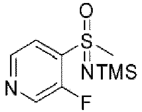

Using reaction (3), hexamethyldisilazane (1.00 mL, 4.77 mmol) was added to (3-fluoropyridin-2-yl)(imino)(methyl)-λ⁶-sulfanone (0.55 g, 3.18 mmol) to synthesize (3-fluoropyridin-2-yl)(methyl)((trimethylsilyl)imino)-λ⁶-sulfanone as a yellow oil (0.76 g, 96%): R*_{f}* = 0.76 (EtOAc 100%); ¹H NMR (400 MHz, DMSO-*d*₆) *δ* 8.85-8.86 (m, Ar**H**), 8.70-8.72 (m, ArH), 7.78-7.82 (m, Ar**H**), 3.28 (s, CH₃), 0.01 (s, Si(CH₃)₃).

### 3.23.4. Synthesis of diethyl ((3-fluoro-N-(trimethylsilyl)pyridine-4-sulfonimidoyl)methyl)phosphonate

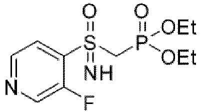

Using reaction (4), (3-fluoropyridin-2-yl)(methyl)((trimethylsilyl)imino)-λ⁶-sulfanone (0.76 g, 3.06 mmol) was fully dissolved in THF, and then diethyl chlorophosphate (0.62 mL, 4.29 mmol) and 2.0 M n-BuLi cyclohexane solution (3.37 mL, 6.74 mmol) were reacted therewith to synthesize diethyl ((3-fluoro-N-(trimethylsilyl)pyridine-4-sulfonimidoyl)methyl)phosphonate as a transparent oil (0.86 g, 73%): R*_{f}* = 0.52 (EtOAc 100%); ¹H NMR (400 MHz, DMSO-*d*₆) *δ* 8.83 (d, *J =* 1.7 Hz, ArH), 8.70 (d, *J* = 4.9 Hz, ArH), 7.83 (dd, *J* = 5.1, 6.0 Hz, ArH), 5.41 (s, NH), 4.30 (dd, *J* = 1.6, 16.5 Hz, SCH₂P), 3.95-4.04 (m, P(OCH₂CH₃)₂), 1.12-1.17 (m, P(OCH₂CH₃)₂).

### 3.23.5. Synthesis of (E)-(2-chlorostyryl)(3-fluoropyridin-4-yl)((trimethylsilyl)imino)-λ⁶-sulfanone (Chemical Formula 1-46)

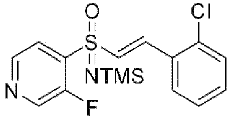

Using reaction (5)-1, the compound of Reaction Scheme 3-4 (0.42 g, 1.09 mmol) was fully dissolved in THF, and then 2-chlorobenzaldehyde (0.18 mL, 1.63 mmol) and 2.0 M n-BuLi cyclohexane solution (0.65 mL, 1.31 mmol) were reacted therewith to synthesize the compound 1-46 as a white powder (0.18 g, 45%): R*_{f}* = 0.80 (n-hexane 1 : EtOAc 1); ¹H NMR (DMSO-*d*₆, 400 MHz) *δ* 8.81 (s, ArH), 8.72 (d, *J* = 4.6 Hz, ArH), 7.94-7.96 (m, ArH, (E)-isomeric H), 7.88 (t, *J=* 5.2 Hz, ArH), 7.67 (d, *J=* 15.1 Hz, (E)-isomeric H), 7.59 (d, *J=* 8.0 Hz, ArH), 7.49 (t, *J=* 7.5 Hz, ArH), 7.41 (t, *J* = 7.6 Hz, ArH), 0.11 (s, Si(CH₃)₃); ¹³C NMR (100 MHz, DMSO-*d*₆) *δ* 152.8, 151.0, 145.01 (d, *J*_{C-F} = 5.8 Hz), 137.9 (d*, J_{C-F}* = 24.2 Hz), 136.7 (d, *J*_{C-F} = 11.8 Hz), 135.2, 131.9, 130.9, 130.4, 127.8 (d, *J*_{C-F} = 26.0 Hz), 126.9, 125.6, 119.5.

### Example 3.24. Synthesis of (E)-(2-chlorostyryl)(3-fluoropyridin-4-yl)(imino)-λ⁶-sulfanone (Chemical Formula 1-47)

### 3.24.1. Synthesis of 3-chloro-4-(methylthio)pyridine

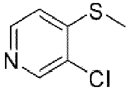

Using reaction (1), 4-bromo-3-chloropyridine (0.80 g, 4.16 mmol) was fully dissolved in DMF, and then sodium thiomethoxide (0.29 g, 4.16 mmol) was reacted therewith to synthesize 3-chloro-4-(methylthio)pyridine as a transparent oil (0.53 g, 79%): R*_{f}* = 0.15 (*n-*hexane 5 : EtOAc 1); ¹H NMR (400 MHz, DMSO-*d*₆) *δ* 8.46 (s, Ar**H**), 8.40 (d, *J =* 5.3 Hz, Ar**H**), 7.34 (d, *J =* 5.3 Hz, Ar**H**), 2.55 (s, C**H₃**).

### 3.24.2. Synthesis of (3-chloropyridin-4-yl)(imino)(methyl)-λ⁶-sulfanone

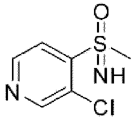

Using reaction (2), 3-chloro-4-(methylthio)pyridine (0.53 g, 3.30 mmol) was fully dissolved in MeOH, and then diacetoxyiodobenzene (2.44 g, 7.58 mmol) and ammonium carbonate (0.48 g, 4.94 mmol) were reacted therewith to synthesize (3-chloropyridin-2-yl)(imino)(methyl)-λ⁶-sulfanone as a pale yellow oil (0.47 g, 74%): R*_{f}* = 0.24 (EtOAc 100%); ¹H NMR (400 MHz, DMSO-*d*₆) *δ* 8.86 (s, Ar**H**), 8.81 (d, *J =* 5.0 Hz, Ar**H**), 7.97 (d, *J* = 5.0 Hz, Ar**H**), 4.96 (s, N**H**), 3.27 (s, C**H**₃).

### 3.24.3. Synthesis of (3-chloropyridin-4-yl)(methyl)((trimethylsilyl)imino)-λ⁶-sulfanone

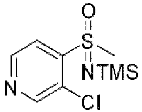

Using reaction (3), hexamethyldisilazane (0.16 mL, 0.79 mmol) was added to (3-chloropyridin-2-yl)(imino)(methyl)-λ⁶-sulfanone (0.10 g, 0.12 mmol) to synthesize (3-chloropyridin-2-yl)(methyl)((trimethylsilyl)imino)-λ⁶-sulfanone as a transparent oil (0.15 g, *100%):* R*_{f}* = 0.73 (EtOAc 100%); ¹H NMR (400 MHz, DMSO-*d*₆) *δ* 8.88 (s, Ar**H**), 8.82 (d, *J =* 5.0 Hz, Ar**H**), 7.96 (d, *J =* 5.0 Hz, Ar**H**), 3.30 (s, C**H**₃), 0.00 (s, Si(C**H**₃)₃).

### 3.24.4. Synthesis of diethyl ((3-chloro-N-(trimethylsilyl)pyridine-4-sulfonimidoyl)methyl)phosphonate

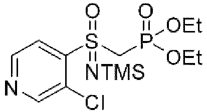

Using reaction (4), (3-chloropyridin-2-yl)(methyl)((trimethylsilyl)imino)-λ⁶-sulfanone (0.50 g, 1.91 mmol) was fully dissolved in THF, and then diethyl chlorophosphate (0.39 mL, 2.67 mmol) and 2.0 M *n*-BuLi cyclohexane solution (2.10 mL, 4.19 mmol) were reacted therewith to synthesize diethyl ((3-chloro-N-(trimethylsilyl)pyridine-4-sulfonimidoyl)methyl)phosphonate as a pale yellow oil (0.62 g, 81%): R*_{f}* = 0.53 (EtOAc 100%); ¹H NMR (400 MHz, DMSO-*d*₆) *δ* 8.87 (s, Ar**H**), 8.82 (d, *J* = 5.0 Hz, ArH), 7.93 (d, *J* = 4.9 Hz, Ar**H**), 4.27-4.43 (m, SC**H₂**P), 3.95-4.04 (m, P(OCH₂C**H**₃)₂), 1.22 (t, *J =* 7.0 Hz, P(OCH₂C**H**₃)₂), 0.03 (s, Si(CH₃)₃).

### 3.24.5. Synthesis of (E)-(2-chlorostyryl)(3-fluoropyridin-4-yl)(imino)-λ⁶-sulfanone (Chemical Formula 1-47)

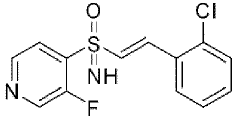

Using reaction (5)-1, the compound of Reaction Scheme 3-4 (0.42 g, 1.09 mmol) was fully dissolved in THF, and then 2-chlorobenzaldehyde (0.18 mL, 1.63 mmol) and 2.0 M *n-*BuLi cyclohexane solution (0.65 mL, 1.31 mmol) were reacted therewith to synthesize the compound 1-47 as a white powder (0.23 g, 58%): R*_{f}* = 0.29 (*n*-hexane 1 : EtOAc 1); ¹H NMR (DMSO-*d*₆, 400 MHz) *δ* 8.79 (s, Ar**H**), 8.71 (d, *J =* 3.2 Hz, Ar**H**), 7.96-8.02 (m, Ar**H**, (*E*)-isomeric **H**), 7.91 (t, *J =* 3.6 Hz, Ar**H**), 7.58-7.62 (m, Ar**H**, (*E*)-isomeric **H**), 7.49 (t, *J =* 4.9 Hz, Ar**H**), 7.41 (t, *J=* 5.0 Hz, Ar**H**), 5.69 (s, N**H**); ¹³C NMR (100 MHz, DMSO-*d*₆) *δ* 155.4, 153.6, 147.4 (d, *J*_{C-F} = 5.9 Hz), 140.42 (d, *J*_{C-F} = 24.3 Hz), 139.1 (d, *J*_{C-F} = 12.5 Hz), 138.9, 134.5, 133.1, 131.5, 130.4 (d, *J*_{C-F} = 16.1 Hz), 129.5, 128.2, 122.6 (d, *J*_{C-F} = 1.7 Hz).

### Example 3.25. Synthesis of (E)-(4-chloropyridin-3-yl)(2-chlorostyryl)(imino)-λ⁶-sulfanone (Chemical Formula 1-48)

### 3.25.1. Synthesis of 4-chloro-3-(methylthio)pyridine

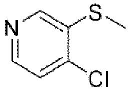

Using reaction (1), 3-bromo-4-chloropyridine (1.00 g, 7.60 mmol) was fully dissolved in DMF, and then sodium thiomethoxide (0.53 g, 7.60 mmol) was reacted therewith to synthesize 4-chloro-3-(methylthio)pyridine as a transparent oil (0.36 g, 30%): R*_{f}* = 0.60 (n-hexane 1 : EtOAc 1); ¹H NMR (400 MHz, DMSO-*d*₆) *δ* 8.48 (s, Ar**H**), 8.34 (d, *J =* 5.2 Hz, Ar**H**), 7.53 (d, *J = 5.2* Hz, Ar**H**), 2.61 (s, CH₃).

### 3.25.2. Synthesis of (4-chloropyridin-3-yl)(imino)(methyl)-λ⁶-sulfanone

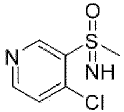

Using reaction (2), 4-chloro-3-(methylthio)pyridine (0.36 g, 2.28 mmol) was fully dissolved in MeOH, and then diacetoxyiodobenzene (1.69 g, 5.24 mmol) and ammonium carbonate (0.33 g, 3.42 mmol) were reacted therewith to synthesize (4-chloropyridin-3-yl)(imino)(methyl)-λ⁶-sulfanone as a yellow oil (0.20 g, 46%): R*_{f}* = 0.43 (EtOAc 10 : MeOH 1); ¹H NMR (400 MHz, DMSO-*d*₆) *δ* 9.09 (s, Ar**H**), 8.76 (d, *J* = 5.3 Hz, Ar**H**), 7.78 (d, *J =* 5.2 Hz, Ar**H**), 4.89 (s, N**H**), 3.26 (s, C**H**₃).

### 3.25.3. Synthesis of (4-chloropyridin-3-yl)(methyl)((trimethylsilyl)imino)-λ⁶-sulfanone

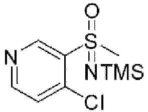

Using reaction (3), hexamethyldisilazane (0.33 mL, 1.59 mmol) was added to (4-chloropyridin-3-yl)(imino)(methyl)-λ⁶-sulfanone (0.20 g, 1.06 mmol) to synthesize (4-chloropyridin-3-yl)(methyl)((trimethylsilyl)imino)-λ⁶-sulfanone as a yellow oil (0.29 g, 100%): R*_{f}* = 0.87 (EtOAc 100%); ¹H NMR (400 MHz, DMSO-*d*₆) *δ* 9.07 (s, ArH), 8.77 (d, *J=* 5.3 Hz, Ar**H**), 7.80 (d, *J =* 5.3 Hz, Ar**H**), 3.30 (s, C**H**₃), 0.00 (s, Si(CH₃)₃).

### 3.25.4. Synthesis of diethyl ((4-chloro-N-(trimethylsilyl)pyridine-3-sulfonimidoyl)methyl)phosphonate

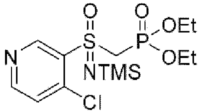

Using reaction (4), (4-chloropyridin-3-yl)(methyl)((trimethylsilyl)imino)-λ⁶-sulfanone (0.50 g, 1.91 mmol) was fully dissolved in THF, and then diethyl chlorophosphate (0.39 mL, 2.67 mmol) and 2.0 M *n*-BuLi cyclohexane solution (2.10 mL, 4.19 mmol) were reacted therewith to synthesize diethyl ((3-chloro-N-(trimethylsilyl)pyridine-4-sulfonimidoyl)methyl)phosphonate as a pale yellow oil (0.62 g, 81%): R*_{f}* = 0.53 (EtOAc 100%); ¹H NMR (400 MHz, DMSO-*d*₆) *δ* 9.02 (s, Ar**H**), 8.76 (d, *J =* 5.3 Hz, Ar**H**), 7.79 (d, *J* = 5.3 Hz, Ar**H**), 4.24-4.39 (m, SC**H₂**P), 3.92-4.01 (m, P(OC**H₂**CH₃)₂), 1.13-1.17 (m, P(OCH₂C**H₃**)₂), 0.03 (Si(C**H₃**)₃).

### 3.25.5. Synthesis of (E)-(4-chloropyridin-3-yl)(2-chlorostyryl)(imino)-λ⁶-sulfanone (Chemical Formula 1-48)

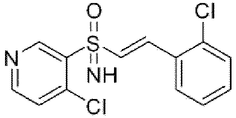

Using reaction (5)-1, the compound of Reaction Scheme 4-4 (0.21 g, 0.52 mmol) was fully dissolved in THF, and then 2-chlorobenzaldehyde (0.09 mL, 0.77 mmol) and 2.0 M *n-*BuLi cyclohexane solution (0.31 mL, 0.62 mmol) were reacted therewith to synthesize compound 1-48 as a white solid (0.019 g, 12%): R*_{f}* = 0.59 (EtOAc 100%); ¹H NMR (400 MHz, DMSO-*d*₆) *δ* 9.21 (s, Ar**H**), 8.76 (d, *J =* 5.2 Hz, Ar**H**), 8.01 (d, *J =* 15.2 Hz, (E)-isomeric **H**), 7.92 (dd, *J* = 1.6, 7.8 Hz, Ar**H**), 7.75 (d, *J =* 5.2 Hz, Ar**H**), 7.58 (dd, *J* = 1.1, 8.0 Hz, Ar**H**), 7.54 (d, *J =* 15.2 Hz, (E)-isomeric **H**), 7.48 (td, *J =* 1.5, 7.4 Hz, Ar**H**), 7.38-7.42 (m, Ar**H**), 5.48 (s, N**H**); ¹³C NMR (DMSO-*d*₆, 100 MHz) *δ* 154.9, 151.2, 142.4, 138.9, 136.9, 134.5, 132.9, 131.2, 130.7, 130.6, 129.5, 128.4, 127.0.

### Example 3.26. Synthesis of (E)-imino(5-methoxypyridin-2-yl)(2-(trifluoromethyl)styryl)-λ⁶-sulfanone (Chemical Formula 1-54)

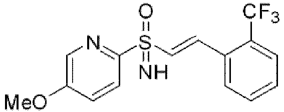

The method used for Examples 3.12.1 to 3.12.4 was used to synthesize diethyl ((N-(trimethylsilyl)pyridine-2-sulfonimidoyl)methyl)phosphonate .

Using reaction (5)-1, diethyl ((N-(trimethylsilyl)pyridine-2-sulfonimidoyl)methyl)phosphonate (0.35 g, 0.90 mmol) was fully dissolved in THF, and then 2-(trifluoromethyl)benzaldehyde (0.14 mL, 1.08 mmol) and 2.0 M *n*-BuLi cyclohexane solution (0.54 mL, 1.08 mmol) were reacted therewith to synthesize compound 1-54 as a white powder (0.13 g, 41%): R*_{f}* = 0.45 (EtOAc 100%); ¹H NMR (400 MHz, DMSO-*d*₆) *δ* 8.40 (d, *J* = 2.9 Hz, Ar**H**), 8.11 (d, *J=* 8.8 Hz, Ar**H**), 8.03 (d, *J =* 7.8 Hz, Ar**H**), 7.82 (d, *J =* 8.0 Hz, Ar**H**), 7.69-7.77 (m, 2 Ar**H**), 7.60-7.65 (m, Ar**H**, (*E*)-isomeric **H**), 7.58 (d, *J=* 15.0 Hz, (*E*)-isomeric **H**), 4.83 (s, N**H**), 3.91 (s, OC**H₃**); ¹³C NMR (100 MHz, DMSO-*d*₆) *δ* 157.9, 151.9, 138.5, 135.6, 134.7, 133.6, 131.1, 129.4, 127.6 (q, *J*_{C-F} = 29.5 Hz), 126.7 (q, *J*_{C-F} = 5.5 Hz), 124.5 (q, *J*_{C-F} = 272.1 Hz), 123.1, 121.9, 56.7.

### Example 3.27. Synthesis of (E)-(2-fluorostyryl)(imino)(5-methoxypyridin-2-yl)-λ⁶-sulfanone (Chemical Formula 1-55)

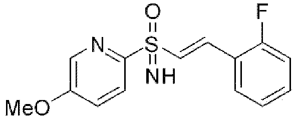

The method used for Examples 3.12.1 to 3.12.4 was used to synthesize diethyl ((N-(trimethylsilyl)pyridine-2-sulfonimidoyl)methyl)phosphonate .

Using reaction (5)-1, diethyl ((N-(trimethylsilyl)pyridine-2-sulfonimidoyl)methyl)phosphonate (0.35 g, 1.08 mmol) was fully dissolved in THF, and then 2-fluorobenzaldehyde (0.14 mL, 1.29 mmol) and 2.0 M *n*-BuLi cyclohexane solution (0.65 mL, 1.29 mmol) were reacted therewith to synthesize compound 1-55 as a white solid (0.24 g, 76%): R*_{f}* = 0.78 (EtOAc 10 : MeOH 1); ¹H NMR (400 MHz, DMSO-*d*₆) *δ* 8.39 (d, *J* = 2.8 Hz, Ar**H**), 8.11 (d, *J* = 8.8 Hz, Ar**H**), 7.87 (t, *J* = 7.3 Hz, Ar**H**), 7.61-7.64 (m, Ar**H**, (*E*)-isomeric **H**), 7.46-7.50 (m, Ar**H**, (*E*)-isomeric **H**), 7.31 (t, *J* = 10.7 Hz, Ar**H**), 7.26 (t, *J* = 7.6 Hz, Ar**H**), 4.78 (s, N**H**), 3.91 (s, OC**H**₃).

### Example 3.28. Synthesis of (E)-(2-fluorostyryl)(5-methoxypyridin-2-yl)(methylimino)-λ6-sulfanone (Chemical Formula 1-56)

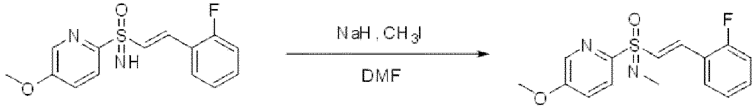

The method used for Examples 3.27 was used to synthesize (E)-(2-fluorostyryl)(imino)(5-methoxypyridin-2-yl)-λ⁶-sulfanone .

Sodium hydride (1.2 eq, 60% dispersion in mineral oil) was dissolved in anhydrous DMF, and then the solution was cooled to 0°C using ice water. To this solution, the synthesized (E)-(2-fluorostyryl)(imino)(5-methoxypyridin-2-yl)-λ⁶-sulfanone (1.0 eq) was slowly added dropwise, and the mixture was stirred for 30 minutes. Then, iodomethane (1.1 eq) was added thereto and the mixture was reacted at room temperature for 15 hours. The reaction was quenched with water. Then, the mixture was extracted with water and EtOAc. The organic layer was dried over anhydrous Na₂SO₄ to remove a small amount of water. The solvent was then distilled off under reduced pressure, and the mixture was dried under vacuum. Thereafter, the residue was separated and purified by column chromatography to synthesize compound 1-56 as a white solid (0.02 g, 65%): R*_{f}* = 0.70 (EtOAc 10 : MeOH 1); ¹H NMR (400 MHz, DMSO-*d*₆) *δ* 8.44 (d, *J =* 2.9 Hz, Ar**H**), 8.09 (d, *J =* 8.7 Hz, Ar**H**), 7.90 (td, *J =* 1.5, 7.9 Hz, Ar**H**), 7.63 (dd, *J =* 2.9, 8.8 Hz, Ar**H**), 7.59 (d, *J* = 15.6 Hz, (*E*)-isomeric **H**), 7.48-7.54 (m, Ar**H**), 7.39 (d, *J* = 15.6 Hz, (E)-isomeric **H**), 7.31-7.34 (m, Ar**H**), 7.27 (t, *J=* 7.6 Hz, Ar**H**), 3.91 (s, OC**H₃**), 2.62 (s, NC**H₃**).

### Example 4. Synthesis of sulfanone derivative in which both A and B are pyridine

In an example of the present disclosure, the synthesis process of a sulfanone derivative, in which both A and B of Chemical Formula 1 are pyridine, is shown by Reaction Scheme 4 presented below.

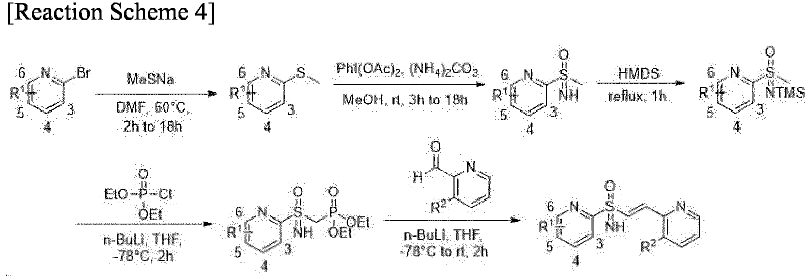

### (5)-2. Synthesis of substituted (E)-imino(pyridin-2-yl)(2-(pyridin-2-yl)vinyl)-λ⁶-sulfanone derivative by reaction with picolinaldehyde derivative

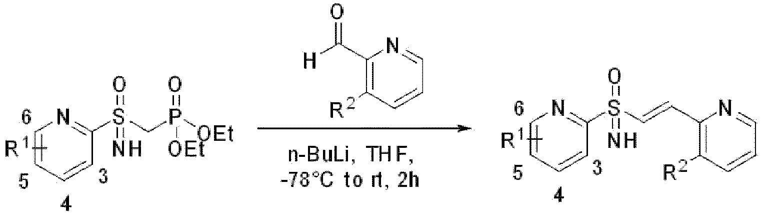

Using reactions (1) to (4) in the way they were used for Example 3, a substituted (E)-imino(pyridine-2-yl)(2-(pyridin-2-yl)vinyl)-λ⁶-sulfanone derivative was synthesized from substituted methyl(pyridin-2-yl)((trimethylsilyl)imino)-λ⁶-sulfanone according to the above shown Reaction Scheme.

The compound (1.0 eq) synthesized in step 2-4 was dissolved in anhydrous THF, and then the solution was cooled to -78°C using dry ice and acetone. n-BuLi (1.2 eq, 2.0 M cyclohexane solution) was slowly added dropwise to this solution, and the mixture was stirred for 1 hour. Then, picolinaldehyde derivatives (1.2 eq) were added thereto and the mixture was reacted for another 1 hour. If the reaction was not completed as confirmed by TLC, the reaction was allowed to proceed for another 30 minutes at room temperature. The reaction was quenched with a small amount of water. Then, the mixture was extracted with water and 10% MeOH/MC. The organic layer was dried over Na₂SO₄ to remove a small amount of water. The solvent was distilled off under reduced pressure and the mixture was dried under vacuum. Subsequently, the residues were separated and purified by column chromatography to obtain 3-, 4-, 5- or 6-substituted (E)-imino(pyridin-2-yl)(2-(pyridin-2-yl)vinyl)-λ⁶-sulfanone derivative.

### Example 4.1. Synthesis of (E)-imino(4-methoxypyridin-2-yl)(2-(3-(trifluoromethyl)pyridin-2-yl)vinyl)-λ⁶-sulfanone (Chemical Formula 1-27)

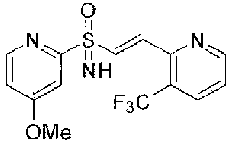

The method used for Examples 3.12.1 to 3.12.4 was used to synthesize diethyl ((4-methoxy-N-(trimethylsilyl)pyridine-2-sulfonimidoyl)methyl)phosphonate .

Using Reaction Scheme (5)-2, diethyl ((4-methoxy-N-(trimethylsilyl)pyridine-2-sulfonimidoyl)methyl)phosphonate (0.14 g, 0.34 mmol) was fully dissolved in THF, and then 3-(trifluoromethyl)picolinaldehyde (0.050 mL, 0.41 mmol) and 2.0 M *n*-BuLi cyclohexane solution (0.21 mL, 0.41 mmol) were reacted therewith to synthesize compound 1-27 as a white powder (0.063 g, 54%): R*_{f}* = 0.35 (EtOAc 100%); ¹H NMR (400 MHz, DMSO-*d*₆) *δ* 8.91 (d, *J* = 4.4 Hz, ArH), 8.53 (d, *J* = 5.6 Hz, ArH), 8.28-8.30 (m, ArH), 7.87 (d, *J* = 14.5 Hz, (E)-isomeric **H**), 7.73-7.77 (m, (*E*)-isomeric **H**), 7.68-7.71 (m, 2 ArH), 7.22 (dd, *J* = 2.5, 5.6 Hz, Ar**H**), 5.17 (s, N**H**), 3.94 (s, OC**H**₃).

### Example 4.2. Synthesis of (E)-(2-(3-chloropyridin-2-yl)vinyl)(imino)(4-methoxypyridin-2-yl)-λ⁶-sulfanone (Chemical Formula 1-28)

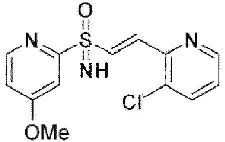

The method used for Examples 3.12.1 to 3.12.4 was used to synthesize diethyl ((4-methoxy-N-(trimethylsilyl)pyridine-2-sulfonimidoyl)methyl)phosphonate .

Using Reaction Scheme (5)-2, diethyl ((4-methoxy-N-(trimethylsilyl)pyridine-2-sulfonimidoyl)methyl)phosphonate (0.26 g, 0.65 mmol) was fully dissolved in THF, and then 3-chloropicolinaldehyde (0.10 mL, 0.78 mmol) and 2.0 M *n*-BuLi cyclohexane solution (0.39 mL, 0.78 mmol) were reacted therewith to synthesize **1-28** as a white powder (0.085 g, 42%): R*_{f}* = 0.33 (EtOAc 100%); ¹H NMR (400 MHz, DMSO-*d*₆) *δ* 8.60-8.61 (m, Ar**H**), 8.52 (d, *J* = 5.6 Hz, Ar**H**), 8.05 (dd, *J* = 1.2, 8.2 Hz, Ar**H**), 7.91 (d, *J* = 14.7 Hz, (E)-isomeric **H**), 7.72 (d, *J* = 14.7 Hz, (*E*)-isomeric **H**), 7.68 (d, *J =* 2.4 Hz, Ar**H**), 7.51 (dd, *J =* 4.5, 8.2 Hz, Ar**H**), 7.21 (dd, *J =* 2.4, 5.6 Hz, Ar**H**), 5.11 (s, N**H**), 3.94 (s, OC**H**₃); ¹³C NMR (100 MHz, DMSO-*d*₆) *δ* 167.3, 162.3, 151.7, 149.1, 147.9, 138.9, 135.8, 134.9, 132.2, 127.2, 113.3, 107.8, 56.6.

### Example 4.3. Synthesis of (E)-(2-(3-fluoropyridin-2-yl)vinyl)(imino)(4-methoxypyridin-2-yl)-λ⁶-sulfanone (Chemical Formula 1-29)

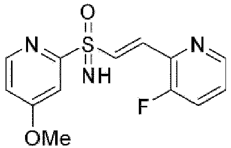

The method used for Examples 3.12.1 to 3.12.4 was used to synthesize diethyl ((4-methoxy-N-(trimethylsilyl)pyridine-2-sulfonimidoyl)methyl)phosphonate .

Using reaction scheme (5)-2, diethyl ((4-methoxy-N-(trimethylsilyl)pyridine-2-sulfonimidoyl)methyl)phosphonate (0.28 g, 0.72 mmol) was fully dissolved in THF, and then 3-fluoropicolinaldehyde (0.11 g, 0.86 mmol) and 2.0 M n-BuLi cyclohexane solution (0.43 mL, 0.86 mmol) were reacted therewith to synthesize **1-29** as a white powder (0.10 g, 49%): R*_{f}* = 0.40 (EtOAc 100%); ¹H NMR (400 MHz, DMSO-*d*₆) *δ* 8.52 (d, *J=* 5.6 Hz, 2 ArH), 7.84-7.89 (m, ArH), 7.64-7.73 (m, ArH, (*E*)-isomeric 2H), 7.56-7.60 (m, ArH), 7.20 (dd, *J=* 2.5, 5.6 Hz, ArH), 5.09 (s, NH), 3.93 (s, OCH₃); ¹³C NMR (100 MHz, DMSO-*d*₆) *δ* 167.3, 162.3, 159.6, 157.0, 151.7, 146.9 (d, *J*_{C-F} = 4.8 Hz), 139.5 (d, *J*_{C-F} = 10.9 Hz), 132.7, 127.9 (d, *J*_{C-F} = 4.7 Hz), 125.2 (d, *J*_{C-F} = 18.9 Hz), 113.3, 107.8, 56.6.

### Example 4.4. Synthesis of (E)-imino(4-methoxypyridin-2-yl)(2-(3-methoxypyridin-2-yl)vinyl)-λ⁶-sulfanone (Chemical Formula 1-32)

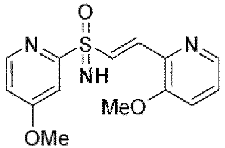

The method used for Examples 3.12.1 to 3.12.4 was used to synthesize diethyl ((4-methoxy-N-(trimethylsilyl)pyridine-2-sulfonimidoyl)methyl)phosphonate .

Using Reaction Scheme (5)-2, diethyl ((4-methoxy-N-(trimethylsilyl)pyridine-2-sulfonimidoyl)methyl)phosphonate (0.22 g, 0.68 mmol) was fully dissolved in THF, and then 3-methoxypicolinaldehyde (0.11 g, 0.82 mmol) and 2.0 M *n*-BuLi cyclohexane solution (0.41 mL, 0.82 mmol) were reacted therewith to synthesize compound 1-32 as a white solid (0.075 g, 36%): R*_{f}* = 0.27 (EtOAc 100%); ¹H NMR (400 MHz, DMSO-*d*₆) *δ* 8.50 (d, *J =* 5.6 Hz, Ar**H**), 8.22 (dd, *J =* 1.0, 4.5 Hz, Ar**H**), 7.84 (d, *J =* 15.0 Hz, (*E*)-isomeric **H**), 7.66 (d, *J =* 2.4 Hz, Ar**H**), 7.59 (dd, *J* = 1.1, 8.5 Hz, Ar**H**), 7.53 (d, *J =* 15.0 Hz, (*E*)-isomeric **H**), 7.44-7.48 (m, Ar**H**), 7.18 (dd, *J* = 2.5, 5.6 Hz, Ar**H**), 4.90 (s, N**H**), 3.93 (s, OC**H**₃), 3.91 (s, OCH₃); ¹³C NMR (100 MHz, DMSO-*d*₆) *δ* 167.2, 162.8, 155.1, 151.6, 142.1, 140.1, 135.5, 132.3, 127.1, 120.4, 113.1, 107.6, 56.6, 56.4.

### Example 4.5. Synthesis of (E)-(2-(3-chloropyridin-2-yl)vinyl)(imino)(3-methoxypyridin-2-yl)-λ⁶-sulfanone (Chemical Formula 1-33)

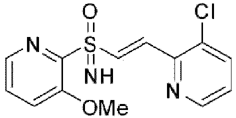

The method used for Examples 3.11.1 to 3.11.4 was used to synthesize diethyl ((3-methoxypyridine-2-sulfonimidoyl)methyl)phosphonate .

Using reaction (5)-2, diethyl ((3-methoxypyridine-2-sulfonimidoyl)methyl)phosphonate (0.15 g, 0.45 mmol) was fully dissolved in THF, and then 3-chloropicolinaldehyde (0.076 g, 0.54 mmol) and 2.0 M *n*-BuLi cyclohexane solution (0.27 mL, 0.54 mmol) were reacted therewith to synthesize compound 1-33 as a white powder (0.056 g, 40%): R*_{f}* = 0.33 (DCM 4 : Acetone 1); ¹H NMR (400 MHz, DMSO-*d*₆) *δ* 8.65 (d, *J=* 4.4 Hz, Ar**H**), 8.19 (d, *J =* 4.4 Hz, Ar**H**), 8.07 (d, *J =* 8.2 Hz, ArH), 7.97 (d, *J =* 14.7 Hz, (*E*)-isomeric **H**), 7.74-7.79 (m, Ar**H**, (*E*)-isomeric **H**), 7.65 (dd, *J =* 4.5, 8.4 Hz, Ar**H**), 7.53 (dd, *J =* 4.5, 8.2 Hz, Ar**H**), 4.89 (s, N**H**), 3.96 (s, OC**H**₃).

### Example 4.6. Synthesis of (E)-imino(3-methoxypyridin-2-yl)(2-(3-(trifluoromethyl)pyridin-2-yl)vinyl)-λ⁶-sulfanone (Chemical Formula 1-34)

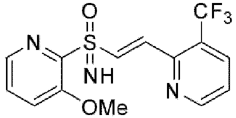

The method used for Examples 3.11.1 to 3.11.4 was used to synthesize diethyl ((3-methoxypyridine-2-sulfonimidoyl)methyl)phosphonate .

Using reaction (5)-2, diethyl ((3-methoxypyridine-2-sulfonimidoyl)methyl)phosphonate (0.15 g, 0.47 mmol) was fully dissolved in THF, and then 3-(trifluoromethyl)picolinaldehyde (0.090 mL, 0.71 mmol) and 2.0 M *n*-BuLi cyclohexane solution (0.28 mL, 0.57 mmol) were reacted therewith to synthesize compound 1-34 as a white powder (0.091 g, 56%): R*_{f}* = 0.29 (DCM 4 : Acetone 1); ¹H NMR (400 MHz, DMSO-*d*₆) *δ* 8.95 (d, *J =* 4.4 Hz, Ar**H**), 8.30 (d, *J* = 7.6 Hz, Ar**H**), 8.19-8.21 (m, Ar**H**), 7.91 (d, *J* = 14.4 Hz, (E)-isomeric **H**), 7.83 (dd, *J =* 1.4, 14.4 Hz, (*E*)-isomeric **H**), 7.78 (d, *J* = 7.9 Hz, Ar**H**), 7.69-7.72 (m, Ar**H**), 7.64-7.67 (m, Ar**H**), 4.95 (s, N**H**), 3.95 (s, OC**H₃**); ¹³C NMR (100 MHz, DMSO-*d*₆) *δ* 153.9, 153.1, 148.8, 148.2, 140.2, 136.5, 135.6 (q, *J*_{C-F} = 5.1 Hz), 135.0, 129.1, 125.5, 124.5 (q, *J*_{C-F} *=* 31.3 Hz), 124.0 (q, *J*_{C-F} *=* 271.1 Hz), 122.8, 56.9.

### Example 4.7. Synthesis of (E)-(2-(3-chloropyridin-2-yl)vinyl)(imino)(5-methoxypyridin-2-yl)-λ⁶-sulfanone (Chemical Formula 1-35)

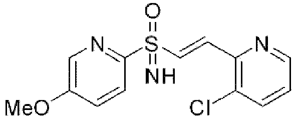

The method used for Examples 3.16.1 to 3.16.4 was used to synthesize diethyl ((5-methoxypyridine-2-sulfonimidoyl)methyl)phosphonate .

Using reaction (5)-2, diethyl ((5-methoxypyridine-2-sulfonimidoyl)methyl)phosphonate (0.15 g, 0.47 mmol) was fully dissolved in THF, and then 3-chloropicolinaldehyde (0.080 g, 0.57 mmol) and 2.0 M n-BuLi cyclohexane solution (0.28 mL, 0.57 mmol) were reacted therewith to synthesize compound 1-35 as a white powder (0.067 g, 42%): R*_{f}* = 0.29 (EtOAc 100%); ¹H NMR (400 MHz, DMSO-*d*₆) *δ* 8.59 (dd, *J=* 1.2, 4.5 Hz, Ar**H**), 8.41 (d, *J =* 2.8 Hz, ArH), 8.14 (d, *J=* 8.8 Hz, Ar**H**), 8.05 (dd, *J* = 1.3, 8.2 Hz, Ar**H**), 7.87 (d, *J =* 14.7 Hz, (*E*)-isomeric H), 7.61-7.67 (m, ArH, (*E*)-isomeric H), 7.49 (dd, *J* = 4.5, 8.2 Hz, Ar**H**), 4.95 (s, N**H**), 3.91 (s, OC**H₃**); ¹³C NMR (100 MHz, DMSO-*d*₆) *δ* 158.1, 151.6, 149.1, 147.9, 138.8, 138.6, 136.7, 134.1, 132.1, 127.1, 123.9, 121.9, 56.7.

### Example 4.8. Synthesis of (E)-(2-(3-fluoropyridin-2-yl)vinyl)(imino)(5-methoxypyridin-2-yl)-λ⁶-sulfanone (Chemical Formula 1-36)

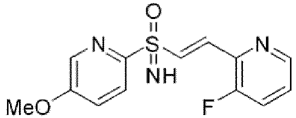

The method used for Examples 3.16.1 to 3.16.4 was used to synthesize diethyl ((5-methoxypyridine-2-sulfonimidoyl)methyl)phosphonate .

Using reaction (5)-2, diethyl ((5-methoxypyridine-2-sulfonimidoyl)methyl)phosphonate (0.13 g, 0.40 mmol) was fully dissolved in THF, and then 3-fluoropicolinaldehyde (0.060 g, 0.48 mmol) and 2.0 M n-BuLi cyclohexane solution (0.24 mL, 0.48 mmol) were reacted therewith to synthesize compound 1-36 as a white powder (0.029 g, 25%): R*_{f}* = 0.43 (n-hexane 1 : EtOAc 1); ¹H NMR (400 MHz, DMSO-*d*₆) *δ* 8.50-8.51 (m, Ar**H**), 8.41 (d, *J =* 2.8 Hz, Ar**H**), 8.13 (d, *J* = 8.8 Hz, Ar**H**), 7.83-7.88 (m, Ar**H**), 7.61-7.68 (m, (*E*)-isomeric 2**H**, Ar**H**), 7.55-7.59 (m, Ar**H**), 4.94 (s, N**H**), 3.91 (s, OC**H**₃); ¹³C NMR (100 MHz, DMSO-*d*₆) *δ* 158.1, 151.6, 146.9 (d, *J*_{C-F} = 4.8 Hz), 139.57 (d, *J*_{C-F} = 10.7 Hz), 138.6, 135.6 (d, *J*_{C-F} = 4.6 Hz), 131.8, 127.8 (d, *J*_{C-F} = 4.9 Hz), 125.2 (d, *J*_{C-F} = 18.9 Hz), 123.9, 121.9, 56.7.

### Example 4.9. Synthesis of (E)-imino(5-methoxypyridin-2-yl)(2-(3-(trifluoromethyl)pyridin-2-yl)vinyl)-λ⁶-sulfanone (Chemical Formula 1-37)

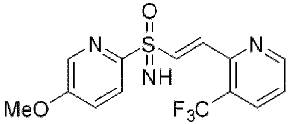

The method used for Examples 3.16.1 to 3.16.4 was used to synthesize diethyl ((5-methoxypyridine-2-sulfonimidoyl)methyl)phosphonate .

Using reaction (5)-2, diethyl ((5-methoxypyridine-2-sulfonimidoyl)methyl)phosphonate (0.13 g, 0.40 mmol) was fully dissolved in THF, and then 3-(trifluoromethyl)picolinaldehyde (0.08 mL, 0.60 mmol) and 2.0 M *n*-BuLi cyclohexane solution (0.24 mL, 0.48 mmol) were reacted therewith to synthesize compound 1-37 as a white powder (0.089 g, 65%): R*_{f}* = 0.50 (n-hexane 1 : EtOAc 1); ¹H NMR (400 MHz, DMSO-*d*₆) *δ* 8.89 (d, *J =* 4.2 Hz, ArH), 8.42 (d, *J* = 2.7 Hz, ArH), 8.28 (d, *J =* 7.9 Hz, ArH), 8.14 (d, *J* = 8.8 Hz, Ar**H**), 7.79 (d, *J =* 14.4 Hz, (*E*)-isomeric H), 7.62-7.73 (m, 2 ArH, (*E*)-isomeric *H*), 5.01 (s, N**H**), 3.91 (s, OC**H**₃).

### Example 4.10. Synthesis of (E)-(2-(3-chloropyridin-2-yl)vinyl)(imino)(pyridin-2-yl)-λ⁶-sulfanone (Chemical Formula 1-49)

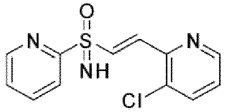

The method used for Examples 3.1.1 to 3.1.4 was used to synthesize diethyl ((N-(trimethylsilyl)pyridine-2-sulfonimidoyl)methyl)phosphonate .

Using reaction (5)-2, diethyl ((N-(trimethylsilyl)pyridine-2-sulfonimidoyl)methyl)phosphonate (0.39 g, 1.06 mmol) was fully dissolved in THF, and then 3-chloropicolinaldehyde (0.18 mg, 1.27 mmol) and 2.0 M n-BuLi cyclohexane solution (0.64 ml, 1.59 mmol) were reacted therewith to synthesize compound 1-49 as a white solid (0.10 g, 35%): R*_{f}* = 0.32 (n-hexane 1 : EtOAc 2); ¹H NMR (400 MHz, DMSO-*d*₆) *δ* 8.73 (d, 4.3 Hz, ArH), 8.73 (dd, 1.0, 4.5 Hz, ArH), 8.18 (d, 7.8 Hz, ArH), 8.12 (td, 1.6 7.6 Hz, ArH), 8.06 (dd, 1.3, 8.2 Hz, Ar**H**), 7.93 (d, *J* = 14.7 Hz, (*E*) - isomeric **H**), 7.73 (d, *J* = 14.7 Hz, (*E*) - isomeric **H**), 7.67 (ddd, 1.0, 4.5, 7.6 Hz, Ar**H**), 7.52 (dd, 4.5, 8.2 Hz, Ar**H**), 5.13 (brs, N**H**); ¹³C NMR (100 MHz, DMSO-*d*₆) *δ* 160.4, 150.4, 149.1, 147.9, 139.3, 138.9, 136.0, 135.0, 122.2, 127.5, 127.2, 121.9.

### Example 4.11. Synthesis of (E)-imino(2-(3-methoxypyridin-2-yl)vinyl)(pyridin-2-yl)-λ⁶-sulfanone (Chemical Formula 1-50)

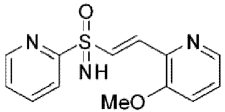

The method used for Examples 3.1.1 to 3.1.4 was used to synthesize diethyl ((N-(trimethylsilyl)pyridine-2-sulfonimidoyl)methyl)phosphonate .

Using reaction (5)-2, diethyl ((N-(trimethylsilyl)pyridine-2-sulfonimidoyl)methyl)phosphonate (0.35 g, 0.97 mmol) was fully dissolved in THF, and then 3-methoxypicolinaldehyde (0.16 g, 1.16 mmol) and 2.0 M n-BuLi cyclohexane solution (0.58 ml, 1.45 mmol) were reacted therewith to synthesize compound 1-50 as a white solid (0.09 g, 33%): R*_{f}* = 0.15 (n-hexane 1 : EtOAc 3); ¹H NMR (400 MHz, DMSO-*d*₆) *δ* 8.71 (d, 4.4 Hz, ArH), 8.23 (d, 4.4 Hz, ArH), 8.15 (d, 7.7 Hz, ArH), 8.10 (t, 7.5 Hz, ArH), 7.85 (d, *J=* 15.0 Hz, (*E*) - isomeric H), 7.66-7.53 (m, 2ArH, (E) - isomeric H), 7.52 (dd, 4.5, 8.4 Hz, ArH), 4.93 (brs, NH), 3.92 (s, OCH₃); ¹³C NMR (100 MHz, CD₃OD) *δ* 159.5, 155.5, 149.9, 141.3, 140.0, 138.7, 137.4, 130.7, 127.0, 126.6, 122.0, 119.5, 55.1.

### Example 4.12. Synthesis of (E)-(2-(3-fluoropyridin-2-yl)vinyl)(imino)(pyridin-2-yl)-λ⁶-sulfanone (Chemical Formula 1-51)

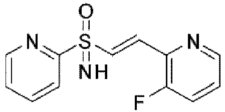

The method used for Examples 3.1.1 to 3.1.4 was used to synthesize diethyl ((N-(trimethylsilyl)pyridine-2-sulfonimidoyl)methyl)phosphonate .

Using reaction (5)-2, diethyl ((N-(trimethylsilyl)pyridine-2-sulfonimidoyl)methyl)phosphonate (0.33 g, 0.91 mmol) was fully dissolved in THF, and then 3-fluoropicolinaldehyde (0.14 g, 1.09 mmol) and 2.0 M n-BuLi cyclohexane solution (0.54 ml, 1.09 mmol) were reacted therewith to synthesize compound 1-51 as a white solid (0.03 g, 14%): R*_{f}* = 0.55 (n-hexane 1: EtOAc 3); ¹H NMR (400 MHz, DMSO-*d*₆) *δ* 8.73 (d, 4.2 Hz, ArH), 8.53 (d, 4.4 Hz, ArH), 8.19 (d, 7.8 Hz, ArH), 8.12 (td, 1.6, 6.8 Hz, ArH), 7.87 (t, 9.0 Hz, ArH), 7.75-7.65 (m, (E) - isomeric 2**H**, ArH), 7.61-7.57 (m, ArH), 5.13 (brs, NH); ¹³C NMR (100 MHz, DMSO-*d*₆) *δ* 160.5, 158.3 (d, *J*_{C-F} = 260.9 Hz), 150.4, 146.9 (d, *J*_{C-F} = 4.9 Hz), 139.5 (d, *J*_{C-F} = 11.0 Hz), 139.3, 134.9 (q, *J*_{C-F} = 4.3 Hz), 132.7, 128.0 (d, *J*_{C-F} = 4.6 Hz), 127.5, 125.2 (d, *J*_{C-F} = 18.9 Hz), 121.9

### Example 4.13. Synthesis of (E)-imino(pyridin-2-yl)(2-(3-(trifluoromethyl)pyridin-2-yl)vinyl)-λ⁶-sulfanone (Chemical Formula 1-52)

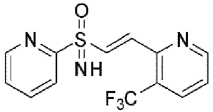

The method used for Examples 3.1.1 to 3.1.4 was used to synthesize diethyl ((N-(trimethylsilyl)pyridine-2-sulfonimidoyl)methyl)phosphonate .

Using reaction (5)-2, diethyl ((N-(trimethylsilyl)pyridine-2-sulfonimidoyl)methyl)phosphonate (0.30 g, 0.82 mmol) was fully dissolved in THF, and then 3-(trifluoromethyl)picolinaldehyde (0.12 ml, 0.91 mmol) and 2.0 M *n*-BuLi cyclohexane solution (0.45 ml, 0.91 mmol) were reacted therewith to synthesize compound 1-52 as a white solid (0.04 g, 17%): R*_{f}* = 0.21 (*n*-hexane 1 : EtOAc 3); ¹H NMR (400 MHz, DMSO-*d*₆) *δ* 8.92 (d, 4.3 Hz, Ar**H**), 8.75 (d, 4.3 Hz, ArH), 8.30 (d, 8.0 Hz, Ar**H**), 8.20 (d, 7.7 Hz, ArH), 8.14 (t, 7.6 Hz, Ar**H**), 7.89 (d, *J* = 14.4 Hz, (*E*) - isomeric **H**), 7.78 (d, *J* = 14.4 Hz, (*E*) - isomeric **H**), 7.72-7.67 (m, 2Ar**H**), 5.21 (brs, N**H**); ¹³C NMR (100 MHz, DMSO-*d*₆) *δ* 160.3, 153.8, 150.4, 148.6, 139.4, 137.5, 135.6 (q, *J*_{C-F} = 5.2 Hz), 134.6 (q, *J*_{C-F} = 2.2 Hz), 127.6, 125.6, 124.5 (q, *J*_{C-F} = 31.3 Hz), 124.0 (q, *J*_{C-F} = 272.1 Hz), 122.0

### Example 4.14. Synthesis of (E)-(2-(3-fluoropyridin-2-yl)vinyl)(imino)(5-(3-morpholinopropoxy)pyridin-2-yl)-λ⁶-sulfanone (Chemical Formula 1-53)

In an embodiment of the present disclosure, the synthesis process of compound 1-53 is shown by Reaction Scheme 5 presented below.

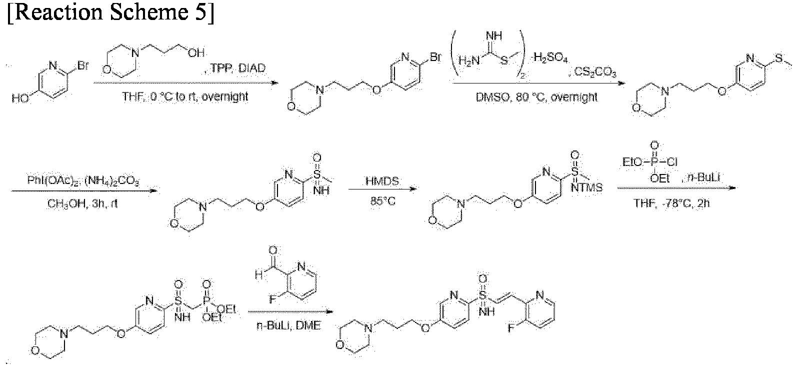

### 4.14.1. Introduction of morpholine group by Mitsunobu reaction

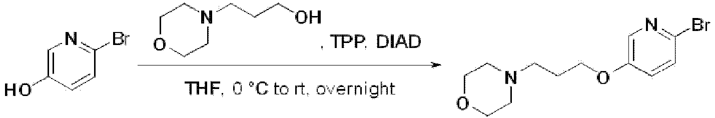

According to the above shown Reaction Scheme, 4-(3-((6-bromopyridin-3-yl)oxy)propyl)morpholine was synthesized. A bromo pyridine starting material (1.0 eq) substituted with a hydroxy group at the 3-position was dissolved in tetrahydrofuran (THF). 4-(3-hydroxypropyl) morpholine (1.3 eq) and triphenylphosphine (1.3 eq) were fully dissolved, and then diisopropyl azodicarboxylate (1.3 eq) was added dropwise thereto at 0°C, and the mixture was stirred at room temperature for 18 hours. After the reaction was completed, the reactant was diluted with ethyl acetate (EtOAc), and was washed with water and brine. The organic layer was dried over anhydrous Na₂SO₄ and was filtered. The solvent was distilled off under reduced pressure, and the residue obtained was purified by column chromatography using a mixed developing solvent of ethyl acetate and methanol to obtain 4-(3-((6-bromopyridin-3-yl)oxy)propyl)morpholine.

### 4.14.2. Synthesis of 4-(3-((6-bromopyridin-3-yl)oxy)propyl)morpholine

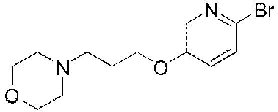

6-bromopyridin-3-ol (1.30 g, 7.47 mmol), 4-(3-hydroxypropyl)morpholine (1.34 ml, 9.71 mmol), triphenylphosphine (2.55 g, 9.71 mmol), and diisopropyl azodicarboxylate (1.91 ml, 9.71 mmol) were reacted to synthesize 4-(3-((6-bromopyridin-3-yl)oxy)propyl)morpholine as a yellow oil (2.12 g, 95%); R*_{f}* = 0.20 (EtOAc 9 : CH₃OH 1); ¹H NMR (400 MHz, CDCl₃) *δ* 8.06 (d, *J =* 3.0 Hz, Ar**H**), 7.36 (d, *J =* 8.7 Hz, Ar**H**), 7.10 (dd, *J =* 3.1, 8.7 Hz, Ar**H**), 4.06 (t, *J* = 6.2 Hz, CH₂), 3.72 (t, *J =* 4.5 Hz, 2C**H**₂), 2.51 (t, *J =* 7.1 Hz, C**H**₂), 2.46 (t, *J =* 4.4 Hz, 2C**H**₂), 2.01-1.94 (m, CH₂).

### 4.14.3. Synthesis of methylthiopyridine derivative from bromopyridine derivative

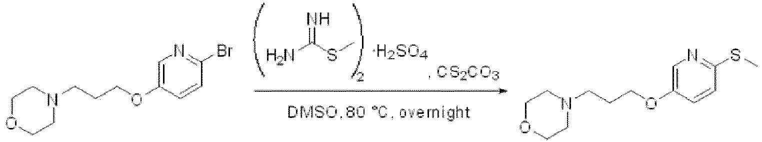

According to the above shown Reaction Scheme, 4-(3-((6-(Methylthio)pyridin-3-yl)oxy)propyl)morpholine was synthesized. 4-(3-((6-bromopyridin-3-yl)oxy)propyl)morpholine (1.0 eq) was dissolved in dimethyl sulfoxide (DMSO), and cesium carbonate (4.0 eq) and *S*-methylisothiourea sulfate (1.0 eq) were added. The solution was stirred at 80°C for 18 hours. After the reaction was completed, the reactant was diluted with ethyl acetate (EtOAc), and was washed with water and brine. The organic layer was dried over anhydrous Na₂SO₄ and was filtered. The solvent was distilled off under reduced pressure, and the residue obtained was purified by column chromatography using a mixed developing solvent of ethyl acetate and methanol to obtain 4-(3-((6-(methylthio)pyridin-3-yl)oxy)propyl)morpholine.

### 4.14.4. Synthesis of 4-(3-((6-(Methylthio)pyridin-3-yl)oxy)propyl)morpholine

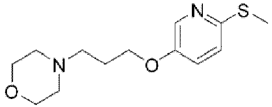

4-(3-((6-bromopyridin-3-yl)oxy)propyl)morpholine (2.12 g, 7.08 mmol), *S-*methylisothiourea sulfate (1.97 g, 7.08 mmol), and cesium carbonate (9.23 g, 28.34 mmol) were reacted to synthesize 4-(3-((6-(methylthio)pyridin-3-yl)oxy)propyl)morpholine as a yellow oil (1.84 g, 97%); R*_{f}* = 0.21 (EtOAc 9 : CH₃OH 1); ¹H NMR (400 MHz, CDCl₃) *δ* 8.17 (t, *J =* 1.8 Hz, Ar**H**), 7.11 (d, *J =* 1.7 Hz, Ar**H**), 4.06 (t, *J =* 6.2 Hz, C**H**₂), 3.72 (t, *J =* 4.6 Hz, 2C**H**₂), 2.55 (s, SC**H**₃) 2.51 (t, *J =* 7.1 Hz, C**H**₂), 2.46 (t, *J =* 4.4 Hz, 2C**H**₂), 2.00-1.93 (m, C**H**₃).

### 4.14.5. Synthesis of imino(methyl)(5-(3-morpholinopropoxy)pyridin-2-yl)-λ⁶-sulfanone

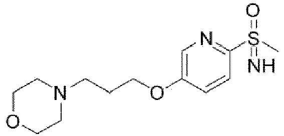

Using reaction (2), 4-(3-((6-(methylthio)pyridin-3-yl)oxy)propyl)morpholine (1.00 g, 3.75 mmol) was fully dissolved in MeOH, and then the solution was reacted with diacetoxyiodobenzene (2.78 g, 8.63 mmol) and ammonium carbonate (0.54 g, 5.63 mmol) to synthesize imino(methyl)(5-(3-morpholinopropoxy)pyridin-2-yl)-λ⁶-sulfanone as a yellow oil (0.61 g, 55%): R*_{f}* = 0.24 (EtOAc 93 : CH₃OH 7); ¹H NMR (400 MHz, CDCl₃) *δ* 8.37 (d, *J =* 2.7 Hz, Ar**H**), 8.08 (d, *J* = 8.7 Hz, Ar**H**), 7.34 (dd, *J =* 2.8, 8.7 Hz, Ar**H**), 4.16 (t, *J =* 6.2 Hz, C**H**₂), 3.73 (t, *J =* 4.4 Hz, 2C**H**₂), 3.49 (s, N**H**), 3.23 (s, SC**H**₃), 2.56 (t, *J* = 7.2 Hz, C**H**₂), 2.50 (t, *J =* 4.4 Hz, 2C**H**₂), 2.07-2.00 (m, C**H**₂).

### 4.14.6. Synthesis of Methyl(5-(3-morpholinopropoxy)pyridin-2-yl)((trimethylsilyl)imino)-λ⁶-sulfanone

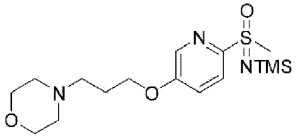

Using reaction (3), hexamethyldisilazane (0.88 mL, 4.19 mmol) was added to imino(methyl)(5-(3-morpholinopropoxy)pyridin-2-yl)-λ⁶-sulfanone (0.84 g, 2.79 mmol) to synthesize methyl(5-(3-morpholinopropoxy)pyridin-2-yl)((trimethylsilyl)imino)-λ⁶-sulfanone as a yellow oil (1.02 g, 100%): R*_{f}* = 0.61 (DCM 9 : CH₃OH 1); ¹H NMR (400 MHz, DMSO-*d*₆) *δ* 8.41 (d, *J =* 2.7 Hz, Ar**H**), 7.96 (d, *J* = 8.7 Hz, Ar**H**), 7.63 (dd, *J* = 2.8, 8.7 Hz, Ar**H**), 4.20 (t, *J =* 6.4 Hz, C**H**₂), 3.58 (t, *J =* 4.6 Hz, 2C**H**₂) , 3.11 (s, SC**H**₃), 2.44 (t, *J* = 7.2 Hz, C**H**₂), 2.38 (t, *J* = 4.4 Hz, 2C**H**₂), 1.96-1.90 (m, C**H**₂), 0.00 (s, Si(C**H**₃)₃).

### 4.14.7. Synthesis of diethyl ((5-(3-morpholinopropoxy)pyridine-2-sulfonimidoyl)methyl)phosphonate

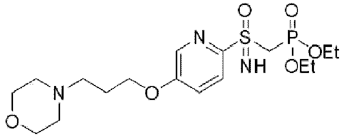

Using reaction (4), methyl(5-(3-morpholinopropoxy)pyridin-2-yl)((trimethylsilyl)imino)-λ⁶-sulfanone (2.70 g, 7.18 mmol) was fully dissolved in THF, and then diethylchlorophosphate (1.56 mL, 10.77 mmol) and 2.0 M n-BuLi cyclohexane solution (7.90 mL, 15.80 mmol) were reacted therewith to synthesize diethyl ((5-(3-morpholinopropoxy)pyridine-2-sulfonimidoyl)methyl)phosphonate as a yellow oil (1.21 g, 39%): R*_{f}* = 0.29 (EtOAc 4 : CH₃OH 1); ¹H NMR (400 MHz, DMSO-*d*₆) *δ* 8.39 (d, *J=* 2.8 Hz, ArH), 8.04 (d, *J* = 8.8 Hz, ArH), 7.63 (dd, *J* = 2.8, 8.8 Hz, ArH), 4.58 (s, NH), 4.22-4.15 (m, CH₂, SCH₂P), 4.06-3.99 (m, 2CH₃CH₂O), 3.58 (t, *J=* 4.6 Hz, 2CH₂), 2.44 (t, *J=* 7.2 Hz, CH₂), 2.37 (t, *J=* 4.4 Hz, 2CH₂), 1.97-1.91 (m, CH₂), 1.16 (t, *J=* 7.1 Hz, 2CH₃CH₂O).

### 4.14.8. Synthesis of (E)-(2-(3-fluoropyridin-2-yl)vinyl)(imino)(5-(3-morpholinopropoxy)pyridin-2-yl)-λ⁶-sulfanone (Chemical Formula 1-53)

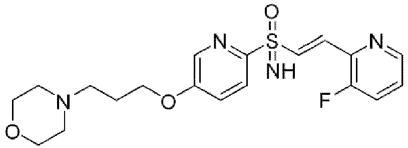

Using the above shown Reaction Scheme (5)-2, diethyl ((5-(3-morpholinopropoxy)pyridine-2-sulfonimidoyl)methyl)phosphonate (0.10 g, 0.23 mmol) was fully dissolved in THF, and then 3-fluoropicolinaldehyde (0.03 g, 0.25 mmol) and 2.0 M n-BuLi cyclohexane solution (0.13 ml, 0.25 mmol) were reacted therewith to synthesize compound 1-53 as a bright yellow oil (0.03 g, 31%): R*_{f}* = 0.11 (EtOAc 4 : CH₃OH 1); ¹H NMR (400 MHz, DMSO-*d*₆) *δ* 8.51 (d, *J=* 4.5 Hz, ArH), 8.40 (d, *J=* 2.8 Hz, ArH), 8.12 (d, *J=* 8.8 Hz, ArH), 7.86 (t, *J=* 9.5 Hz, ArH), 7.68-7.55 (m, (E)-isomeric 2H, 2ArH), 4.93 (s, NH), 4.18 (t, *J* = 6.3 Hz, CH₂), 3.56 (t, *J =* 4.4 Hz, 2CH₂), 2.42 (t, *J* = 6.9 Hz, CH₂), 2.36 (t, *J* = 4.4 Hz, 2CH₂), 1.94-1.87 (m, CH₂).

### Experimental Example 1. Evaluation of Nrf2 activation effect and cytotoxicity

In order to confirm the pharmacological activity of the compounds synthesized according to the results of Examples 1 to 4 of the present disclosure, an Nrf2 functional cell-based assay (DiscoveRx) was established to evaluate the effect of the synthesized derivatives on inducing the activation of Nrf2, a major regulator of oxidative stress defense. U2OS cells were used, and the cell pellet was dissolved with CP 0 reagent, then 80 µL of the cell pellet solution was seeded into a 96-well plate at a density of 1.5×10⁴ cells/well and was cultured for 24 hours. The compounds synthesized according to the above Examples 1 to 4 were diluted 3-fold in DMSO to seven concentrations to prepare stock solutions. 10 µL of each compound solution was mixed with 90 µL of CP 0, and 20 µL of the mixed solution was added to each well of the 96-well plate. Each experiment was repeated 3 times. As a positive control, the cell pellet was treated with 5 µM sulforaphane and Dimethyl fumarate (DMF), and the plate was wrapped in foil and incubated for 6 hours. Substrate reagents 1 and 2 were mixed in cell assay buffer. Then, 50 µL of the substrate reagent was added to each well of the 96-well plate. The plate was wrapped in foil, and incubated for 1 hour. Then, the luminescence value was measured at all wavelengths and an integration time of 1000 ms using a luminescence spectrophotometer (Molecular Devices). The effect of each compound was expressed as the concentration that activated 50% of the maximum effect at high concentration (Table 1).

**[Table 1]**

| **Compound** | **Nrf2 activation EC₅₀ (µM)** | **Compound** | **Nrf2 activation EC₅₀ (µM)** |
|---|---|---|---|
| 1-1 | 5.77 | 1-31 | 1.715 |
| 1-2 | > 10 | 1-32 | 5.9 |
| 1-3 | 1.014 | 1-33 | 0.198 |
| 1-4 | 0.154 | 1-34 | 0.378 |
| 1-5 | 1.54 | 1-35 | 0.339 |
| 1-6 | 0.316 | 1-36 | 3.278 |
| 1-7 | 2.596 | 1-37 | 1.074 |
| 1-8 | 1.778 | 1-38 | 0.119 |
| 1-9 | 0.34 | 1-39 | 0.75 |
| 1-10 | 1.22 | 1-40 | 0.143 |
| 1-11 | 1.241 | 1-41 | 0.042 |
| 1-12 | > 10 | 1-42 | 0.201 |
| 1-13 | 1.036 | 1-43 | 0.687 |
| 1-14 | 3.278 | 1-44 | 5.203 |
| 1-15 | 0.717 | 1-45 | 0.104 |
| 1-16 | > 10 | 1-46 | 0.12 |
| 1-17 | 1.345 | 1-47 | 0.102 |
| 1-18 | 2.7 | 1-48 | 0.735 |
| 1-19 | 0.264 | 1-49 | 0.352 |
| 1-20 | > 10 | 1-50 | 9.177 |
| 1-21 | 0.422 | 1-51 | 1.286 |
| 1-22 | 2.525 | 1-52 | 0.487 |
| 1-23 | 4.531 | 1-53 | 0.604 |
| 1-24 | 4.958 | 1-54 | < 0.300 |
| 1-25 | 0.437 | 1-55 | < 0.300 |
| 1-26 | 0.604 | 1-56 | 2.129 |
| 1-27 | 0.328 | 1-57 | 0.075 |
| 1-28 | 0.108 | 1-58 | 0.658 |
| 1-29 | 0.723 | DMF | 4.327 |
| 1-30 | 0.316 | Sulforaphane | 0.58 |

As shown in Table 1 above, it was found that the compounds of Examples 1 to 4 of the present disclosure (Compounds 1 to 55) induced Nrf2 activity, and in particular, compound 1-4, 1-6, 1-9, 1-19, 1-21, 1-25, 1-27, 1-28, 1-30, 1-33, 1-34, 1-35, 1-38, 1-40, 1-41, 1-42, 1-45, 1-46, 1-47, 1-49, 1-52, 1-54, 1-55, and 1-57 exhibited a better Nrf2 activation effect than Sulforaphane, which is well known as an Nrf2 activator.

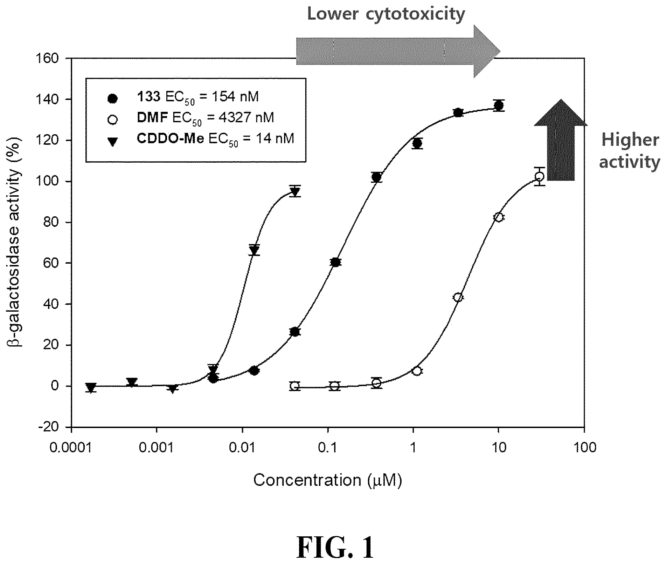

Among the compounds of the present disclosure, compound 1-41, which exhibited the best Nrf2 activation activity, exhibited approximately 14-fold superiority over sulforaphane in activation effect. Also, compound 1-4 exhibited approximately 30-fold superiority over DMF, and exhibited approximately 4-fold superiority over sulforaphane in Nrf2 activation activity, and exhibited 250-fold lower cytotoxicity than Bardoxolone-methyl (CDDO-Me) (Fig. 1).

Sulforaphane is known to exhibit high toxicity at concentrations of 5 µM or higher due to its strong non-selective reactivity despite its high level of activity resulting from the presence of an isothiocyanate group (NCS). The results according to the experimental examples of the present disclosure suggest that the compounds of the present disclosure, which were found to induce Nrf2 activity even at 10 µM without causing cytotoxicity, can be used as Nrf2 activators in place of sulforaphane.

### Experimental Example 2. Evaluation of CYP activity inhibition ability

In order to determine the feasibility of utilizing compounds according to embodiments of the present disclosure as drugs actually administered in the body, the inhibition ability of CYP activity, which is involved in about 75% of drug metabolism, was examined. Compared to compounds containing a sulfonic group as a core and having other similar functional groups, the inhibition activity of compounds 1-1, 1-2, 1-6, 1-8, 1-9, 1-10, 1-12, and 1-13 of the present disclosure containing sulfanone was evaluated against five isoenzymes, 2C19, 2D6, 2C9, 1A2, and 3A4, which reportedly account for more than 90% of the total metabolism by CYP.

Human liver microsomes (0.25 mg/mL), 0.1 M phosphate buffer (pH 7.4), a substrate drug cocktail of the above five drug-metabolizing enzymes (Phenacetin 50 µM, Diclofenac 10 µM, S-mephenytoin 100 µM, Dextromethorphan 5 µM, and Midazolam 2.5 µM) and 1-1, 1-2, 1-6, 1-8, 1-9, 1-10, 1-12, and 1-13 were added at concentrations of 0 or 10 µM, respectively, and this mixture was pre-incubated at 37°C for 5 minutes. Then, a NADPH generation system solution was added thereto and the mixture_was incubated at 37°C for 15 minutes. Thereafter, the reaction was quenched by adding an acetonitrile solution containing an internal standard (Terfenadine), and the mixture was centrifugated for 5 minutes (14,000 rpm, 4°C), and then the supernatant obtained was injected into an LC-MS/MS system to simultaneously analyze metabolites of the substrate drugs and evaluate the inhibition ability for the drug-metabolizing enzymes of these compounds. The metabolites of each CYP isoenzyme indicator drug generated through the above mentioned reaction were analyzed using a Shimadzu Nexera XR system and TSQ vantage (Thermo). A Kinetex C18 column was used as an HPLC column, and distilled water containing 0.1% formic acid and acetonitrile containing 0.1% formic acid were used as mobile phases. The generated metabolites were quantified using the multiple reaction monitoring (MRM) quantitative mode, and the data were analyzed with Xcalibur (version 1.6.1). The activity inhibition capacity for each CYP isoenzyme was converted into % activity relative to the negative control group to which the above mentioned compounds were not added (Table 2).

**[Table 2]**

| **Compound** | **Chemical Formula** | **CYP activity (%)** | | | | |
|---|---|---|---|---|---|---|
| | | **2C19** | **2D6** | **2C9** | **1A2** | **3A4** |
| 1-1 | 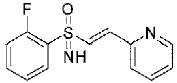 | **66.9** | 82.7 | 85.7 | 87.8 | **121** |
| Comparative example | 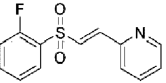 | 38.4 | 92.4 | 230 | 93.0 | 104 |
| 1-2 | 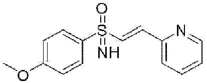 | **70.4** | 98.6 | 96.6 | **88.9** | 97.5 |
| Comparative example | 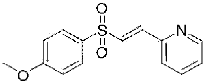 | 9.7 | 102 | 105 | 82.8 | 82.9 |
| 1-6 | 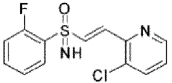 | **69.8** | **93.5** | **86.7** | 85.7 | **118** |
| Comparative example | 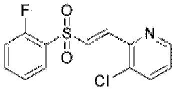 | 16.6 | 86.1 | 45.5 | 95.8 | 105 |
| 1-8 | 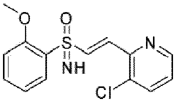 | **24.4** | 93.9 | 192 | **84.4** | **78.4** |
| Comparative example | 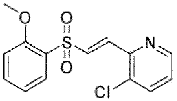 | 11.1 | 98.4 | 209 | 78.5 | 48.6 |
| 1-9 | 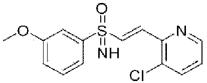 | 11.1 | **98.4** | **209** | 78.5 | **48.6** |
| Comparative example | 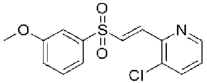 | 3,4 | 93.2 | 31.9 | 81.3 | 38.0 |
| 1-10 | 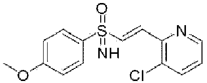 | **6.0** | **112** | 133 | **103** | **109** |
| Comparative | 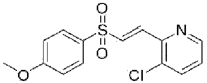 | 3.8 | 97.5 | 196 | 88.2 | 91.8 |
| example | | | | | | |
| 1-12 | 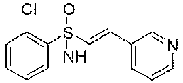 | **68.2** | 94.4 | 87.1 | **86.8** | **104** |
| Comparative example | 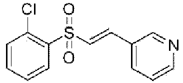 | 33.4 | 101 | 118 | 84.0 | 74.2 |
| 1-13 | 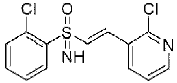 | 27.8 | **95.7** | **169** | **93.3** | 59.8 |
| Comparative example | 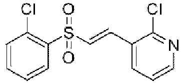 | 28.3 | 93.8 | 88.7 | 85.6 | 81.2 |

As shown in Table 2 above, the results from this evaluation of the feasibility of drug interaction with drug-metabolizing enzymes for each compound show that the compounds of the present disclosure did not exhibit inhibitory activity against the five CYP isoenzymes, suggesting that there is little feasibility of drug interaction with drug-metabolizing enzymes for the present compounds, compared to the comparative examples.

### Experimental Example 3. Evaluation of metabolic stability

Metabolic stability is a factor that may affect pharmacokinetic parameters (PK parameters), such as drug clearance, half-life, and oral bioavailability, and is therefore one of the characteristics that drug candidates must possess. Therefore, in order to predict the extent of drug metabolism by the liver, the main organ for drug metabolism, through *in vitro* experiments, the metabolic stability of drugs was evaluated using liver microsomes.

A stability test using microsomes was performed in which compounds 1-2, 1-4, and 1-6 of the present disclosure containing sulfanone and compounds containing a sulfone group as a core and having the same functional groups were treated with microsomes and this mixture was incubated for a certain period of time to determine the amount of drug that remains. The compounds were added at a concentration of 1 µM to human liver microsomes (0.5 mg/mL) and 0.1 M phosphate buffer solution (pH 6.4), and this mixture was pre-incubated at 37°C for 5 minutes. Then, an NADPH regeneration system solution was added thereto and the mixed solution was incubated at 37°C for 30 minutes. Thereafter, the reaction was quenched by adding an acetonitrile solution containing an internal standard (chloropropamide), and the mixture was centrifugated for 5 minutes (14,000 rpm, 4°C). Then, the supernatant obtained was injected into an LC-MS/MS system to analyze the substrate drugs, and thereby evaluate the metabolic stability of said compounds. The amount of substrate remaining during the reaction was analyzed using a Shimadzu Nexera XR system and TSQ vantage. A Kinetex C18 column was used as an HPLC column, and distilled water containing 0.1% formic acid and acetonitrile containing 0.1% formic acid were used as mobile phases. The data were analyzed with Xcalibur (version 1.6.1) (Table 3).

**[Table 3]**

| **Compound** | **Chemical Formula** | **Human (% remaining)** | **Mouse (% remaining)** | **Rat (% remaining)** |
|---|---|---|---|---|
| 1-2 | 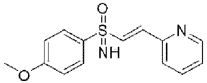 | 76 | 59 | |
| Comparative example | 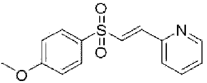 | 56 | | |
| 1-4 | 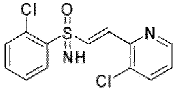 | 84 | 47 | |
| Comparative example | 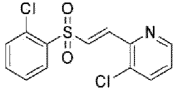 | 66 | 4.5 | |
| 1-6 | 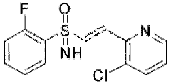 | 96 | 72 | |
| Comparative example | 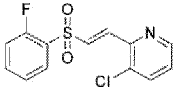 | 72 | 19 | 23 |

As shown in Table 3 above, the amount of drug remaining after incubation of each compound with microsomes for 30 minutes was measured, and it was found that compounds 1-2, 1-4, and 1-6 of the present disclosure were not degraded by metabolism after 30 minutes and remained at a high concentration. This suggests that the compounds of the embodiments of the present disclosure have significantly improved stability against human liver microsomes compared to the comparative examples.

### Experimental Example 4. Evaluation for Solubility

In drug discovery, a drug's solubility is one of the most important factors associated with bioabsorption rate. For example, compounds with poor solubility in aqueous solutions may exhibit poor bioabsorption rates in general. In addition, drugs with low solubility may crystallize in tissues or cause serious toxicity, so the FDA requires additional research results for oral drugs with low solubility. Furthermore, low solubility is also the biggest cause of a failure in the development of candidate drugs. Therefore, the solubility of compound 1-4 of the present disclosure in 1% DMSO was measured.

Compound 1-4 showed high solubility of 0.5 mg/mL or more, whereas compounds containing a sulfone group as a core and having similar functional groups (comparative examples) exhibited very low solubility of 0.01 mg/mL or less. This suggests that the solubility of the compounds of the present disclosure was improved more than 50-fold by adopting sulfanone as a core.

### Experimental Example 5. Anti-inflammatory effect

The activation of Nrf2, an anti-inflammatory factor, by the compound of the present disclosure was confirmed through experimental example 1 mentioned above. As such, in order to confirm the anti-inflammatory effect of the compounds of the present disclosure, the anti-inflammatory effect of activated Nrf2 was determined by evaluating the NO (nitric oxide) inhibitory activity (Table 4).

BV-2 mouse microglial cells were seeded at a density of 1.5×10⁵ cells/well in a 12-well plate and were incubated for 24 hours. After all the cell culture medium was removed, the compound stock solutions dissolved in DMSO were diluted with serum-free medium (DMEM/High glucose + 100 U/mL penicillin/streptomycin) to make a final concentration of 0.1 µM, 1 µM, and 10 µM in 0.1% DMSO, and 900 µl per well was treated and incubated for 4 hours. To induce an inflammatory response in microglial cells, 100 µl of lipopolysaccharide (LPS) was treated per well to make a final concentration of 1 µg/ml and additionally incubated for 24 hours. The cell culture medium was centrifuged at 2356 g for 3 minutes to remove dead cell debris, and 50 µl of the supernatant was added to each well in a transparent 96-well plate. 50 µl of sulfanilamide solution (1% sulfanilamide in 5% phosphoric acid) was added to each well and the mixed solution was reacted for 5 minutes at room temperature. Then, 50 µl of NED solution (0.1% N-1-napthylethylenediamine dihydrochloride in water) was added thereto and the solution was reacted for 5 minutes at room temperature. The NO concentration in each cell culture medium was determined by measuring the absorbance at a wavelength of 540 nm using a microplate reader (SpectraMax^{®}i3 microplate reader, Molecular Devices) and comparing it to a standard curve using sodium nitrite. The NO concentration in each compound treatment was converted into % activity relative to the positive control treated with LPS.

**[Table 4]**

| **Compound** | **Amount of NO (% of control)** | | |
|---|---|---|---|
| | **0.1 µM** | **1 µM** | **10 µM** |
| 1-1 | 96.96 | 74.22 | < 3.00 |
| 1-2 | 110.08 | 115.06 | 35.83 |
| 1-3 | 90.94 | 51.43 | < 3.00 |
| 1-4 | 68.92 | 30.51 | < 3.00 |
| 1-5 | 101.32 | 69.24 | 7.96 |
| 1-6 | 82.94 | 43.68 | < 3.00 |
| 1-7 | nd | nd | 1.48 |
| 1-8 | nd | nd | 14.22 |
| 1-9 | nd | nd | < 3.00 |
| 1-10 | nd | nd | < 3.00 |
| 1-12 | nd | nd | 24.84 |
| 1-13 | nd | nd | 9.26 |
| 1-14 | nd | nd | < 3.00 |
| 1-15 | nd | nd | < 3.00 |
| 1-16 | nd | nd | < 3.00 |
| 1-17 | nd | nd | < 3.00 |
| 1-18 | nd | nd | < 3.00 |
| 1-19 | nd | nd | < 3.00 |
| 1-20 | nd | nd | 20.31 |
| DMF | 113.02 | 60 | 16.32 |
| Sulforaphane | 107.3 | 79.89 | 14.92 |

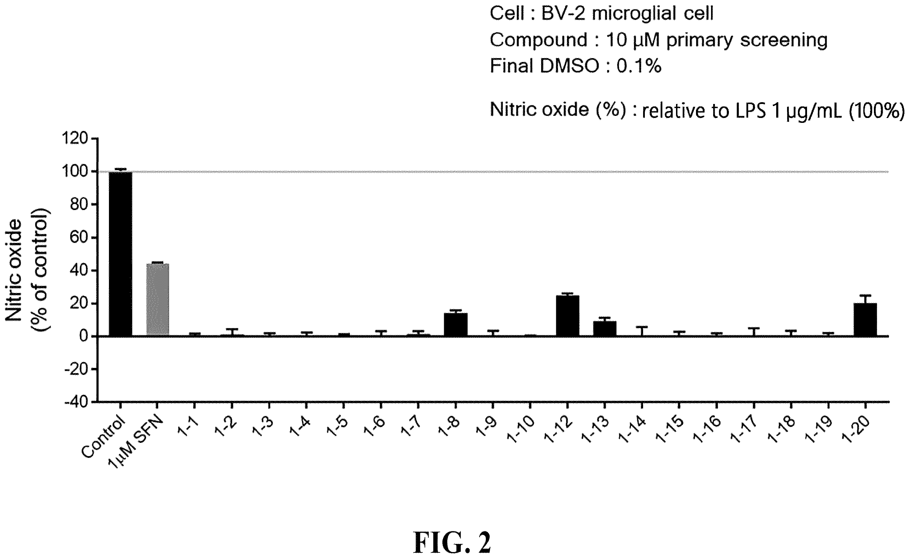

As shown in Table 4 above, the compounds of the present disclosure were shown to inhibit NO, and most of the compounds exhibited better anti-inflammatory effects than sulforaphane and DMF, which are well known Nrf2 activators. In particular, compound 1-4 inhibited NO by about 70% compared to the negative control group treated only with LPS even at a low concentration of 1 µM (Fig. 2).

This suggests that the compounds of the present disclosure may exhibit anti-inflammatory effects through concentration-dependent NO inhibition.

### Experimental Example 6. Antioxidant Effect

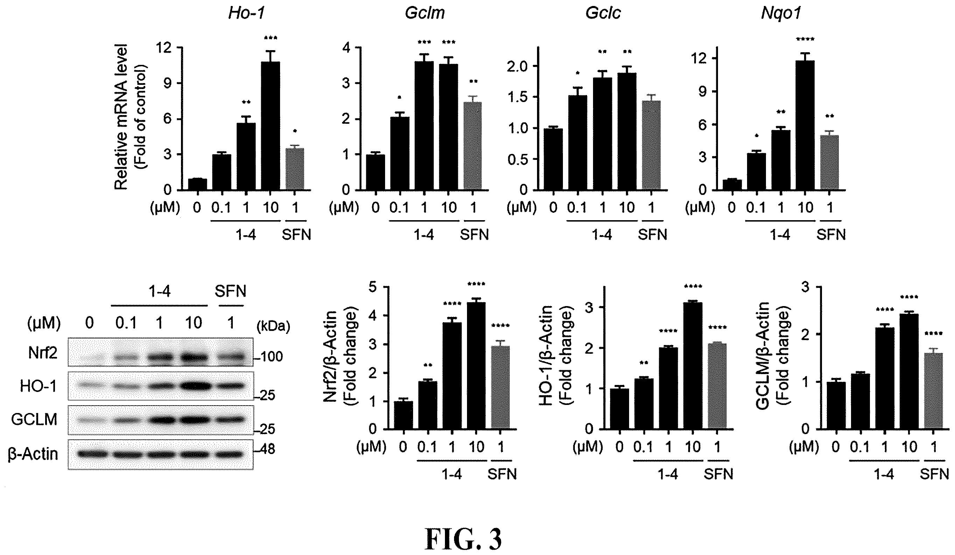

Nrf2 is a transcription factor that acts to protect cells by increasing antioxidant enzymes in the nucleus. Through experimental example 1 mentioned above, the activation of Nrf2 by the compound of the present disclosure was confirmed. Hence, whether the activated Nrf2 increases antioxidant enzymes was examined by measuring the amount of antioxidant enzymes using Western blot. As antioxidant enzymes to be detected, GCLC, GCLM, and HO-1, whose expression is known to be increased by Nrf2, were selected. Specifically, 1.5 mL of BV2 cells were seeded at a density of 5×10⁵ cells/well in a 6-well plate and were cultured in RPM1640 medium for 24 hours. The next day, compound 1-4 were mixed into each medium to a final concentration of 0 to 10 µM under 0.1% DMSO conditions, and then the cells were added thereto and the mixture was cultured for 24 hours. The cultured cells were harvested, lysed, and total proteins were extracted from the lysed cells and were subjected to electrophoresis using 10% SDS-PAGE. All the electrophoresis extracts were transferred to a PVDF membrane and were blocked with bovine serum albumin. Afterwards, the amount of antioxidant enzymes expressed in the cells was measured using antibodies corresponding to each antioxidant enzyme. The results are shown in Fig. 3.

A significant increase in antioxidant enzymes was visually observed when compound 1-4 were untreated and treated at low concentrations of 0.1 µM to high concentrations of 10 µM, with all showing the highest expression at 10 µM. This suggests that compounds of the present disclosure, such as compound 1-4, may increase the expression of antioxidant enzymes through concentration-dependent Nrf2 activation, thereby exhibiting a cell-protective effect.

### Experimental Example 7. Cytoprotective effect in MPTP mouse, a Parkinson's disease model

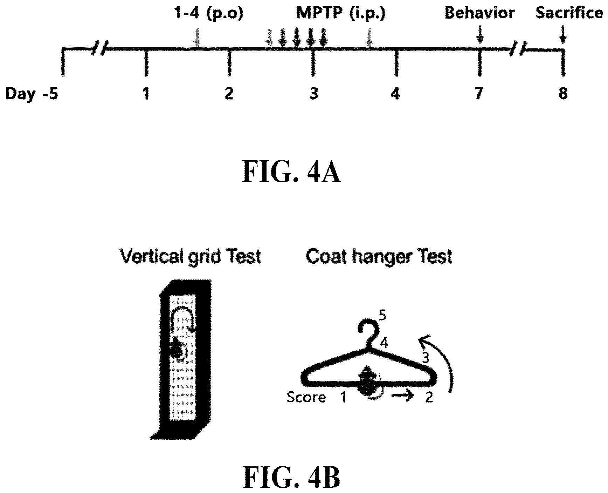

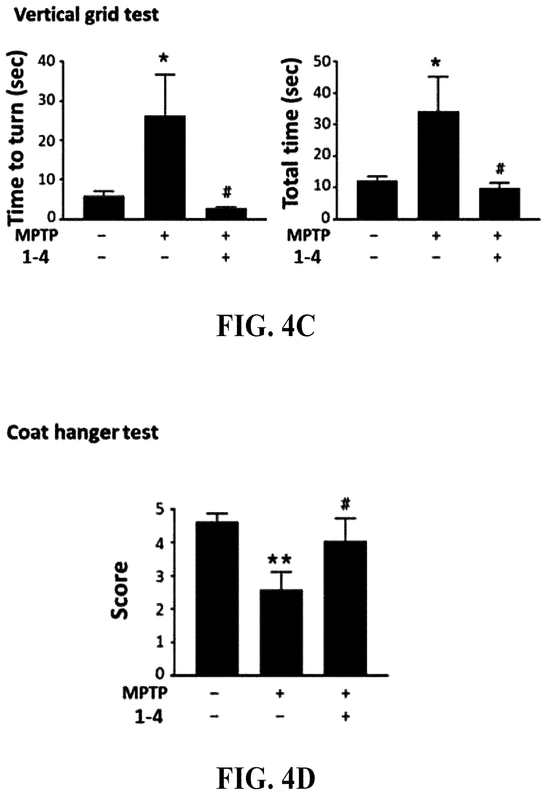

Experimental Examples 1 and 6 directly demonstrated that compound 1-4 of the present disclosure activate intracellular Nrf2 and increase the expression of antioxidant enzymes. Hence, in order to determine whether the compounds of the present disclosure also exhibit a cytoprotective effect in an animal model, a mouse animal model with Parkinson's disease was used in which the neurotoxin MPTP (1-methyl-4-phenyl-1,2,3,6-tetrahydropyridine) was administered to kill only dopaminergic neurons to induce Parkinson's disease. A specifically designed animal experiment schedule is shown in Fig. 4a. The animal model was prepared using 10-week-old C57/BL6 mice, which were divided into three groups: a negative control group that received saline intraperitoneally and were orally administered with a solution containing no compound 1-4; a positive control group that received MPTP intraperitoneally and were orally administered with a solution containing no compound 1-4; and an experimental group that received MPTP intraperitoneally and were orally administered with a solution containing compound 1-4. The MPTP-induced Parkinson's disease model was prepared by intraperitoneally injecting 20 mg/kg of MPTP at 2-hour intervals four times a day. The negative control group was injected with MPTP-free saline at the same time points. In order to confirm whether Parkinson's disease was induced, motor impairment due to dopaminergic neuron death, which is known as a major symptom of Parkinson's disease, was examined using the vertical grid test and the coat-hanger test. Compound 1-4 were orally administered once a day at a dose of 20 mg/mL for 3 days starting from the day before MPTP injection, and behavior experiments were conducted 6 days after the day of MPTP injection. The results are shown in Fig. 4, respectively.

The vertical grid test evaluates the time it takes to place an experimental animal model on a vertical ladder and for the animal to descend and return. In the present disclosure, the time the mouse turns toward the bottom from when the mouse is placed on the ladder is measured, as well as the total time it takes the mouse to descend the ladder. As shown in the graph of Fig. 4c, in the positive control group that received MPTP alone, the time to turn and the total time were increased compared to the negative control group of normal mice, indicating that motor ability was reduced in the MPTP-induced Parkinson's disease model. On the other hand, in the experimental group that were administered with compound 1-4 of the present disclosure, the time to turn and the total time were decreased to a level similar to that of the negative control group. The experimental group showed a significant difference from the positive control group described above.

The coat-hanger test, which is another behavior test for evaluating motor ability, was performed. In this test, as shown in the graph of Fig. 4b, the mouse is placed on the middle of coat-hanger 30 cm away from the floor, the mouse is given three minutes to climb up to a safe position at the top of the suspended coat hanger and then a score is given to the position the mouse reaches in that time. As shown in the graph in Fig. 4d, most of the mice in the negative control group moved to the top of the coat hanger, scoring 5 points, but in the case of the positive control group, most of the mice in the positive control group stopped at one end or the other of the bottom of the coat-hanger, scoring 2 to 3 points, and a significant number of the mice in the experimental group administered with compound 1-4 of the present disclosure moved to the top of the coat hanger, scoring 4 or 5 points, the group scoring a high score of an average of 4 points or more.

Taken together, the results of the above mentioned two motor ability experiments show that the compound of the present disclosure recovered the motor ability lowered by MPTP to a level similar to that of normal mice.

### Experimental Example 8. Effect on dopaminergic neurons in Parkinson's disease model, MPTP mice

From the results of Experimental Example 7, the effect of the compounds of the present invention of recovering motor ability was confirmed. In order to determine whether the recovery of motor ability was caused by the neuroprotective effect of the compounds of the present disclosure, from each mouse the brain was extracted and was analyzed by immunohistochemistry (IHC) using tyrosine hydroxylase, which is used as a marker of dopaminergic neurons. The striatum and substantia nigra, where dopaminergic neurons are densely located, were selected as the areas for analysis.

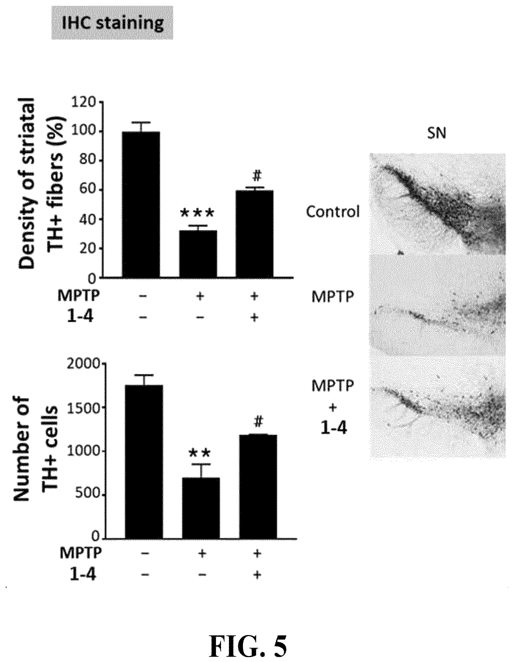

The negative control group, the positive control group, and the experimental group of mice were anesthetized with Avertin, physiological saline and 4% formaldehyde were perfused into the heart of each mouse, and the brains of the mice were extracted and immersed in 4% formaldehyde and 30% sucrose in that order. The brains were stored in a refrigerator for one day. Then, tissue sections of the striatum and substantia nigra were obtained from the stored brains using Cryomicrotome. The brain tissue sections were stained with DAB staining using an antibody for tyrosine hydroxylase specifically expressed in dopaminergic neurons by performing immunohistochemical staining, and these tissue sections were observed under a microscope. The results are shown in Fig. 5.

By immunohistochemical staining as described above, the areas containing tyrosine hydroxylase were stained brown, indicating the presence of living dopaminergic neurons. As shown in the right side of Fig. 5, the stained striatum and substantia nigra of the negative control group, which includes normal mice, were stained dark brown, but the stained striatum and substantia nigra of the positive control group in which the disease was induced with MPTP, were stained a considerably lighter brown, which may be because a large number of dopaminergic neurons were killed by the MPTP treatment. On the other hand, as also shown in the right side of Fig. 5?, the stained striatum and substantia nigra of the experimental group treated with compound 1-4 of the present disclosure, were stained dark brown, which is considered to be caused by the antioxidant effect of administering compound 1-4, which has the cytoprotective effect of inhibiting the death of dopaminergic neurons by MPTP. The above mentioned results indicate that the compounds of the present disclosure have the effect of preventing or treating neurodegenerative diseases such as Parkinson's disease caused by the death of dopaminergic neurons.

### Experimental Example 9. Effects of enhancing memory and cognitive function in Alzheimer's disease model APP/PS1 mice

To confirm whether the compounds of the present disclosure exhibit cognitive function improvement efficacy in an animal model, a mouse animal model of APP/PS1 (APPswe/PSEN1dE9, stock number 004462) genetically modified to induce Alzheimer's dementia was used. Compound 1-4 were dissolved in a mixed excipient containing 12.5% DMSO, 5% Solutol HS15, and 82.5% tertiary distilled water, and mice were orally administered with compound 1-4 at a dosage of 20 mg/kg and 250 µl per day for 2 months from the age of 10 months. A behavior experiment to evaluate cognitive function in the mice was conducted when the mice reached the age of 12 months when cognitive impairment appeared.

### Example 9.1. Morris water maze test

The spatial learning and memory ability of mice was evaluated using the Morris water maze test. A white circular water tank (diameter 150 cm, height 58 cm) was filled with water at 22±2°C, and a white circular escape platform having a diameter of 20 cm with a support was positioned 1.5 cm lower than the water surface. The water maze was divided into four quadrants: northeast, northwest, southeast, and southwest, and the escape platform was placed in the northwest quadrant. Then, mice were released from the northeast, east, south, and southwest directions. Differently shaped spatial cues were attached to the north, east, and south facing walls so that the mice could recognize each direction, and white water-based non-toxic paint was dissolved in the water so that the mice could not recognize the location of the escape platform. The mice were trained to find the escape platform for 60 seconds in each direction, a total of four times a day at two-hour intervals, for 8 days (acquisition test), and on day 9, the escape platform was removed and the mice were allowed to swim freely for 90 seconds (probe test). Then, the escape platform was positioned in the opposite southeast quadrant, and the mice were trained for 4 days from day 10 to day 13 (reversal test). On day 14, the escape platform was removed and the mice were allowed to swim freely for 90 seconds (reversal probe test). All experimental procedures were recorded with a video camera, and the Ethovision XT 11.5 (Noldus) program was used to analyze the time the mice spent climbing onto the escape platform during the training process (acquisition test, reversal test), the time the mice stayed in the target quadrant where the escape platform was located (time spent in target zone), the number of times the mice passed the escape platform location (platform crossover), and the time it took for the mice to first reach the escape platform location (latency to platform).

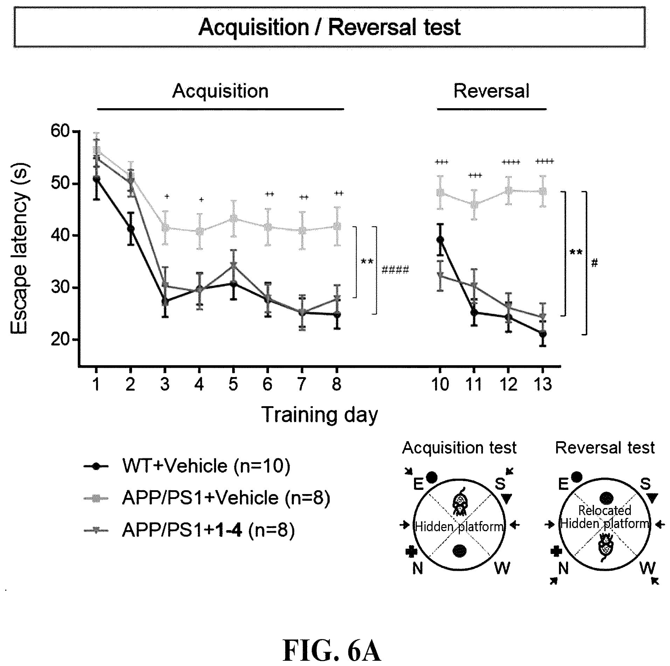

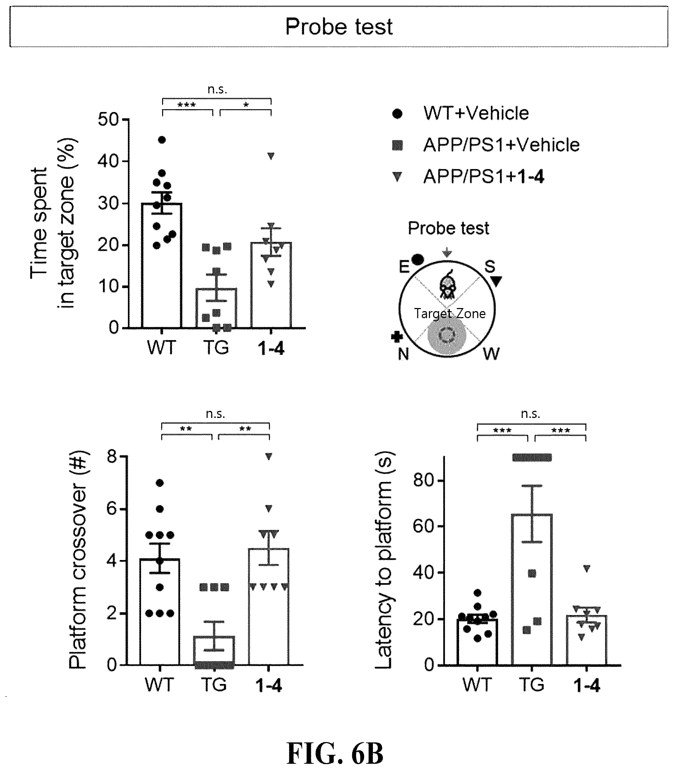

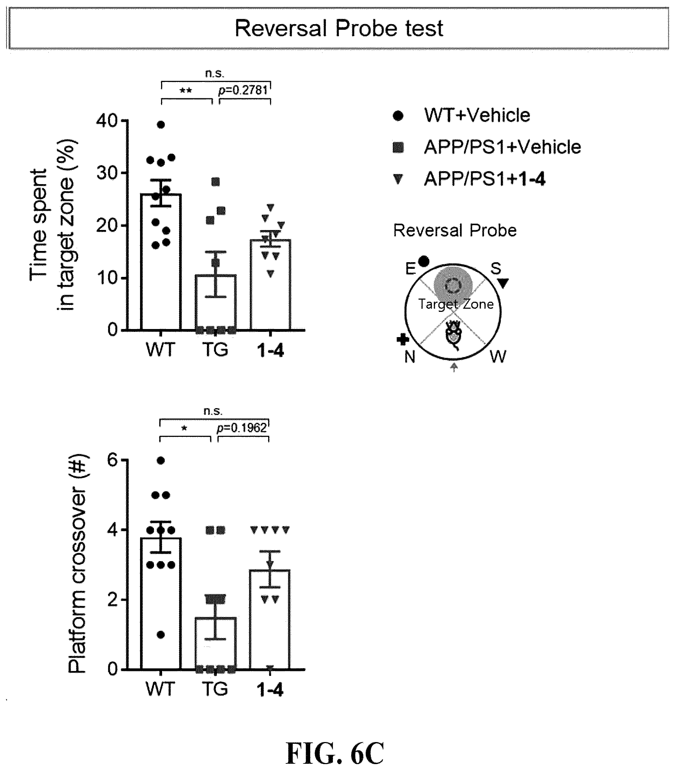

In the swimming training process (acquisition test), as the number of training sessions increased wild type normal mice memorized the location of the escape platform and they reached the escape platform within a short time, APP/PS1 Alzheimer's disease mice reached the escape platform relatively late, maintaining an escape latency of 40 seconds, and APP/PS1 mice administered with compound 1-4 reached the escape platform within a time that decreased over time to a level similar to that of the normal mice. Even when the escape platform was moved to a new location (reversal test), the compound 1-4 administered group reached the escape platform within a significantly reduced amount of time. This confirms that compound 1-4 improved the spatial learning ability of mice. After the training process, in the free swimming process (probe test, reversal probe test) for which the escape platform was removed, the APP/PS1 mice did not memorize the location of the escape platform, and thus spent less time in the target quadrant where the escape platform was located, compared to normal mice, and for the APP/PS1 mice administered with compound 1-4, the time they stayed in the target quadrant (time spent in target zone), the number of times they passed the escape platform location (platform crossover), and the time it took them to first reach the escape platform location (latency to platform) were each restored to the level of normal mice, confirming the efficacy of compound 1-4 in improving spatial learning and memory ability (Fig. 6a-6c).

### Example 9.2. Y-maze test

The Y-maze test was used to evaluate the spatial learning and memory ability of mice. The Y-shaped maze was made of 5 mm thick, black acrylic matte material and had three identical arms (length 41 cm, width 7 cm, and height 15 cm) arranged at a 120° angle to each other. The mouse was placed at the end of one arm of the maze, and its movements were recorded for 10 minutes to calculate and record the spontaneous alternation % (alternation % = number of alternations/(total number of arm entries - 2)), which is a measure of cognitive ability. The mouse was considered to have entered an arm when the center point of the mouse passed two-thirds of the arm, and alternation (change behavior) was defined as the mouse entering all three arms once without overlapping.

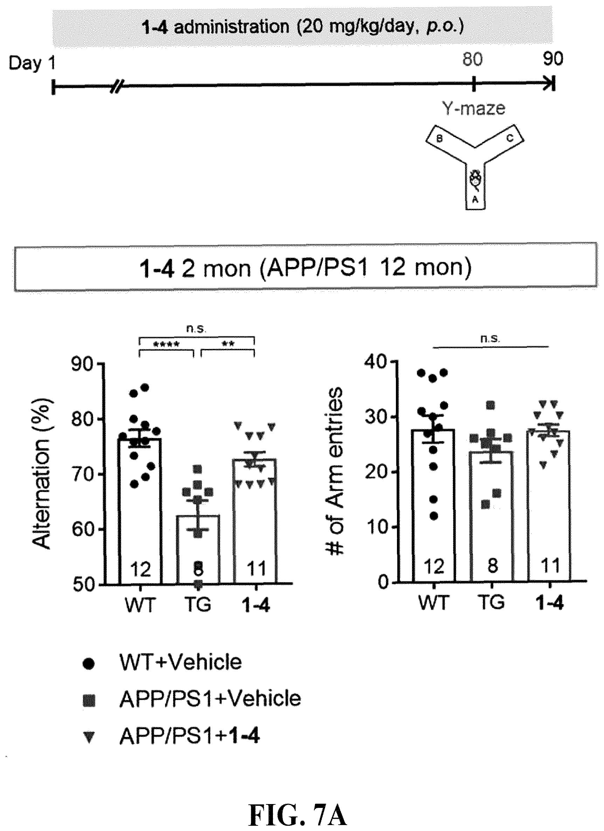

Wild-type normal mice showed a high alternation rate of about 77% by memorizing the arm entry order in the maze, APP/PS1 Alzheimer's disease mice statistically showed significantly reduced spatial learning and memory abilities compared to wild-type normal mice, and APP/PS1 mice administered with compound 1-4 statistically showed a significant increase in alternation, suggesting that spatial learning and memory abilities of those mice were restored to the level of wild-type normal mice. During the experiment, the average total number of arm entries of all mouse groups did not show mutual significance, confirming that the mobility of the mice was the same between the administration groups and thus alternation was not affected (Fig. 7a).

### Example 9.3. Passive avoidance test

The hippocampus-dependent contextual learning and memory of mice was evaluated using the passive avoidance test. The passive avoidance test apparatus was a GEMINI Avoidance System (San Diego Instruments), which has a dark room and a bright room separated by an automatic door. On the first day of the experiment (habituation), the mice were placed in the test apparatus and were allowed to roam freely for 3 minutes to acclimate to the environment of the apparatus. On the second day of the experiment (acquisition), taking advantage of a mouse's natural preference for dark places, the mice were placed in the bright room. 30 seconds later the automatic door was opened. If the mice voluntarily crossed into the dark room, an electric shock of 0.4 mA intensity was delivered for 1 second. 24 hours later (retention), the mice were placed back in the same bright room, and the time it took for the mice to cross into the dark room (step-through latency) was measured to determine learning and memory abilities related to the electric stimulus and the apparatus environment.

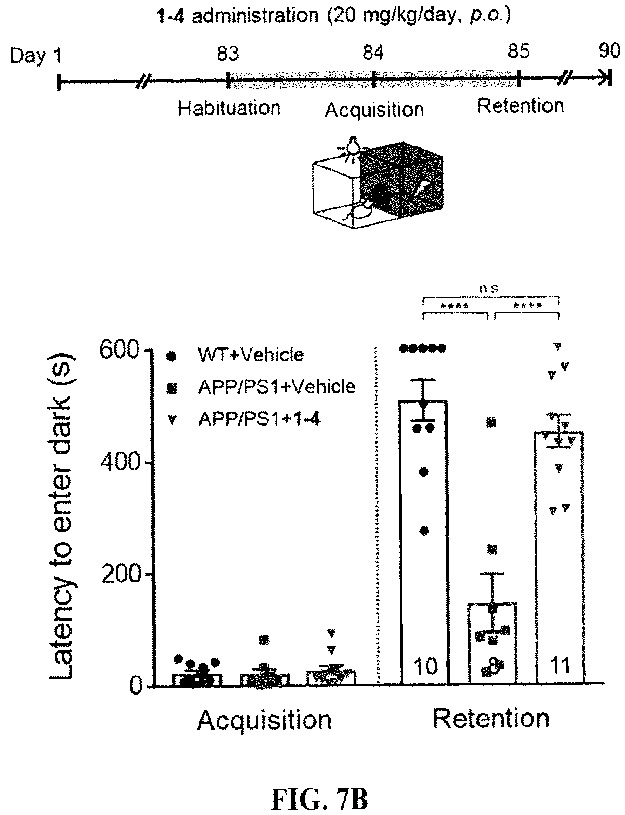

In the passive avoidance test, the APP/PS 1 Alzheimer's disease mice were unable to recall the electrical stimulus delivered during the acquisition trial, showing a significant decrease in step-through latency, while the APP/PS1 mice administered with compound 1-4 showed an increased step-through latency similar to that of normal mice, indicating that hippocampus-dependent contextual learning and memory ability was restored due to compound 1-4 administration (Fig. 7b).

While embodiments are illustrated with limited drawings as described above, it will be apparent to one of ordinary skill in the art that various alterations and modifications in form and details may be made to these embodiments without departing from the spirit and scope of the claims and their equivalents. For example, suitable results may be achieved if the described techniques are performed in a different order, and/or if components in a described system, architecture, device, or circuit are combined in a different manner, or replaced or supplemented by other components or their equivalents.

Therefore, other implementations, other embodiments, and equivalents to the claims are also within the scope of the following claims.

## Claims

1. A sulfanone derivative represented by the following [Chemical Formula 1] or a pharmaceutically acceptable salt thereof: 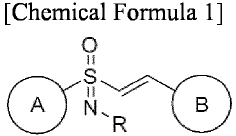 In Chemical Formula 1,
A and B are each independently phenyl or pyridine;
R is hydrogen, a cyano group, a trimethylsilyl group (Trimethylsilane, TMS), or a methyl group (except when A and B are both phenyl, and when R is hydrogen);
wherein each of the A and B is unsubstituted or substituted with any one or more substituents selected from a group consisting of a hydroxy group, a halogen group, a cyano group, a nitro group, a C₁-C₇ alkyl group, and a C₁-C₇ alkoxy group (except when the A and B are both unsubstituted), and
wherein one or more hydrogen of the alkyl group or alkoxy group are unsubstituted or substituted with any one or more substituent selected from a group consisting of a hydroxy group, a cyano group, a nitro group, and a morpholine group.

2. The sulfanone derivative or the pharmaceutically acceptable salt thereof of claim 1, wherein the sulfanone derivative represented by [Chemical Formula 1] is any one or more selected from a group consisting of compounds represented by the following chemical formulae:

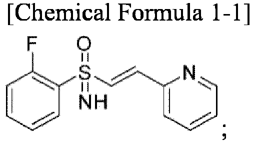

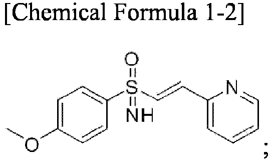

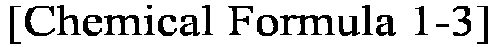

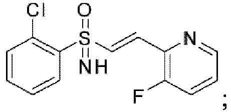

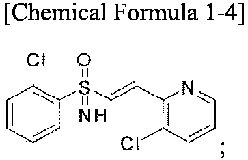

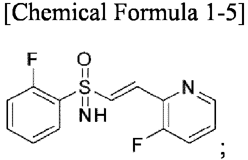

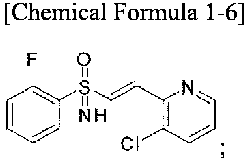

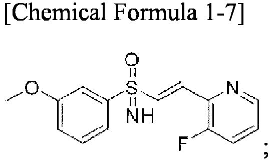

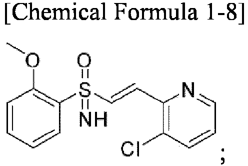

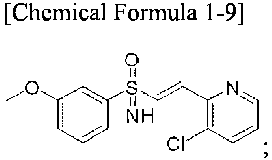

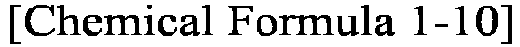

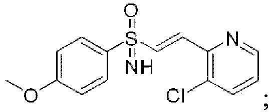

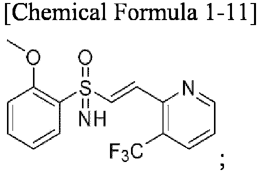

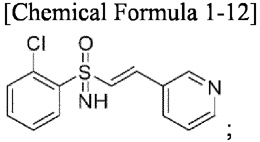

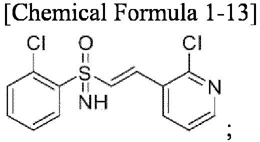

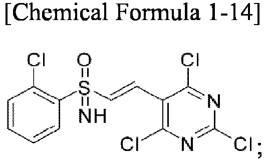

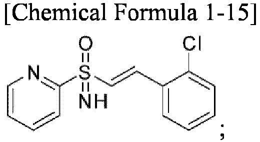

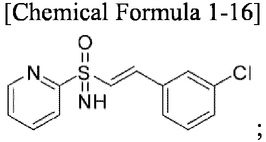

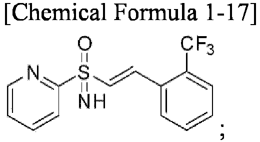

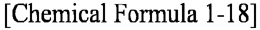

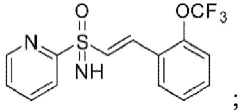

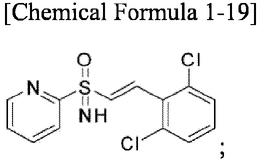

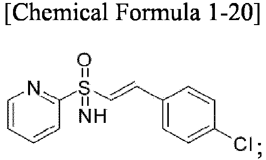

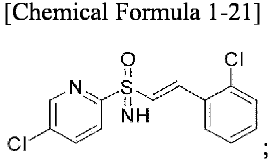

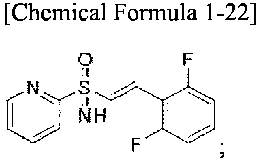

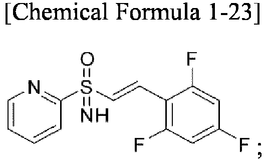

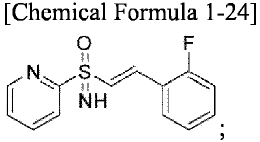

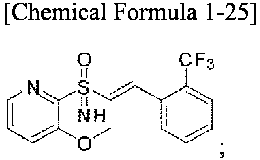

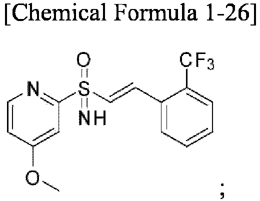

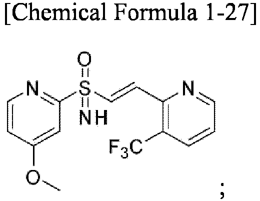

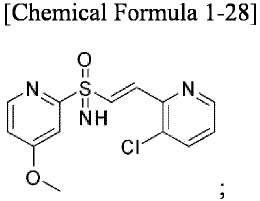

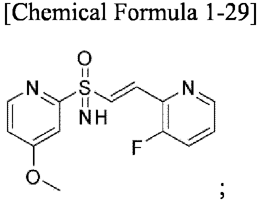

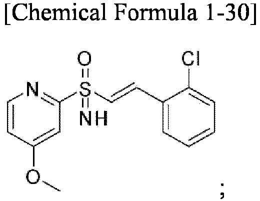

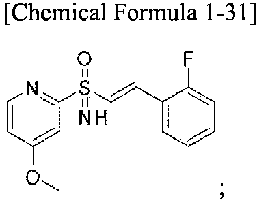

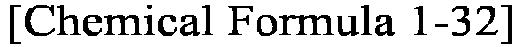

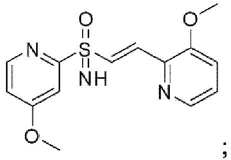

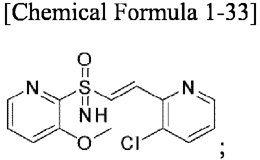

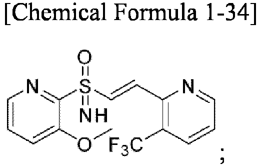

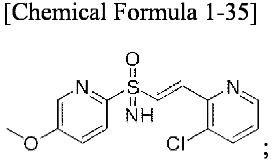

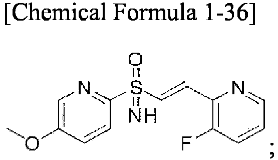

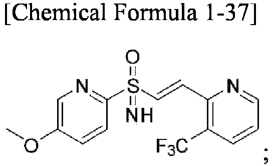

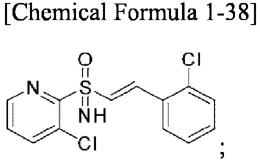

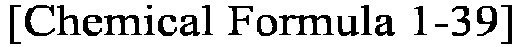

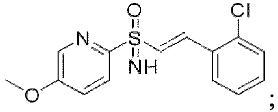

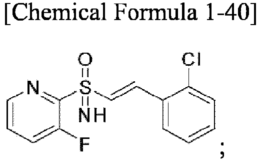

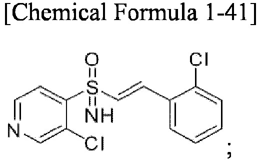

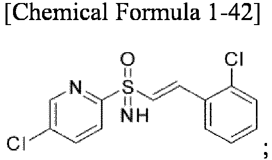

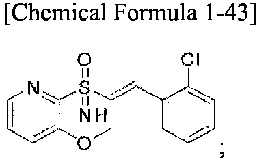

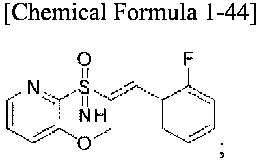

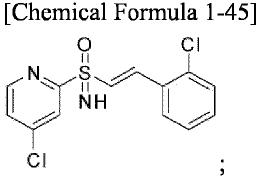

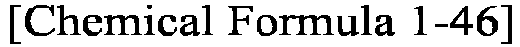

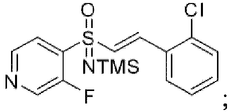

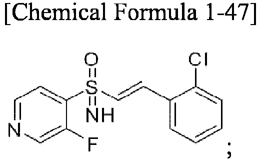

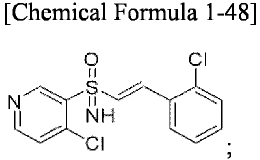

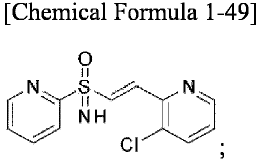

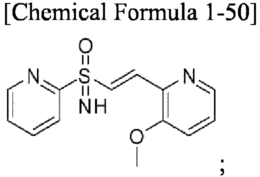

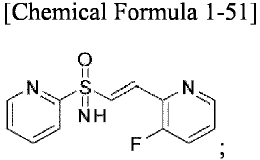

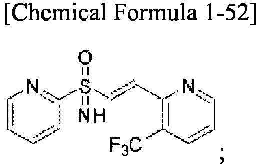

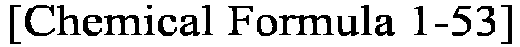

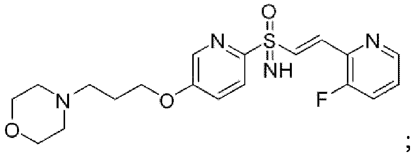

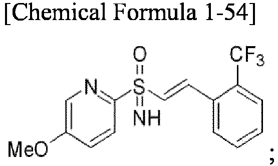

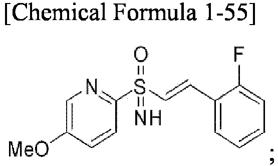

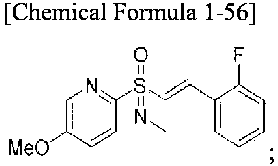

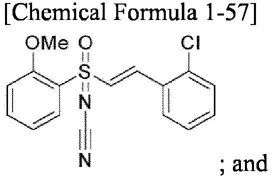

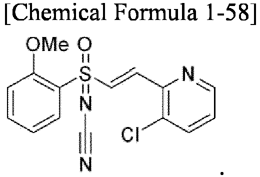

3. A composition for activating nuclear factor erythroid-derived 2-related factor 2 (Nrf2) comprising a sulfanone derivative represented by the following [Chemical Formula 1] or a pharmaceutically acceptable salt thereof as an active ingredient: 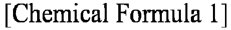 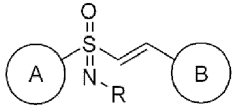 In Chemical Formula 1,
A and B are each independently phenyl or pyridine;
R is hydrogen, a cyano group, a trimethylsilyl group (Trimethylsilane, TMS), or a methyl group (except when the A and B are both phenyl, and when R is hydrogen);
wherein each of the A and B is unsubstituted or substituted with any one or more substituents selected from a group consisting of a hydroxy group, a halogen group, a cyano group, a nitro group, a C₁-C₇ alkyl group, and a C₁-C₇ alkoxy group (except when the A and B are both unsubstituted), and
wherein one or more hydrogen of the alkyl group or alkoxy group are unsubstituted or substituted with any one or more substituent selected from a group consisting of a hydroxy group, a cyano group, a nitro group, and a morpholine group.

4. A pharmaceutical composition for preventing or treating one or more disease selected from a group consisting of liver disease, kidney disease, pulmonary disease, neurodegenerative disease, mitochondrial myopathy, Friedreich's ataxia, corneal endothelial cell loss and psoriasis, comprising a sulfanone derivative represented by the following [Chemical Formula 1] or a pharmaceutically acceptable salt thereof as an active ingredient: 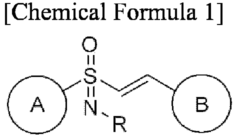 In Chemical Formula 1,
A and B are each independently phenyl or pyridine;
R is hydrogen, a cyano group, a trimethylsilyl group (Trimethylsilane, TMS), or a methyl group (except when the A and B are both phenyl, and when R is hydrogen);
wherein each of the A and B is unsubstituted or substituted with any one or more substituents selected from a group consisting of a hydroxy group, a halogen group, a cyano group, a nitro group, a C₁-C₇ alkyl group, and a C₁-C₇ alkoxy group (except when the A and B are both unsubstituted); and
wherein one or more hydrogen of the alkyl group or alkoxy group are unsubstituted or substituted with any one or more substituent selected from a group consisting of a hydroxy group, a cyano group, a nitro group, and a morpholine group.

5. The pharmaceutical composition of claim 4, wherein the disease is a disease induced by decreased Nrf2 activity.

6. The pharmaceutical composition of claim 4, wherein the liver disease is any one or more disease selected from a group consisting of alcoholic liver disease, non-alcoholic liver disease, non-alcoholic fatty liver disease (NAFLD), chronic liver injury, viral hepatitis and hepatocellular carcinoma.

7. The pharmaceutical composition of claim 4, wherein the kidney disease is any one or more disease selected from a group consisting of diabetic nephropathy, focal segmental glomerulosclerosis, renal fibrosis, lupus-like autoimmune nephritis, chronic kidney disease (CKD) and hypertensive kidney disease.

8. The pharmaceutical composition of claim 4, wherein the pulmonary disease is any one or more disease selected from a group consisting of chronic obstructive pulmonary disease (COPD), pulmonary emphysema, ventilation-associated lung injury, acute lung injury (ALI), acute respiratory distress syndrome (ARDS), pulmonary artery hypertension (PAH) and right heart failure induced by the pulmonary artery hypertension.

9. The pharmaceutical composition of claim 4, wherein the neurodegenerative disease is any one or more disease selected from a group consisting of Parkinson's disease (PD), Alzheimer's disease (AD), Huntington's disease, Lou Gehrig's disease, epilepsy, depression, insomnia, anxiety and multiple sclerosis (MS).

10. The pharmaceutical composition of claim 4, wherein the pharmaceutical composition comprises the sulfanone derivative or a pharmaceutically acceptable salt thereof; and
further comprises any one or more additional components selected from a group consisting of a pharmaceutically acceptable carrier, excipient, diluent, stabilizer and preservative.

11. The pharmaceutical composition of claim 10, wherein the pharmaceutical composition has any one or more dosage form selected from a group consisting of powders, granules, tablets, capsules, and injections.

12. A method for preparing a sulfanone derivative or a pharmaceutically acceptable salt thereof, the method comprising the following steps:
(1) preparing a compound represented by [Chemical Formula 3] from a compound represented by [Chemical Formula 2];
(2) preparing a compound represented by [Chemical Formula 4] from the compound represented by [Chemical Formula 3];
(3) preparing a compound represented by [Chemical Formula 5] from the compound represented by [Chemical Formula 4]; and
(4) preparing a compound represented by [Chemical Formula 1] by adding a compound represented by [Chemical Formula 6] to the compound represented by [Chemical Formula 5]. 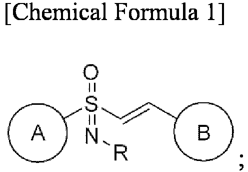 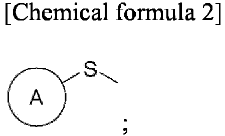 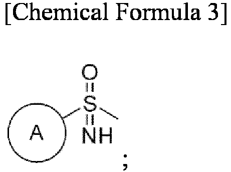 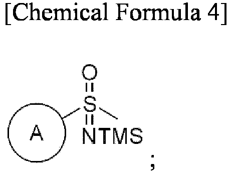 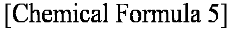 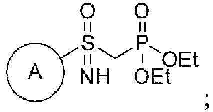 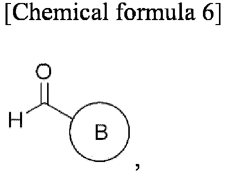 in Chemical Formulae 1 to 6,
A and B are each independently phenyl or pyridine (except when the A and B are both phenyl);
R is hydrogen, a trimethylsilyl group (Trimethylsilane, TMS), or a methyl group;
wherein each of the A and B is unsubstituted or substituted with any one or more substituents selected from a group consisting of a hydroxy group, a halogen group, a cyano group, a nitro group, a C₁-C₇ alkyl group, and a C₁-C₇ alkoxy group (except when the A and B are both unsubstituted); and
wherein one or more hydrogen of the alkyl group or alkoxy group are unsubstituted or substituted with any one or more substituent selected from a group consisting of a hydroxy group, a cyano group, a nitro group, and a morpholine group.

13. The method of claim 12, wherein in step (1), the compound represented by [Chemical Formula 2] is dissolved in methanol (MeOH), and then ammonium carbonate ((NH₄)₂CO₃) and diacetoxyiodobenzene (PhI(OAc)₂) are sequentially added.

14. The method of claim 12, wherein in step (2), hexamethyldisilazane (HMDS) is added to the compound represented by [Chemical Formula 3], followed by stirring under reflux.

15. The method of claim 12, wherein in step (3), the compound represented by [Chemical Formula 4] is dissolved in tetrahydrofuran (THF), and then n-butyllithium (n-BuLi) and diethyl chlorophosphate are sequentially added.
